# FASCICULE DE BREVET EUROPEEN

(11) **EP 0 494 819 B1**
(45) Date de publication et mention de la délivrance du brevet: **10.07.1996**
(21) Numéro de dépôt: 92400038.3
(22) Date de dépôt: 08.01.1992
(51) Int. Cl.: C07D 233/86, C07D 233/84, C07D 233/72, C07D 405/06, A61K 31/415, C07D 233/88, C07D 405/04

(54) **Nouvelles phénylimidazolidines, leur procédé de préparation, leur application comme médicaments et les compositions pharmaceutiques les renfermant**
Phenylimidazolidine, Verfahren zu deren Herstellung, deren Anwendung als Arzneimittel und diese enthaltende pharmazeutische Zusammenstellungen
Phenylimidazolidines, their process for fabrication, their application as medicaments and the pharmaceutical compositions containing them

(30) Priorité: 09.01.1991 FR 9100185
(43) Date de publication de la demande: 15.07.1992
(73) Titulaire: ROUSSEL UCLAF, F-93230 Romainville (FR)
(72) Inventeur: Gaillard-Kelly, Martine, F-75009 Paris (FR); Goubet, François, F-75015 Paris (FR); Philibert, Daniel, F-94210 La Varenne Saint Hilaire (FR); Teutsch, Jean-Georges, F-93500 Pantin (FR)
(74) Mandataire: Vieillefosse, Jean-Claude

(56) Documents cités:
- EP-A- 0 001 813
- EP-A- 0 024 570
- EP-A- 0 185 961
- EP-A- 0 305 270
- FR-A- 2 329 276
- US-A- 3 823 240
- US-A- 4 672 101

## Description

La présente invention concerne de nouvelles phénylimidazolidines, leur procédé de préparation, leur application comme médicaments et les compositions pharmaceutiques les renfermant.

Dans la demande japonaise J 48087030 sont décrites des 3-phényl 2-thiohydantoïnes qui sont présentées comme inhibant la germination de certaines plantes.

Dans le brevet français 2.329.276 ainsi que dans EP-A-0 01 813 sont décrites des imidazolidines qui sont présentées comme possédant une activité antiandrogène. Les produits de ce brevet sont cependant différents des produits de la présente demande de brevet.

La présente invention a donc pour objet les produits de formule générale (I) : 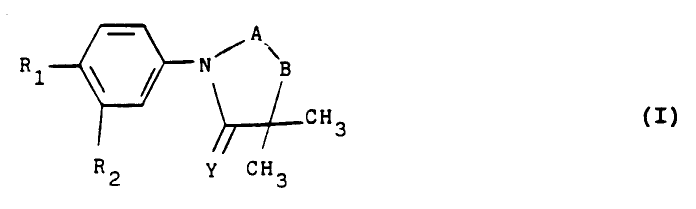 dans laquelle :
R_{**1**} représente un radical cyano ou nitro ou un atome d'halogène,
R_{**2**} représente un radical trifluorométhyle ou un atome d'halogène,
le groupement -A-B- est choisi parmi les radicaux 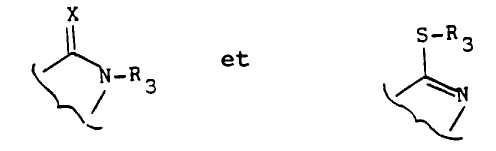 dans lesquels X représente un atome d'oxygène ou de soufre et
R_{**3**} est choisi parmi les radicaux suivants :

- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, S-aryle éventuellement substitué, dans lequel l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,
   les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone,
   les radicaux aryle et aralkyle étant de plus éventuellement substitués par un radical alkyle, alkényle ou alkynyle, alkoxy, alkényloxy, alkynyloxy ou trifluorométhyle,
   Y représente un atome d'oxygène ou de soufre ou un radical =NH,
   à l'exception des produits dans lesquels le groupement A-B représente le radical : 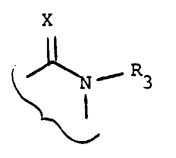 dans lequel X représente un atome d'oxygène et R_{**3**} représente un atome d'hydrogène et Y représente un atome d'oxygène ou un radical NH et R_{**2**} représente un atome d'halogène ou un radical trifluorométhyle et R_{**1**} représente un radical nitro ou un atome d'halogène.

Pour la définition de R_{**3**} et dans ce qui suit, les définitions utilisées peuvent avoir les valeurs suivantes.

Par alkyle ayant au plus 12 atomes de carbone on entend par exemple les valeurs méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, isohexyle,tert-hexyle, heptyle, octyle, décyle, undécyle, dodécyle, linéaires ou ramifiés.

On préfère les radicaux alkyle ayant au plus 4 atomes de carbone et notamment les radicaux méthyle, éthyle, propyle, isopropyle.

Par alkényle ayant au plus 12 atomes de carbone on entend par exemple les valeurs suivantes :
- vinyle, allyle, 1-propényle, butényle, pentényle, hexenyle.

On préfère les valeurs alkényle ayant au plus 4 atomes de carbone et notamment les valeurs vinyle ou allyle.

Par alkynyle ayant au plus 12 atomes de carbone on entend par exemple les valeurs suivantes :
- éthynyle, propargyle, butynyle, pentynyle ou hexynyle.

On préfère les valeurs alkynyle ayant au plus 4 atomes de carbone et notamment les valeurs éthynyle et propargyle.

Par aryle on entend les radicaux aryles carbocyclique tels que le phényle ou le naphtyle ou les aryles hétérocycliques à 5 ou 6 chaînons comportant un ou plusieurs hétéroatomes choisis de préférence parmi l'oxygène, le soufre et l'azote. Parmi les aryles hétérocycliques à 5 chaînons on peut citer les radicaux furyle, thiényle, pyrrolyle, thiazolyle, oxazolyle, imidazolyle, thiadiazolyle, pyrazolyle, isoxazolyle.

Parmi les aryles hétérocycliques à 6 chaînons on peut citer les radicaux pyridyle, pyrimidinyle, pyridazinyle, pyrazinyle.

Parmi les radicaux aryles condensés on peut citer les radicaux indolyle, benzofurannyle, benzothiényle, quinoléïnyle.

On préfère le radical phényle.

Par arylalkyle on entend les radicaux résultant de la combinaison des radicaux alkyle cités précédemment et les radicaux aryle également cités ci-dessus.

On préfère les radicaux benzyle ou phényléthyle.

Par halogène, on entend bien entendu, les atomes de fluor, de chlore, de brome ou d'iode.

On préfère les atomes de fluor, de chlore ou de brome.

Comme exemples particuliers de radicaux alkyle substitués par un ou plusieurs halogènes, on peut citer les monofluoro, chloro, bromo ou iodométhyle, les difluoro, dichloro ou dibromométhyle, le trifluorométhyle.

Comme exemples particuliers de radicaux aryles ou aralkyles substitués, on peut citer ceux dans lesquels le radical phényle est substitué en position para, par un atome de fluor ou par un radical méthoxy ou trifluorométhyle.

Par radical acyle, on entend de préférence un radical ayant au plus 7 atomes de carbone tel que le radical acétyle, propionyle, butyryle ou benzoyle, mais peut également représenter un radical valéryle, hexanoyle, acryloyle, crotonoyle ou carbamoyle : on peut également citer le radical formyle.

Par radical acyloxy, on entend les radicaux dans lesquels les radicaux acyle ont la signification indiquée ci-dessus et par exemple les radicaux acétoxy ou propionyloxy.

Par carboxy estérifié on entend par exemple les radicaux tels que les radicaux alkyloxycarbonyle par exemple méthoxycarbonyle, éthoxycarbonyle, propoxycarbonyle, butyl ou tert-butyloxycarbonyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les radicaux du type 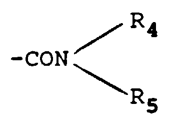 dans lesquels les radicaux R_{**4**} et R_{**5**} identiques ou différents représentent un atome d'hydrogène ou un radical alkyle ayant de 1 à 4 atomes de carbone tels que les radicaux méthyle, éthyle, propyle, isopropyle, butyle, isobutyle, sec-butyle ou tert-butyle.

Parmi les radicaux 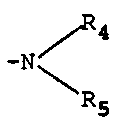 on préfère les radicaux amino, mono ou diméthylamino.

Le radical 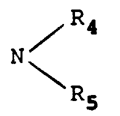 peut également représenter un hétérocycle qui peut ou non comporter un hétéroatome supplémentaire. On peut citer les radicaux pyrrolyle, imidazolyle, pyridyle, pyrazinyle, pyrimidyle, indolyle, pipéridino, morpholino, pipérazinyle. On préfère les radicaux pipéridino, ou morpholino.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine.

On préfère le sel de sodium.

Par radical alkylamino on entend les radicaux méthylamino, éthylamino, propylamino ou butyl, linéaire ou ramifié, amino. On préfère les radicaux alkyle ayant au plus 4 atomes de carbone, les radicaux alkyle peuvent être choisis parmi les radicaux alkyle cités ci-dessus.

Par radical dialkylamino on entend par exemple les radicaux diméthylamino, diéthylamino, méthyléthylamino. Comme précédemment on préfère les radicaux alkyle ayant au plus 4 atomes de carbone choisis dans la liste indiquée ci-dessus.

Par radical hétérocyclique renfermant un ou plusieurs hétéroatomes, on entend par exemple les radicaux monocycliques, hétérocycliques saturés tels que les radicaux oxirannyle, oxolannyle, dioxolannyle, imidazolidinyle, pyrazolidinyle, pipéridyle, pipérazinyle ou morpholinyle.

Par radicaux alkyle, alkényle, ou alkynyle éventuellement interrompus par un hétéroatome choisis parmi les atomes de soufre, d'oxygène ou d'azote, on entend les radicaux comprenant un ou plusieurs de ces atomes, identiques ou différents dans leur structure. Ces hétéroatomes ne pouvant évidemment pas être situés à l'extrémité du radical. On peut citer par exemple les radicaux alkoxyalkyle tels que méthoxyméthyle ou méthoxyéthyle ou encore les radicaux alkoxy alkoxyalkyle tels que méthoxyéthoxyméthyle.

Lorsque les produits de formule (I) comportent un radical amino salifiable par un acide il est bien entendu que ces sels d'acides font également partie de l'invention. On peut citer les sels fournis avec les acides chlorhydrique en méthanesulfonique par exemple.

L'invention a notamment pour objet les produits de formule (I) telle que définie ci-dessus dans laquelle Y représente un atome d'oxygène à l'exception des produits dans lesquels :
le groupement -A-B- représente le radical : 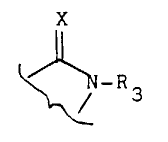 dans lequel X représente un atome d'oxygène et R_{**3**} représente un atome d'hydrogène et R_{**2**} représente un atome d'halogène ou un radical trifluorométhyle et R_{**1**} représente un radical nitro ou un atome d'halogène.

Parmi ces produits, l'invention a particulièrement pour objet ceux dans lequel le groupement -A-B- représente le radical : 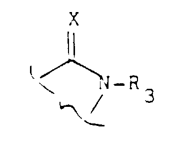 dans lequel X représente un atome de soufre et R_{**3**} a la signification indiquée précédemment.

Parmi ces produits, l'invention a particulièrement pour objet ceux dans lesquels R représente un atome d'hydrogène ou un radical alkyle ayant au plus 4 atomes de carbone éventuellement substitué par un radical hydroxy.

Parmi ces produits, l'invention a tout particulièrement pour objet ceux dans lesquels R_{**1**} représente un radical cyano ou un atome d'halogène et notamment un atome de chlore.

L'invention a aussi particulièrement pour objet les produits de formule (I) telle que définie ci-dessus dans laquelle le groupement -A-B- représente un groupement : 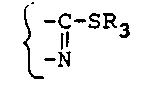 ou un groupement : 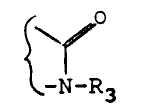 dans lequel R_{**3**} représente un radical alkyle ou alkényle ayant au plus 4 atomes de carbone ou un radical aralkyle éventuellement substitué.

La présente invention a encore pour objet les produits de formule (I) telle que définie ci-dessus et répondant à la formule (I') : 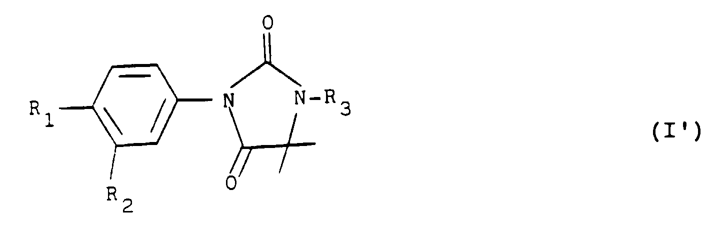 dans laquelle R_{**1**}, R_{**2**} et R_{**3**} ont la signification indiquée ci-dessus à l'exception des produits dans lesquels R_{**1**} représente un radical nitro, R_{**2**} représente un radical trifluorométhyle et R_{**3**} représente un atome d'hydrogène.

Parmi ces produits, la présente invention a aussi pour objet les produits de formule (I) telle que définie ci-dessus dans laquelle R_{**1**} représente un radical nitro et R_{**3**} représente un radical alkyle ou alkényle ayant au plus 4 atomes de carbone éventuellement substitué par un radical carboxy libre estérifié ou salifié.

Parmi les produits préférés de l'invention, on peut citer plus précisément les produits de formule (I) telle que définie ci-dessus dont les noms suivent :
- le 4-(5-oxo-2-thioxo-3,4,4-triméthyl-1-imidazolidinyl)-2-(trifluorométhyl)-benzonitrile,
- le 4-(4,4-diméthyl-5-oxo-2-thioxo 1-imidazolidinyl)-2-(trifluorométhyl)-benzonitrile,
- le 4-[4,4-diméthyl 3-(2-hydroxyéthyl) 5-oxo 2-thioxo 1-imidazolidinyl] 2-(trifluorométhyl) benzonitrile,
- le 3-(3,4-dichlorophényl) 2-thioxo 1,5,5-triméthyl 4-imidazolidinone,
- le 1-(4-nitro-3-(trifluorométhyl) phényl)-3,4,4-triméthyl-2,5-imidazolidinedione,
- le 4-[[4,5-dihydro 4,4-diméthyl 5-oxo 2-(phénylméthyl) thio] 1H-imidazol-1-yl] 2-(trifluorométhyl) benzonitrile.

L'invention a aussi pour objet un procédé de préparation des produits de formule générale (I) telle que définie ci-dessus caractérisé en ce que :
soit l'on fait agir en présence d'une base tertiaire un produit de formule (II) : 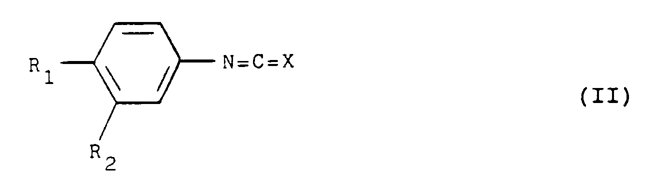 dans laquelle R_{**1**}, R_{**2**} et X ont la signification indiquée ci-dessus, avec un produit de formule (III) : 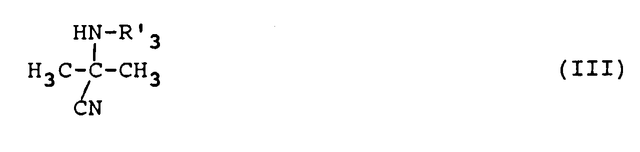 dans laquelle R'_{**3**} a les valeurs indiquées ci-dessus pour R_{**3**} dans lequel les éventuelles fonctions réactives sont éventuellement protégées et étant entendu que si R_{**1**} représente un radical nitro ou un atome d'halogène, si R_{**2**} représente un atome d'halogène ou un radical CF_{**3**} et X représente un atome d'oxygène, R'_{**3**} ne peut pas représenter un atome d'hydrogène, pour obtenir un produit de formule (IV) : 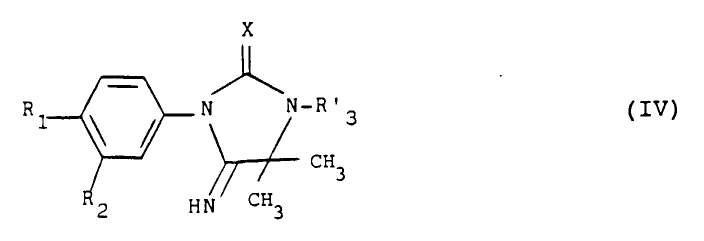 dans laquelle R_{**1**}, R_{**2**}, X et R′_{**3**} ont la signification précédente, produits de formule (IV) que, si nécessaire ou si désiré l'on soumet à l'une quelconque ou plusieurs des réactions suivantes, dans un ordre quelconque :
a) réaction d'élimination des éventuels groupements protecteurs que peut porter R′_{**3**} ;
b) réaction d'hydrolyse du groupement >C=NH en fonction cétone et le cas échéant transformation du groupement >C=S en groupement >C=O ;
c) réaction de transformation du ou des groupements >C=O en groupement >C=S ;
d) action sur les produits de formule (IV) dans laquelle R′_{**3**} représente un atome d'hydrogène, et après hydrolyse au groupement >C=NH en fonction cétone d'un réactif de formule Hal-R˝_{**3**} dans laquelle R˝_{**3**} a les valeurs de R′_{**3**} à l'exception de la valeur hydrogène et Hal représente un atome d'halogène pour obtenir des produits de formule (I) dans laquelle le groupement -A-B- représente le groupement 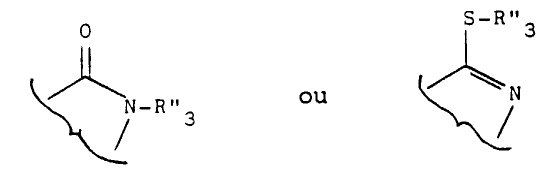 dans lesquels R˝_{**3**} a la signification indiquée précédemment puis, si désiré, action sur ces produits, d'un agent d'élimination des éventuels groupements protecteurs que peut porter R˝_{**3**} ou le cas échéant, action d'un agent d'estérification, d'amidification ou de salification,
soit l'on fait agir un réactif de formule Hal-R˝_{**3**} dans laquelle Hal et R˝_{**3**} ont les valeurs indiquées précédemment sur un produit de formule (IV′) : 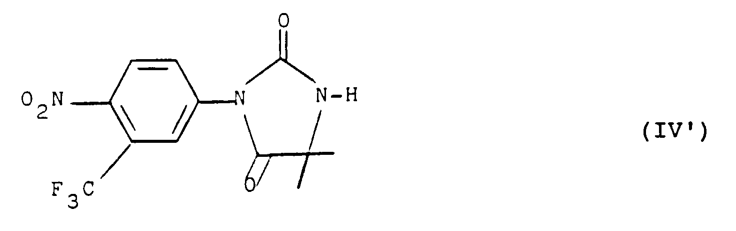 pour obtenir un produit de formule (IV˝) : 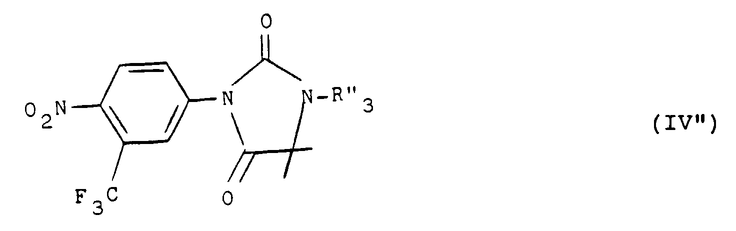 produit de formule (IV˝) que, si nécessaire ou si désiré l'on soumet à l'une quelconque ou plusieurs des réactions suivantes dans un ordre quelconque :
a) réaction d'élimination des éventuels groupements protecteurs que peut porter R˝_{**3**} puis le cas échéant action d'un agent d'estérification, d'amidification ou de salification ;
b) réaction de transformation du ou des groupements >C=O en groupements >C=S.

L'action des produits de formule (II) avec les produits de formule (III) est effectuée de préférence dans un solvant organique tel que le tétrahydrofuranne ou le dichloroéthane mais on peut également utiliser l'éther éthylique ou l'éther isopropylique.

On opère en présence d'une base tertiaire telle que la triéthylamine ou encore la pyridine ou la méthyléthylpyridine.

Les éventuelles fonctions réactives que peut comporter R_{**3**} et qui sont éventuellement protégées dans le produit de formule (III) ou (IV˝) sont les fonctions hydroxy ou amino. On utilise pour protéger ces fonctions des groupements protecteurs usuels. On peut citer par exemples les groupements protecteurs suivants du radical amino : tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthylsilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français BF 2.499.995 dont le contenu est incorporé ici par référence.

Les réactions éventuelles d'élimination des groupements protecteurs sont effectuées comme indiqué dans ledit brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.

La réaction éventuelle d'hydrolyse du groupement >C=NH en groupement cétone est également effectuée de préférence à l'aide d'un acide tel que l'acide chlorhydrique aqueux par exemple au reflux.

Lorsque l'hydrolyse du groupement >C=NH en groupement cétone est effectuée sur une molécule comportant également un groupement >C=S, celui-ci peut être transformé en groupement >C=O. Le radical OH libre que peut comporter éventuellement R_{**3**} peut être alors transformé en radical SH.

La réaction de transformation du ou des groupements >C=O en groupement >C=S est effectuée à l'aide du réactif dit de Lawesson de formule : 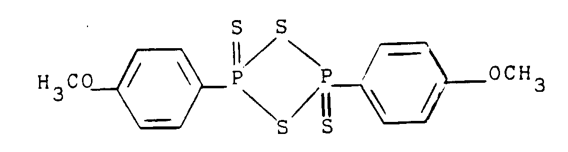 qui est un produit commercialisé par exemple par la firme FLUKA et dont l'utilisation est décrite par exemple dans la publication : Bull. Soc. Chim. Belg. vol 87, N° 3, (1987) p. 229.

Lorsque l'on veut transformer deux fonctions >C=O en deux fonctions >C=S on opère en présence d'un excès de réactif de Lawesson. Il en est de même lorsque l'on part d'une molécule comportant une fonction >C=S et une fonction >C=O et que l'on veut transformer ladite fonction >C=O en fonction >C=S.

Par contre lorsque l'on part d'une molécule comportant deux fonctions >C=O et que l'on veut obtenir un produit ne comportant qu'une seule fonction >C=S. On opère en présence d'un déficit de réactif de Lawesson. On obtient alors en général un mélange de trois produits : chacun des deux produits comportant une fonction >C=O et une fonction >C=S et le produit comportant deux fonctions >C=S. Ces produits peuvent être ensuite séparés par les méthodes usuelles telles que la chromatographie.

L'action sur les produits de formules (IV) ou (IV′) du réactif de formule Hal-R˝_{**3**} est effectuée en présence d'une base forte telle que l'hydrure de sodium ou de potassium. On peut opérer par réaction de transfert de phase en présence de sels d'ammonium quaternaires tels que le tert-butyl ammonium.
- Les groupements protecteurs que peut porter le substituant R˝_{**3**} pouvant être par exemple un de ceux précédemment cités pour R_{**3**}. Les réactions d'élimination des groupements protecteurs s'effectuent dans les conditions indiquées ci-dessus.

Un exemple d'élimination du groupement terbutyldiméthylsilyle au moyen de l'acide chlorhydrique est donné ci-après dans les exemples.
- L'estérification éventuelle des produits de formule (I) dans laquelle R˝_{**3**} comporte un radical OH libre est effectuée dans des conditions classiques. On peut utiliser par exemple un acide ou un dérivé fonctionnel, par exemple un anhydride tel que l'anhydride acétique en présence d'une base telle que la pyridine.

L'estérification ou la salification éventuelle des produits de formule (I) dans laquelle R˝_{**3**} représente un groupement COOH est effectuée dans les conditions classiques connues de l'homme du métier.
- L'amidification éventuelle des produits de formule (I) dans laquelle R˝_{**3**} comporte un radical COOH est effectuée dans des conditions classiques. On peut utiliser une amine primaire ou secondaire sur un dérivé fonctionnel de l'acide par exemple un anhydride symétrique ou mixte.

La présente invention a également pour objet un procédé de préparation des produits de formule (I˝) : 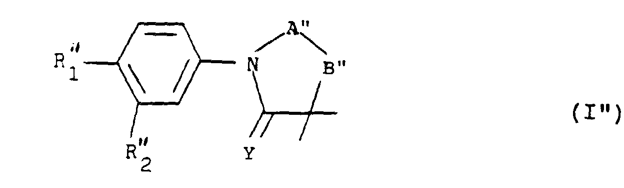 dans laquelle R˝_{**1**}, R˝_{**2**}, -A˝-B˝- ont les significations indiquées ci-dessus pour R_{**1**}, R_{**2**} et -A-B- étant entendu que lorsque -A˝-B˝- représente un groupement -CO-N(R˝′_{**3**})- dans lequel R˝′_{**3**} représente un atome d'hydrogène ou un radical alkyle linéaire ou ramifié ayant au plus 7 atomes de carbone et Y représente un atome d'oxygène, R˝_{**1**} représente un radical cyano, ce procédé étant caractérisé en ce que l'on fait réagir un produit de formule (V) : 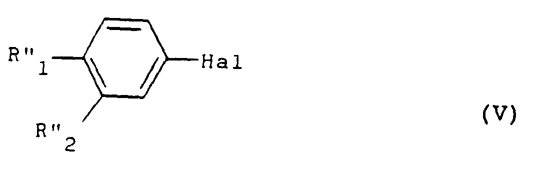 dans laquelle R˝_{**1**} et R˝_{**2**} ont les significations précédentes et Hal représente un atome d'halogène avec un produit de formule (VI) : 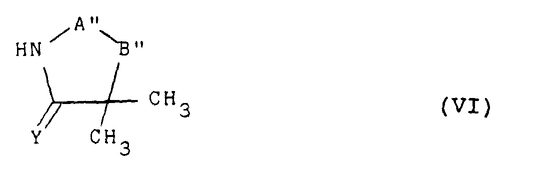 dans laquelle -A˝-B˝- et Y ont la signification indiquée ci-dessus, la réaction s'effectuant en présence d'un catalyseur et éventuellement d'un solvant.

En ce qui concerne les produits de formule (V), le terme Hal désigne de préférence l'atome de chlore, mais peut aussi représenter un atome de brome ou d'iode.

Le rôle du catalyseur est vraisemblablement de piéger l'halogénure d'hydrogène qui se dégage et ainsi de faciliter la réaction de condensation du produit de formule (V) avec le produit de formule (VI) pour donner le produit recherché.

L'invention a plus précisément pour objet un procédé tel que défini ci-dessus dans lequel le catalyseur est un métal sous forme native ou oxydée ou une base.

Le catalyseur utilisé peut être un métal sous forme native, sous forme d'oxyde métallique ou encore sous forme de sels métalliques. Le catalyseur peut également être une base. Quand le catalyseur utilisé est un métal, ce métal peut être du cuivre ou du nickel.

Les sels métalliques peuvent être un chlorure ou un acétate.

Quand le catalyseur est une base, cette base peut être par exemple la soude ou la potasse et on peut, si désiré, ajouter au milieu réactionnel du diméthylsulfoxyde.

L'invention a plus précisément pour objet un procédé tel que défini ci-dessus dans lequel le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

L'invention a particulièrement pour objet un procédé tel que défini ci-dessus dans lequel le catalyseur est l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

L'invention a plus particulièrement pour objet un procédé tel que défini ci-dessus caractérisé en ce que l'on opère en présence d'un solvant de type éther tel que l'oxyde de phényle, le diglyme, le triglyme ou le diméthylsulfoxyde.

L'invention a tout particulièrement pour objet un procédé tel que défini ci-dessus dans lequel le solvant utilisé est l'oxyde de phényle ou le triglyme.

Le procédé de préparation du produit recherché défini ci-dessus peut être réalisé sous pression ou à la pression atmosphérique, à une température préférentiellement élevée.

L'invention a ainsi pour objet un procédé tel que défini ci-dessus caractérisé en ce que la réaction est réalisée à une température supérieure à 100°C et de préférence supérieure à 150°C.

L'invention a plus précisément pour objet un procédé tel que défini ci-dessus caractérisé en ce que la réaction est réalisée pendant plus de 2 heures.

L'invention a très précisément pour objet un procédé tel que défini ci-dessus caractérisé en ce que la réaction est réalisée en présence d'oxyde cuivreux, dans le triglyme, à une température supérieure ou égale à 200°C et pendant plus de 3 heures.

Les produits objets de la présente invention sont doués de propriétés pharmacologiques intéressantes ; on a constaté notamment qu'ils inhibaient les effets des androgènes sur les récepteurs périphériques.

Des tests donnés dans la partie expérimentale illustrent cette activité anti-androgène.

Du fait de cette activité anti-androgène, les produits de l'invention peuvent être utilisés en thérapeutique chez les adultes sans avoir à redouter certains effets d'une castration chimique.

Ces propriétés rendent les produits de formule générale (I) de la présente invention utilisables comme médicaments pour le traitement des adénomes et des néoplasies de la prostate ainsi que pour lutter contre l'hypertrophie bénigne de la prostate.

Ces propriétés rendent les produits de formule générale (I) également utilisables dans le traitement des tumeurs bénignes ou malignes dont les cellules contiennent notamment des récepteurs androgènes. On peut en particulier citer principalement les cancers du sein, du cerveau, de la peau et des ovaires mais également les cancers de la vessie, du système lymphatique, du rein, du foie.

Les produits de formule générale (I) de l'invention trouvent également leur utilisation dans le traitement de l'hirsutisme, de l'acné, de la seborrhée, de l'alopécie androgénique, de l'hyperpilosité.

Ils peuvent également être utilisés dans le domaine vétérinaire.

L'invention a donc pour objet l'application, à titre de médicaments, des produits de formule générale (I) pharmaceutiquement acceptables.

L'invention a particulièrement pour objet l'application à titre de médicaments, des produits dont les noms suivent :
- le 4-(5-oxo-2-thioxo-3,4,4-triméthyl-1-imidazolidinyl)-2-(trifluorométhyl)-benzonitrile,
- le 4-(4,4-diméthyl-5-oxo-2-thioxo-1-imidazolidinyl)-2-(trifluorométhyl)-benzonitrile,
- le 4-[4,4-diméthyl 3-(2-hydroxyéthyl) 5-oxo 2-thioxo 1-imidazolidinyl] 2-(trifluorométhyl) benzonitrile,
- le 3-(3,4-dichlorophényl) 2-thioxo 1,5,5-triméthyl 4-imidazolidinone,
- le 1-(4-nitro-3-(trifluorométhyl) phényl)-3,4,4-triméthyl-2,5-imidazolidinedione,
- le 4-[[4,5-dihydro 4,4-diméthyl 5-oxo 2-(phénylméthyl) thio] 1H-imidazol-A-yl] 2-(trifluorométhyl) benzonitrile.

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule générale (I).

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) :

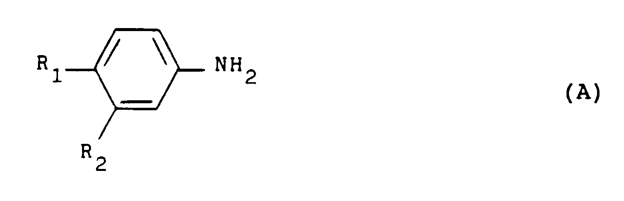

Un exemple d'une telle préparation est donné ci-après dans la partie expérimentale. Un produit de ce type est décrit également dans le brevet français BF 2.329.276.

Les amines de formule (A) sont décrites dans le brevet européen EP 0.002.892 ou le brevet français BF 2.142.804.

Les produits de formule (III) sont connus ou peuvent être préparés à partir de la cyanhydrine correspondante selon le procédé décrit dans la publication : J. Am. Chem. Soc. (1953), 75, 4841.

Les produits de formule (III) dans lesquels R′_{**3**} est différent d'un atome d'hydrogène peuvent être obtenus par action d'un produit de formule R˝_{**3**} Hal sur le 2-cyano 2-amino propane dans les conditions énoncées ci-dessus pour l'action de R˝_{**3**} Hal sur les produits de formule (IV). Un exemple de préparation de ce type est décrit dans la référence :
- Jilek et Coll. Collect. Cyech. Chem. Comm. 54(8) 2248 (1989).

Les produits de formule (IV′) sont décrits dans le brevet français BF 2.329.276.

Les produits de départ de formules (V) et (VI), sur lesquels s'exerce un procédé, objet de l'invention, pour l'obtention des produits de formule (I), sont connus et disponibles dans le commerce ou peuvent être préparés selon des méthodes connues de l'homme de métier.

La préparation de produits de formule (VI) est décrite notamment dans les publications suivantes :
- Zhur. Préklad. Khim. 28, 969-75 (1955) (CA 50, 4881a, 1956)
- Tétrahédron 43, 1753 (1987)
- J. Org. 52, 2407 (1987)
- Zh. Org. Khim. 21, 2006 (1985)
- J. Fluor. Chem. 17, 345 (1981) ou dans les brevets :
- allemand DRP 637.318 (1935)
- européen EP 0.130.875
- japonais JP 81.121.524.

Les produits de formule (VI) qui sont des dérivés de l'hydantoïne sont largement utilisés et cités dans la littérature comme par exemple dans les articles suivants :
- J. Pharm. Pharmacol., 67, Vol. 19(4), p. 209-16 (1967)
- J. Chem. Soc., 74, (2), p. 219-21 (1972)
- Khim. Farm. Zh., 67, Vol. 1 (5) p. 51-2
- Brevet allemand 2.217.914
- Brevet européen 0.091.596
- J. Chem. Soc. Perkin. Trans. 1, 74 (2) p. 48, p. 219-21.

L'invention a également pour objet, à titre de produits industriels nouveaux et notamment à titre de produits industriels nouveaux utilisables comme intermédiaires pour la préparation des produits de formule générale (I).

Les produits de formule (IVi) : 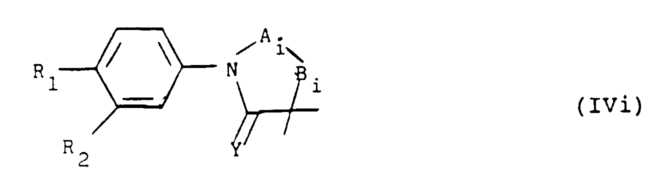 dans laquelle R_{**1**}, R_{**2**} et Y ont les significations indiquées ci-dessus et le groupement : 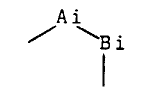 est choisi parmi les radicaux : 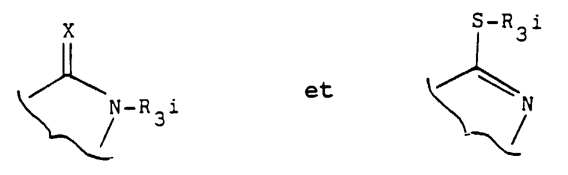 dans lesquels X représente un atome d'oxygène ou de soufre et R_{**3i**} est choisi parmi les valeurs de R_{**3**} comportant une fonction réactive protégée,
à l'exception des produits dans lesquels le groupement -A_{**i**}B_{**i**}- représente le radical : 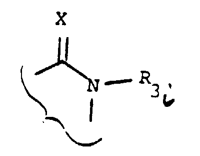 dans lequel X représente un atome d'oxygène et R_{**3i**} représente un atome d'hydrogène et Y représente un atome d'oxygène ou un radical NH et R_{**2**} représente un atome d'halogène ou un radical trifluorométhyle et R_{**1**} représente un radical nitro ou un atome d'halogène.

Parmi les fonctions réactives protégées on peut citer les fonctions hydroxyle et amino. Ces fonctions peuvent être protégées comme indiqué ci-dessus pour le substituant R_{**3**}.

Les exemples suivants illustrent l'invention sans toutefois la limiter.

### Exemple 1 : 1-(4-nitro-3-(trifluorométhyl) phényl)-3,4,4-triméthyl-2,5-imidazolidinedione.

A une suspension de 492 mg d'hydrure de sodium à 50 % dans l'huile et 3 cm^{**3**} de diméthyl formamide, on ajoute, à une température comprise entre 23 et 26°C, une solution de 3,17 g de 1-(3'-trifluorométhyl 4-nitrophényl) 4,4-diméthyl imidazoline 2,5-dione (obtenu selon BF 2.329.276) et 32 cm^{**3**} de diméthyl formamide, on agite 15 minutes et ajoute une solution de 0,7 cm^{**3**} d'iodure de méthyle dans 2 cm^{**3**} de diméthyl formamide. On agite 25 minutes entre 24 et 28°C puis verse sur 200 g d'un mélange 1-1 d'eau et de glace. On extrait avec de l'éther, lave à l'eau saturée de chlorure de sodium, sèche, filtre et évapore à sec sous pression réduite, on obtient 3,6 g du produit recherché F=116°C.

Un échantillon analytique a été obtenu par recristallisation dans l'alcool isopropylique on recueille ainsi 2,73 g du produit attendu F = 116°C.

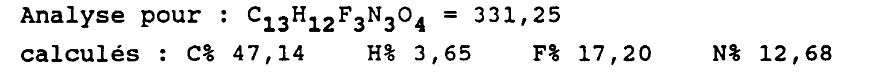

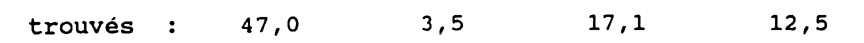

Spectre IR (CHCl_{**3**})
C=O (1780, 1727 cm^{**-1**})
aromatiques (1615, 1596, 1497 cm^{**-1**})
NO_{**2**} (1545, 1357 cm^{**-1**})

### Exemple 2 : 5,5-diméthyl-1-éthyl-3-(4-nitro-3-(trifluorométhyl) phényl)-2,4-imidazolidinedione.

On opère comme à l'exemple 1 à partir de 1 g de 1-(3'-trifluorométhyl 4'-nitrophényl) 4,4-diméthyl imidazoline-2,5-dione obtenu selon BF 2.329.276 en utilisant 0,33 cm^{**3**} d'iodure d'éthyle et 166 mg d'hydrure de sodium à 50 % dans l'huile. On obtient 1,19 g du produit recherché F = 110-111°C. Le produit ci-dessus est recristallisé dans l'isopropanol. On obtient 934 mg du produit attendu F = 110-111°C.

| Analyse pour C_{**14**}H_{**14**}F_{**3**}N_{**3**}O_{**4**} = 345,28 | | | | | | | | |
|---|---|---|---|---|---|---|---|---|
| calculés : | C% | 48,70 | H% | 4,09 | F% | 16,51 | N% | 12,17 |
| trouvés : | | 48,6 | | 4, 0 | | 16,8 | | 12,1 |

Spectre IR (CHCl_{**3**})
C=O (1777 cm^{**-1**}, 1724 (F))
NO_{**2**} (1545, 1356 cm^{**-1**})
aromatique (1614, 1596, 1497 cm^{**-1**})

### Exemple 3 : 5,5-diméthyl-3-(4-nitro-3-(trifluorométhyl) phényl)-1-propyl-2,4-imidazolidine-dione.

On opère comme à l'exemple 1 à partir de 1 g de 1-(3'-trifluorométhyl 4-nitrophényl) 4,4-diméthyl imidazoline 2,5-dione (obtenu selon BF 2.329.276) en utilisant 155 mg d'hydrure de sodium à 50 % dans l'huile et 0,35 cm^{**3**} de 1-iodo propane. Après chromatographie sur silice éluant acétone-chlorure de méthylène 1-99 on obtient 1,087 g de produit brut (F=102°C). Après recristallisation dans l'isopropanol, on recueille 945 mg de produit recherché (F = 102°C).

| Analyse pour C_{**15**}H_{**16**}F_{**3**}N_{**3**}O_{**4**} = 359,31 | | | | | | | | |
|---|---|---|---|---|---|---|---|---|
| calculés : | C% | 50,14 | H% | 4,49 | F% | 15,86 | N% | 11,69 |
| trouvés : | | 50,1 | | 4,4 | | 15,9 | | 11,5 |

spectre IR (CHCl_{**3**})
C=O (1778, 1724 cm^{**-1**})
NO_{**2**} (1544, 1358 cm^{**-1**})
aromatique (1615, 1596, 1497 cm^{**-1**})

### Exemple 4 : 5,5-diméthyl-1-(1-méthyl éthyl)-3-(4-nitro-3-(trifluorométhyl) phényl)-2,4-imidazolidinedione.

On opère comme à l'exemple 1 à partir de 1 g de 1-(3′-trifluorométhyl 4-nitrophényl) 4,4-diméthyl imidazoline 2,5-dione (obtenu selon BF 2.329.276) en utilisant 166 mg d'hydrure de sodium à 50 % dans l'huile et 0,4 cm^{**3**} de 2-iodo propane pendant 18 heures à 50°C. Après chromatographie sur silice (éluant chlorure de méthylène-acétone 99-1) on obtient 685 mg du produit attendu F = 130°C. On recristallise le produit obtenu ci-dessus dans l'isopropanol et on recueille 661 mg de produit recherché F = 130°C.

| Analyse pour C_{**15**}H_{**16**}F_{**3**}N_{**3**}O_{**4**} = 359,31 | | | | | | | | |
|---|---|---|---|---|---|---|---|---|
| calculés : | C% | 50,14 | H% | 4,49 | F% | 15,86 | N% | 11,69 |
| trouvés : | | 50,1 | | 4,4 | | 16,2 | | 11,6 |

Spectre IR (CHCl_{**3**})
C=O (1779, 1771, 1723 cm^{**-1**})
NO_{**2**} (1544, 1361 cm^{**-1**})
aromatiques (1615, 1596, 1497 cm^{**-1**})

### Exemple 5 : 5,5-diméthyl-3-(4-nitro-3-trifluorométhyl) phényl)-1-(2-propényl)-2,4-imidazolidinedione.

On opère comme à l'exemple 1 à partir de 1 g de 1-(3′-trifluorométhyl 4-nitrophényl) 4,4-diméthyl imidazoline 2,5-dione (obtenu selon BF 2.329.276) en utilisant 166 mg d'hydrure de sodium à 50 % dans l'huile et 0,35 cm^{**3**} de bromure d'allyle. Après chromatographie sur silice, éluant chlorure de méthylène-acétone 99-1, on obtient 1,19 g de produit que l'on recristallise dans l'isopropanol, on recueille 1,01 g de produit recherché F = 105°C.

| Analyse pour C_{**15**}H_{**14**}F_{**3**}N_{**3**}O_{**4**} = 357,29 | | | | | | | | |
|---|---|---|---|---|---|---|---|---|
| calculés : | C% | 50,42 | H% | 3,95 | F% | 15,95 | N% | 11,76 |
| trouvés : | | 50,4 | | 3,8 | | 15,8 | | 11,7 |

Spectre IR (CHCl_{**3**})
C=O (1779, 1724 cm^{**-1**})
NO_{**2**} (1545, 1358 cm^{**-1**})
aromatique (1615, 1596, 1497 cm^{**-1**})
CH=CH_{**2**} (1643, 930 cm^{**-1**})

### Exemple 6 : 5,5-diméthyl-3-(4-nitro-3-(trifluorométhyl) phényl)-1-méthyl phényl-2,4-imidazolidinedione.

On opère comme à l'exemple 1 à partir de 2 g de 1-(3′-trifluorométhyl 4-nitrophényl) 4,4-diméthyl imidazoline 2,5-dione (obtenu selon BF 2.329.276) en utilisant 332 mg d'hydrure de sodium à 50 % dans l'huile et 0,71 cm^{**3**} de bromure de benzyle. Après chromatographie sur silice, éluant chlorure de méthylène-acétone 99-1 on obtient 2,375 g de produit que l'on recristallise dans l'isopropanol, on recueille 2,165 g du produit recherché F = 99°C.

| Analyse pour C_{**19**}H_{**16**}N_{**3**}F_{**3**}O_{**4**} = 407,3 | | | | | | | | |
|---|---|---|---|---|---|---|---|---|
| calculés : | C% | 56,02 | H% | 3,96 | N% | 10,31 | F% | 14,00 |
| trouvés : | | 56,1 | | 3,8 | | 10,2 | | 13,9 |

Spectre IR
C=O (1799 cm^{**-1**} (m), 1723 cm^{**-1**} (F))
- aromatique + NO₂: (1608 cm^{**-1**})
(1594 cm^{**-1**} (m))
(1545 cm^{**-1**} (F))
(1497 cm^{**-1**})

### Exemple 7 : 4-(4,4-diméthyl-5-imino-2-oxo-1-imidazolidinyl)-2-(trifluorométhyl)-benzonitrile.

A une solution de 2,63 g de 2-amino-2-cyano propane et 36 cm^{**3**} de 1,2-dichloroéthane avec 0,9 cm^{**3**} de triéthylamine, on ajoute à 5°C, une solution de 6,6 g de 4-isocyanate de 2-(trifluorométhyl) benzonitrile préparé comme indiqué dans la préparation ci-après dans 10 cm^{**3**} de dichloréthane. On agite 16 heures à température ambiante. On évapore à secet chromatographie le résidu (7,7 g) sur silice, éluant chlorure de méthylène-acétone 85-15, on obtient 3,54 g du produit attendu F = 228°C.

Un échantillon analytique a été préparé par recristallisation de 300 mg du produit ci-dessus dans l'isopropanol, on recueille 267 mg de produit recherché F = 228°C.

| Analyse pour C_{**13**}H_{**11**}F_{**3**}N_{**4**}O = 296,25 | | | | | | | | |
|---|---|---|---|---|---|---|---|---|
| calculés : | C% | 52,71 | H% | 3,74 | F% | 19,24 | N% | 18,91 |
| trouvés : | | 52,7 | | 3,6 | | 19,1 | | 18,6 |

Spectre IR (nujol)
NH/OH (3340, 3290 cm^{**-1**})
C≡N 2240 cm^{**-1**}
C=O 1760 cm^{**-1**}
C=N 1655 cm^{**-1**}
aromatiques (1606, 1570, 1502 cm^{**-1**})

### Préparation : 4-isocyanate de 2-(trifluorométhyl) benzonitrile.

A 33,6 cm^{**3**} d'une solution toluénique de phosgène à 1,93 M/l portée à 0-5°C on ajoute en 20 minutes 10 g de 4-cyano 3-(trifluorométhyl) aniline (décrite dans le brevet européen EP 0.002.892) en solution dans 30 cm^{**3**} d'acétate d'éthyle. On agite 30 minutes à une température comprise entre 0 et 5°C puis laisse remonter à 25°C. On chauffe jusqu'à distillation en compensant le volume distillé par du toluène jusqu'à ce que la température de distillation atteigne 110°C. On met alors le réfrigérant en position reflux jusqu'à cessation du dégagement d'acide chlorhydrique (soit 4 heures 30). On ramène à température ambiante essore le léger insoluble blanc sous azote sur sulfate de sodium puis rince par trois fois 10 cm^{**3**} de toluène et évapore à sec sous pression réduite. On termine par un chauffage à 60°C pendant 1 heure puis revient sous atmosphère d'argon et obtient 11,6 g de produit attendu utilisé tel quel dans le stade suivant :
Infra-rouge
-N=C=O 2268 cm^{**-1**}
-CN 2233 cm^{**-1**}

### Exemple 8 : 4-(4,4-diméthyl-2,5-dioxo-1-imidazolidinyl)-2-(trifluorométhyl)-benzonitrile.

On chauffe 35 minutes au reflux une suspension de 2,76 g du produit obtenu à l'exemple 7 et 60 cm^{**3**} d'acide chlorhydrique au demi. On verse sur 100 g d'eau et glace et extrait avec de l'acétate d'éthyle. On lave à l'eau, sèche et évapore à sec. On obtient 2,70 g du produit recherché F = 210°C.

Un échantillon analytique a été obtenu par recristallisation de 440 mg du produit ci-dessus, dans l'isopropanol on recueille 383 mg de produit attendu F = 210-211°C.

| Analyse pour C_{**13**}H_{**10**}F_{**3**}N_{**3**}O_{**2**} = 297,24 | | | | | | | | |
|---|---|---|---|---|---|---|---|---|
| calculés : | C% | 52,53 | H% | 3,39 | F% | 19,17 | N% | 14,14 |
| trouvés : | | 52,4 | | 3,2 | | 19,4 | | 13,9 |

Spectre IR (CHCl_{**3**})
C≡N 2245 cm^{**-1**}
C=O (1788, 1722 cm^{**-1**})
aromatique (1610, 1572, 1502 cm^{**-1**})
NH (max) 3340 cm^{**-1**}

### Exemple 9 : Acide 3-(4-cyano-3-(trifluorométhyl) phényl)-5,5-diméthyl-2,4-dioxo-1-imidazolidine acétique.

A une suspension de 210 mg d'hydrure de sodium (à 50 % dans l'huile) et 3 cm^{**3**} de diméthyl formamide on ajoute une solution de 600 mg du produit obtenu à l'exemple 8 dans 6 cm^{**3**} de diméthyl formamide on agite 15 minutes puis ajoute 290 mg d'acide bromoacétique et agite 16 heures à température ambiante. On ajoute à nouveau 105 mg d'hydrure de sodium puis 15 minutes après 145 mg d'acide bromoacétique. On agite 30 minutes puis verse dans une solution composée de 50 cm^{**3**} d'eau et 5 cm^{**3**} d'acide chlorhydrique 2N. On extrait à l'éther, lave avec une solution saturée de chlorure de sodium, sèche, filtre et évapore à sec ; on obtient 1,22 g de produit brut que l'on chromatographie sur silice éluant chlorure de méthylèneméthanol-acide acétique (90-10-0,5). On obtient 367 mg du produit recherché.
Spectre IR : C≡N 2238 cm^{**-1**}
C=O hydantoïne et acide (1784, 1725, 1710 cm^{**-1**})
aromatique (1616, 1580, 1508 cm^{**-1**})
Ultra-violet
- EtOH Hcl 0,1 N: max 258 nm Epsilon = 13300
infl 277 nm Epsilon = 5000
infl 285 nm Epsilon = 2600
- EtOH NaOH 0,1 N: max 287 nm Epsilon = 19100
max 342 nm Epsilon = 1900

### Exemple 10 : 3-(4-cyano-3-(trifluorométhyl) phényl)-5,5-diméthyl-2,4-dioxo-1-imidazolidine acétate d'éthyle.

A une suspension de 100 mg d'hydrure de sodium à 50 % dans l'huile et 3 cm^{**3**} de diméthylformamide, on ajoute 600 mg du produit obtenu à l'exemple 8 en solution dans 6 cm^{**3**} de diméthyl formamide. On agite 15 minutes puis ajoute lentement sans dépasser 30°C 0,25 cm^{**3**} de bromoacétate d'éthyle. On agite 30 minutes, verse sur 50 g d'un mélange eau + glace (1-1), ajoute 0,5 g de phosphate monopotassique et extrait à l'éther. On lave la phase organique à l'eau, sèche et évapore à sec on recueille 1,1 g de produit brut que l'on chromatographie sur silice (éluant chlorure de méthylène-acétone (97-3)). On obtient 709 mg du produit attendu F = 152°C.

On a obtenu un échantillon analytique en recristallisant dans l'isopropanol le produit ci-dessus et recueilli ainsi 667 mg du produit recherché F = 152°C.

| Analyse pour C_{**17**}H_{**16**}F_{**3**}N_{**3**}O_{**4**} = 383,33 | | | | | | | | |
|---|---|---|---|---|---|---|---|---|
| calculés : | C% | 53,21 | H% | 4,21 | F% | 14,83 | N% | 10,96 |
| trouvés : | | 53,3 | | 4,0 | | 14,9 | | 10,8 |

Spectre IR (CHCl_{**3**})
C≡N 2225 cm^{**-1**}
imidazolidine (1786, 1729 cm^{**-1**})
CO_{**2**}Et 1751 cm^{**-1**}
aromatiques (1616, 1572, 1505 cm^{**-1**})

### Exemple 11 : 4-(5-imino-2-thioxo-3,4,4-triméthyl-1-imidazolidinyl)-2-(trifluorométhyl)-benzonitrile.

### a) Préparation de l'isothiocyanate

A une solution de 22 cm^{**3**} d'eau distillée et 1 cm^{**3**} de thiophosgène on ajoute lentement 2,23 g de 1-trifluorométhyl-4-amino benzonitrile (préparé selon EP 0002892) on agite pendant 1 heure, extrait avec du chloroforme, lave à l'eau salée, sèche et évapore à sec sous pression réduite on obtient 3 g de produit utilisé tel quel pour l'obtention de l'imine.

### b) Obtention de l'imine

On agite pendant 40 minutes au reflux 3 g du produit obtenu ci-dessus avec 1,33 cm^{**3**} de 2-méthylamino 2-cyanopropane, 23 cm^{**3**} de tétrahydrofuranne et 0,23 cm^{**3**} de triéthylamine. On évapore à sec et chromatographie le résidu (3,07 g) sur silice (éluant : cyclohexane-acétate d'éthyle (1-1) puis chlorure de méthylène-acétone (95-5)), on obtient 2,83 g de produit attendu que l'on recristallise dans l'isopropanol pour obtenir 2,63 g de produit recherché F = 173-174°C.

| Analyse pour C_{**14**}H_{**13**}F_{**3**}N_{**4**}S = 326,35 | | | | | | | | | | |
|---|---|---|---|---|---|---|---|---|---|---|
| calculés : | C% | 51,53 | H% | 4,01 | F% | 17,17 | N% | 17,46 | S% | 9,82 |
| trouvés : | | 51,7 | | 3,9 | | 17,2 | | 17,2 | | 9,9 |

Spectre IR
C=NH (3308, 1679 cm^{**-1**})
C=S + aromatiques (1608, 1575, 1505, 1488 cm^{**-1**})
C≡N 2230 cm^{**-1**}
CF_{**3**} = 1185 cm^{**-1**}

### Exemple 12 : 4-(5-oxo-2-thioxo-3,4,4-triméthyl-1-imidazolidinyl)-2-(trifluorométhyl)-benzonitrile.

On agite 1 heure au reflux 2,21 g du produit obtenu à l'exemple 11 et 44 cm^{**3**} d'acide chlorhydrique au demi. On verse le milieu réactionnel sur un mélange eau + glace (1-1) 200 g, extrait avec du chlorure de méthylène, lave avec de l'eau saturée de chlorure de sodium, sèche et évapore à sec, on chromatographie le résidu sur silice, éluant cyclohexaneacétate d'éthyle 1-1 on obtient 2,1 g de produit (F=171°C) que l'on recristallise dans l'isopropanol pour obtenir 1,99 g de produit recherché F = 171°C.

| Analyse pour C_{**14**}H_{**12**}F_{**3**}N_{**3**}OS = 327,33 | | | | | | | | | | |
|---|---|---|---|---|---|---|---|---|---|---|
| calculés : | C% | 51,37 | H% | 3,69 | F% | 12,84 | N% | 17,41 | S% | 9,79 |
| trouvés : | | 51,4 | | 3,5 | | 12,7 | | 17,6 | | 10,79 |

Spectre IR (CHCl_{**3**})
C=O (1761, 1756 cm^{**-1**})
aromatiques (1610, 1578, 1505 cm^{**-1**})
C≡N 2230 cm^{**-1**}
CF_{**3**} 1178 cm^{**-1**}

### Exemple 13 : 4-(2,5-dithioxo-3,4,4-triméthyl-1-imidazolidinyl)-2-(trifluorométhyl)-benzonitrile.

On agite 24 heures au reflux 839 mg de produit obtenu à l'exemple 12 avec 518 mg de réactif de Lawesson et 4,7 cm^{**3**} de toluène. On évapore à sec sous pression réduite on recueille 1,36 g de produit que l'on chromatographie sur silice éluant chlorure de méthylène-acétate d'éthyle (99-1) puis cyclohexane-acétate d'éthyle (85-15). On obtient 783 mg de produit que l'on recristallise dans l'isopropanol on recueille 690 mg de produit recherché F = 211-212°C.

| Analyse pour C_{**14**}H_{**12**}F_{**3**}N_{**3**}S_{**2**} = 343,40 | | | | | | | | | | |
|---|---|---|---|---|---|---|---|---|---|---|
| calculés : | C% | 48,97 | H% | 3,52 | F% | 16,60 | N% | 12,24 | S% | 18,67 |
| trouvés : | | 49,0 | | 3,4 | | 16,6 | | 12,2 | | 18,6 |

Spectre IR (CHCl_{**3**})
C≡N 2230 cm^{**-1**}
aromatique + syst. conjugué (1612, 1582, 1508 cm^{**-1**})
CF_{**3**} 1178 cm^{**-1**}

### Exemple 14 : 4-(4,4-diméthyl-5-imino-2-thioxo-1-imidazolidinyl)-2-(trifluorométhyl)-benzonitrile.

A un mélange de 2,54 g de produit obtenu comme en a) de l'exemple 11 avec 20 cm^{**3**} de tétrahydrofuranne et 0,2 cm^{**3**} de triéthylamine on ajoute 1 g de 2-amino-2-cyano propane et 1 cm^{**3**} de tétrahydrofuranne on agite à température ambiante. On évapore à sec et chromatographie le résidu (3,5 g) sur silice éluant acétate d'éthyle-cyclohexane (7-3) puis cyclohexaneacétate d'éthyle (1-1) et obtient 940 mg de produit recherché dont on recristallise 300 mg dans l'isopropanol pour recueillir 263 mg de produit F = 296°C.

| Analyse pour C_{**13**}H_{**11**}F_{**3**}N_{**4**}S = 312,32 | | | | | | | | | | |
|---|---|---|---|---|---|---|---|---|---|---|
| calculés : | C% | 50,00 | H% | 3,55 | F% | 18,25 | N% | 17,94 | S% | 10,27 |
| trouvés : | | 49,9 | | 3,4 | | 18,3 | | 17,6 | | 10,4 |

Spectre IR (Nujol)
OH/NH 3260 cm^{**-1**}
C≡N 2230 cm^{**-1**}
C=S 1764 cm^{**-1**}
aromatique + C=C (1612, 1575, 1530, 1501 cm^{**-1**})

Une nouvelle préparation du produit a été effectuée en remplaçant le tétrahydrofuranne par le 1,2-dichloroéthane.

Le produit attendu, insoluble, précipite. On obtient ainsi le produit recherché avec un rendement de 60 %.

### Exemple 15 : 4-(4,4-diméthyl-5-oxo-2-thioxo-1-imidazolidinyl)-2-(trifluorométhyl)-benzonitrile.

On agite pendant 1 heure au reflux 635 mg du produit obtenu à l'exemple 14 et 14 cm^{**3**} d'acide chlorhydrique dilué au demi. On refroidit, ajoute 100 cm^{**3**} d'eau et extrait avec de l'acétate d'éthyle, lave à l'eau salée, sèche et évapore à sec on obtient 600 mg de produit que l'on chromatographie sur silice éluant chlorure de méthylène-acétone (95-5) on obtient 590 mg du produit attendu (F = 190-191°C) que l'on recristallise dans l'isopropanol pour obtenir 490 mg du produit recherché F = 190-191°C.

| Analyse pour C_{**13**}H_{**10**}F_{**3**}N_{**3**}OS = 313,30 | | | | | | | | | | |
|---|---|---|---|---|---|---|---|---|---|---|
| calculés : | C% | 49,84 | H% | 3,22 | F% | 18,19 | N% | 13,41 | S% | 10,23 |
| trouvés : | | 49,6 | | 3,1 | | 18,4 | | 13,2 | | 10,0 |

Spectre IR (CHCl_{**3**})
=C-NH 3430 cm^{**-1**}
C≡N 2230 cm^{**-1**}
C=O 1766 cm^{**-1**}
Syst. conjugué + aromatiques (1612, 1578, 1505 cm^{**-1**})

### Exemple 16 : 5,5-diméthyl-3-(4-nitro-3-(trifluorométhyl) phényl)-1-pentyl-2,4-imidazolidinedione.

On opère comme à l'exemple 1 à partir de 1 g de 1-(3′-trifluorométhyl 4-nitrophényl) 4,4-diméthyl imidazoline-2,5-dione (obtenu selon BF 2.329.276) en utilisant 170 mg d'hydrure de sodium et 0,47 cm^{**3**} de 1-bromopentane, après chromatographie sur silice, éluant chlorure de méthylène-cyclohexane (8-2) on obtient 1,23 g de produit recherché que l'on cristallise dans l'isopropanol pour recueillir 995 mg de produit F = 84°C.

| Analyse pour C_{**17**}H_{**20**}O_{**4**}F_{**3**}N_{**3**} = 387,35 | | | | | | | | |
|---|---|---|---|---|---|---|---|---|
| calculés : | C% | 52,71 | H% | 5,20 | F% | 14,71 | N% | 10,85 |
| trouvés : | | 52,8 | | 5,1 | | 14,8 | | 10,7 |

Spectre IR (CHCl_{**3**})
C=O (1778, 1723 cm^{**-1**})
NO_{**2**} (1544, 1360 cm^{**-1**})

### Exemple 17 : 5,5-diméthyl-3-(4-nitro-3-(trifluorométhyl) phényl)-1-nonyl-2,4-imidazolidinedione.

On opère comme à l'exemple 1 à partir de 1 g de 1-(3′-trifluorométhyl 4-nitrophényl) 4,4-diméthyl imidazoline 2,5-dione (obtenu selon BF 2.329.276) en utilisant 170 mg d'hydrure de sodium à 50 % dans l'huile et 0,7 cm^{**3**} de 1-bromononane. Après chromatographie sur silice on obtient 1,08 g du produit recherché F = 63°C.

| Analyse pour C_{**21**}H_{**28**}O_{**4**}F_{**3**}N_{**3**} = 443,46 | | | | | | | | |
|---|---|---|---|---|---|---|---|---|
| calculés : | C% | 56,87 | H% | 6,36 | F% | 12,85 | N% | 9,48 |
| trouvés : | | 57,0 | | 6,5 | | 12,8 | | 9,5 |

Spectre IR (CHCl_{**3**})
C=O (1788, 1723 cm^{**-1**})
NO_{**2**} (1544, 1359 cm^{**-1**})

### Exemple 18 : 4-(3,4,4-triméthyl-2,5-dioxo 1-imidazolidinyl) 2-(trifluorométhyl) benzonitrile.

En opérant comme à l'exemple 1 au départ de 300 mg de produit décrit à l'exemple 8 on obtient 275 mg de produit attendu (F=158°C).
Spectre IR (CHCl_{**3**}) : C=O (1780, 1727 cm^{**-1**})
aromatiques : (1615, 1574, 1505 cm^{**-1**})
C≡N : 2238 cm^{**-1**}

### Exemple 19 : 4-(5-thioxo-2-oxo-3,4,4-triméthyl-1-imidazolidinyl) 2-(trifluorométhyl)-benzonitrile (produit A) 4-(5-oxo-2-thioxo-3,4,4-triméthyl 1-imidazolidinyl) 2-(trifluorométhyl)-benzonitrile (produit B) 4-(2,5-dithioxo-3,4,4-triméthyl-1-imidazolidinyl) 2-(trifluorométhyl)-benzonitrile (produit C).

On porte au reflux pendant 9 heures une suspension de 230 mg de produit obtenu à l'exemple 18 dans 1,4 cm^{**3**} de toluène et 78 mg de réactif de Lawesson, ramène à température ambiante puis évapore à sec. On purifie les 330 mg de produit obtenus par chromatographie sur silice (éluant chlorure de méthylène-acétone 99:1).

On obtient par ordre d'élution :
- 46 mg de produit C (Rf=0,63 F=210-211°C) identique au produit décrit à l'exemple 13 ;
- 26 mg de produit B (Rf=0,49 F=170-171°C) identique au produit décrit à l'exemple 12 ;
- 42 mg de produit A (Rf=0,34 F=194°C).

Analyse physique du produit A.
- Spectre IR (CHCl_{**3**}) :: C=O :1760 cm^{**-1**}
-C≡N : 2235 cm^{**-1**}
- aromatiques :: (1615, 1580, 1508 cm^{**-1**})
Spectre ultra-violet (éthanol)
max 228 nm epsilon = 19400
256 nm epsilon = 12100
298 nm epsilon = 8600
390 nm epsilon = 70

### Exemple 20 : 4-(4,5-dihydro 4,4-diméthyl 2-(méthylthio) 5-oxo 1H-imidazol-1-yl) 2-(trifluorométhyl)-benzonitrile.

On ajoute une solution de 626 mg de produit de l'exemple 15 dans 6 cm^{**3**} de diméthylformamide à une suspension constituée de 108 mg d'hydrure de sodium à 50 % dans l'huile et 1,8 cm^{**3**} de diméthylformamide. On rince avec 0,3 cm^{**3**} de diméthylformamide et agite pendant 10 minutes après cessation du dégagement d'hydrogène. On ajoute alors, goutte à goutte, 0,19 cm^{**3**} d'iodure de méthyle dans 1 cm^{**3**} de diméthylformamide.

Après 45 minutes de réaction on verse sur 50 g d'un mélange glace-eau contenant 0,5 g de phosphate monopotassique et extrait 4 fois à l'éther. On lave la phase organique à l'eau salée, sèche sur sulfate de magnésium et évapore à sec. On purifie les 668 mg de produit obtenu par chromatographie sur silice (éluant CH_{**2**}Cl_{**2**}-AcOEt 95:5).

On obtient 640 mg de produit que l'on chromatographie à nouveau sur silice (éluant cyclohexane-AcOEt 7:3) et obtient après reprise à l'éther 507 mg de produit recherché F = 62°C.
Spectre Infra-Rouge
C=O : 1747 cm^{**-1**}
C=N et aromatique (1614, 1581, 1569, 1503 cm^{**-1**})
Spectre Ultra-Violet (EtOH)
max 209 nm Epsilon = 26000
infl 236 nm Epsilon = 11500
infl 264 nm Epsilon = 8700

### Exemple 21 : 4-[4,5-dihydro 4,4-diméthyl 5-oxo 2-[(phénylméthyl) thio] 1H-imidazol-1-yl] 2-(trifluorométhyl) benzonitrile.

A 53 mg d'hydrure de sodium en suspension dans 0,5 cm^{**3**} de diméthylformamide, on ajoute en 5 minutes 313 mg de 4-(4,4**-**diméthy**l**-5-oxo-2-thioxo-1-imidazolidinyl)-2-(trifluorométhyl)-benzonitrile préparé comme à l'exemple 15 en solution dans 3 cm^{**3**} de diméthylformamide. On agite 10 minutes, ajoute 0,1 cm^{**3**} de bromure de benzyle et maintient 30 minutes sous agitation. On verse le milieu réactionnel dans l'eau glacée additionnée de 500 mg de phosphate de potassium, extrait à l'éther, lave la phase organique à l'eau salée, sèche et évapore le solvant. On obtient 450 mg de produit brut que l'on chromatographie sur silice (éluant : chlorure de méthylèneacétate d'éthyle 97,5-2,5). On recueille 316 mg de produit attendu. Rf = 0,38.

| Analyse | | | | | | | | |
|---|---|---|---|---|---|---|---|---|
| calculés : | C% | 59,54 | H% | 4,0 | F% | 14,12 | N% | 10,41 |
| trouvés : | | 59,6 | | 4,0 | | 14,1 | | 10,2 |

Spectre IR (CHCl_{**3**})
- C=O :: 1746 cm^{**-1**}
- C≡N :: 2236 cm^{**-1**}
- Système conjugué + aromatiques :: 1614, 1580, 1570, 1503, 1499 cm^{**-1**}

### Exemple 22 : 4-[4,4-diméthyl 3-(2-hydroxyéthyl) 5-imino 2-thioxo 1-imidazolidinyl] 2-(trifluorométhyl) benzonitrile.

On porte 30 minutes au reflux une solution comprenant 2,11 g de l'isothiocyanate préparé au stade a) de l'exemple 11 avec 1,18 g de mélange de 2-[(2-hydroxyéthyl) amino] 2-méthylpropane nitrile et de 2,2-diméthyloxazolidine (8-2) dans 20 cm^{**3**} de tétrahydrofuranne en présence de 0,5 cm^{**3**} de triéthylamine. On évapore le solvant, chromatographie le résidu sur silice (éluant : chlorure de méthylène-acétone 95-5) et obtient 1,26 g de produit attendu brut et 686 mg de N-[4-cyano 2-(trifluorométhyl) phényl] 2,2-diméthyl 3-oxazolidinecarbothioamide. On dissout les 686 mg de ce produit dans 10 cm^{**3**} d'acétate d'éthyle, ajoute 30 cm^{**3**} de cyclohexane, concentre à 4 cm^{**3**}, essore et sèche pour obtenir 518 mg de produit attendu supplémentaire. On dissout le produit brut dans 20 cm^{**3**} d'isopropanol, concentre à 5 cm^{**3**} essore et sèche. On obtient 1,04 g de produit attendu. F = 181°C.

| Analyse | | | | | | | | | | |
|---|---|---|---|---|---|---|---|---|---|---|
| calculés : | C% | 50,55 | H% | 4,24 | F% | 16,00 | N% | 15,72 | S% | 9,00 |
| trouvés : | | 50,4 | | 4,1 | | 15,9 | | 15,6 | | 9,0 |

Spectre IR (CHCl_{**3**})
- OH: : 3630 cm^{**-1**}
- >=NH: : 3314, 1677 cm^{**-1**}
- C≡N: : 2230 cm^{**-1**}
- aromatiques: : 1611, 1576, 1504 cm^{**-1**}

### Préparation du 2-[(2-hydroxyéthyl) amino] 2-méthylpropanenitrile utilisé au départ de l'exemple 22.

On ajoute goutte à goutte à une température comprise entre 20°C et 30°C, 8 cm^{**3**} d'éthanoline à 12,3 cm^{**3**} de cyanhydrine de l'acétone. On agite pendant 18 heures, distille sous pression réduite et recueille 2,3 g de mélange comprenant le produit attendu et du 2,2-diméthyloxazolidine, utilisé tel quel pour le stade suivant.

### Exemple 23 : 4-[4,4-diméthyl 3-(2-hydroxyéthyl) 5-oxo 2-thioxo 1-imidazolidinyl] 2-(trifluorométhyl) benzonitrile (Produit A) et 4-[4,4-diméthyl 2,5-dioxo 3-(2-mercaptoéthyl) 1-imidazolidinyl] 2-(trifluorométhyl) benzonitrile (Produit B).

On chauffe pendant 10 minutes au reflux 680 mg de produit obtenu à l'exemple 22 dans 7 cm^{**3**} d'eau en présence de 7 cm^{**3**} d'acide chlorhydrique, refroidit à température ambiante, extrait à l'acétate d'éthyle, lave la phase organique à l'eau salée, sèche et évapore le solvant. Après chromatographie du résidu sur silice (éluant : cyclohexane-acétate d'éthyle 1-1), on obtient 119 mg de produit B soit le dérivé 2,5-dioxo 3-(2-mercaptoéthyl) rf = 0,35 et 569 mg de produit A soit le dérivé 5-oxo 2-thioxo 3-(2-hydroxyéthyl) rf = 0,14; F ≈ 130°C.

| Analyse pour C_{**15**}H_{**14**}F_{**3**}N_{**3**}O_{**2**}S = 357,36 | | | | | | | | | | |
|---|---|---|---|---|---|---|---|---|---|---|
| calculés : | C% | 50,42 | H% | 3,95 | F% | 15,95 | N% | 11,76 | S% | 8,97 |
| Produit A : | | | | | | | | | | |
| trouvés : | | 50,7 | | 4,0 | | 15,7 | | 11,5 | | 9,1 |
| Produit B : | | | | | | | | | | |
| trouvés : | | 50,6 | | 3,8 | | 15,9 | | 11,6 | | 9,1 |

Spectre IR (CHCl_{**3**})
Produit A:
- OH: : 3626 cm^{**-1**}
- C≡N: : 2236 cm^{**-1**}
- C=O: : 1763 cm^{**-1**}
- aromatiques: : 1615, 1578, 1504 cm^{**-1**}
Produit B :
Absence d'OH
- C≡N: : 2228 cm^{**-1**}
- C=O: : 1780, 1726 cm^{**-1**}
- aromatiques: : 1615, 1578, 1505 cm^{**-1**}

En opérant comme indiqué dans les exemples 1 à 23 :
A) en utilisant le 4-(4,4-diméthyl 2,5-dioxo 1-imidazolidinyl) 2-(trifluorométhyl) benzonitrile préparé comme à l'exemple 8 et les réactifs appropriés, on a obtenu les composés des exemples suivants :

### Exemple 24 : 4-(4,4-diméthyl 2,5-dioxo 3-éthyl 1-imidazolidinyl) 2-(trifluorométhyl) benzonitrile.

F= 100-101°C.

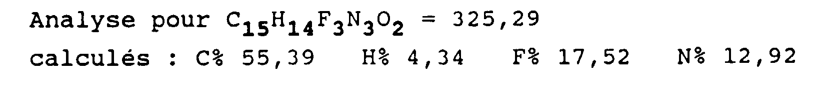

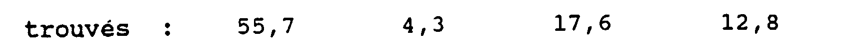

Spectre IR (CHCl_{**3**})
- C≡N: : 2238 cm^{**-1**}
- C=O: : 1777, 1724 cm^{**-1**}
- aromatiques: : 1617, 1575, 1505 cm^{**-1**}

### Exemple 25 : 4-(4,4-diméthyl 2,5-dioxo 3-(2-propényl) 1-imidazolidinyl) 2-(trifluorométhyl) benzonitrile.

F = 109-110°C.

| Analyse pour C_{**16**}H_{**14**}F_{**3**}N_{**3**}O_{**2**} = 337,35 | | | | | | | | |
|---|---|---|---|---|---|---|---|---|
| calculés : | C% | 56,97 | H% | 4,18 | F% | 16,90 | N% | 12,46 |
| trouvés : | | 57,0 | | 4,1 | | 16,2 | | 12,3 |

Spectre IR (CHCl_{**3**})
- C≡N: : 2238 cm^{**-1**}
- C=O: : 1728, 1725 cm^{**-1**}
- HC=CH_{**2**}: : 1645 cm^{**-1**}
- aromatiques: : 1616, 1575, 1505 cm^{**-1**}

### Exemple 26 : 4-(4,4-diméthyl 2,5-dioxo 3-(phénylméthyl) 1-imidazolidinyl) 2-(trifluorométhyl) benzonitrile.

F = 98-99°C.

| Analyse pour C_{**20**}H_{**16**}F_{**3**}N_{**3**}O_{**2**} = 387,36 | | | | | | | | |
|---|---|---|---|---|---|---|---|---|
| calculés : | C% | 62,01 | H% | 4,16 | F% | 14,71 | N% | 10,85 |
| trouvés : | | 62,0 | | 4,1 | | 14,7 | | 10,8 |

Spectre IR (CHCl_{**3**})
- =C-NH: : 3430 cm^{**-1**}
- C≡N: : 2238 cm^{**-1**}
- C=O: : 1779, 1724 cm^{**-1**}
- aromatiques: : 1615, 1605, 1575, 1504, 1497 cm^{**-1**}

### Exemple 27 : 4-[4,4-diméthyl 2,5-dioxo 3-[(4-fluorophényl) méthyl] 1-imidazolidinyl] 2-(trifluorométhyl) benzonitrile.

F = 101-102°C.

| Analyse pour C_{**20**}H_{**15**}F_{**4**}N_{**3**}O_{**2**} = 405,35 | | | | | | | | |
|---|---|---|---|---|---|---|---|---|
| calculés : | C% | 59,26 | H% | 3,73 | F% | 18,75 | N% | 10,37 |
| trouvés : | | 59,1 | | 3,5 | | 18,9 | | 10,3 |

Spectre IR (CHCl_{**3**})
- C≡N: : 2238 cm^{**-1**}
- C=O: : 1780, 1724 cm^{**-1**}
- aromatiques: : 1615, 1612, 1505 cm^{**-1**}

### Exemple 28 : 4-[4,4-diméthyl 2,5-dioxo 3-[(4-méthoxyphényl) méthyl] 1-imidazolidinyl] 2-(trifluorométhyl) benzonitrile.

F = 95-96°C.

| Analyse pour C_{**21**}H_{**18**}F_{**3**}N_{**3**}O_{**3**} = 417,39 | | | | | | | | |
|---|---|---|---|---|---|---|---|---|
| calculés : | C% | 60,43 | H% | 4,35 | F% | 13,65 | N% | 10,07 |
| trouvés : | | 59,1 | | 3,5 | | 18,9 | | 10,3 |

Spectre IR (CHCl_{**3**})
- C≡N: : 2238 cm^{**-1**}
- C=O: : 1778, 1723 cm^{**-1**}
- aromatiques: : 1615, 1584, 1514, 1505 cm^{**-1**}

### Exemple 29 : 4-[4,4-diméthyl 2,5-dioxo 3-[[4-[trifluorométhyl) phényl] méthyl] 1-imidazolidinyl] 2-(trifluorométhyl) benzonitrile.

F ≈ 89-90°C.

| Analyse pour C_{**21**}H_{**15**}F_{**6**}N_{**3**}O_{**2**} = 313,30 | | | | | | | | |
|---|---|---|---|---|---|---|---|---|
| calculés : | C% | 55,39 | H% | 3,32 | F% | 25,03 | N% | 9,23 |
| trouvés : | | 55,2 | | 3,2 | | 25,3 | | 9,2 |

Spectre IR (CHCl_{**3**})
- C≡N: : 2238 cm^{**-1**}
- C=O: : 1615, 1505 cm^{**-1**}
- aromatiques: : 1615, 1505 cm^{**-1**}

### Exemple 30 : 4-[4,4-diméthyl 2,5-dioxo 3-(2-époxyméthyl) 1-imidazolidinyl] 2-(trifluorométhyl) benzonitrile.

F =112-113°C.

| Analyse pour C_{**16**}H_{**14**}F_{**3**}N_{**3**}O_{**3**} = 353,30 | | | | | | | | |
|---|---|---|---|---|---|---|---|---|
| calculés : | C% | 54,39 | H% | 3,99 | F% | 16,13 | N% | 11,89 |
| trouvés : | | 54,7 | | 4,0 | | 16,1 | | 11,8 |

Spectre IR (CHCl_{**3**})
- C≡N: : 2235 cm^{**-1**}
- C=O: : 1781, 1725 cm^{**-1**}
- aromatiques: : 1615, 1576,1505 cm^{**-1**}

### Exemple 31 : 4-(4,4-diméthyl 2,5-dioxo 3-propyl-1H-imidazolidinyl) 2-(trifluorométhyl) benzonitrile.

F = 113-114°C.

| Analyse pour C_{**16**}H_{**16**}F_{**3**}N_{**3**}O_{**2**} = 339,32 | | | | | | | | |
|---|---|---|---|---|---|---|---|---|
| calculés : | C% | 56,64 | H% | 4,75 | F% | 16,80 | N% | 12,38 |
| trouvés : | | 56,7 | | 4,7 | | 16,7 | | 12,2 |

Spectre IR (CHCl_{**3**})
- C≡N: :2236 cm^{**-1**}
- C=O: : 1778, 1725 cm^{**-1**}
- aromatiques: : 1616, 1505 cm^{**-1**}

### Exemple 32 : 4-(4,4-diméthyl 2,5-dioxo 3-(1-méthyléthyl) 1-imidazolidinyl] 2-(trifluorométhyl) benzonitrile.

F = 138-139°C.

| Analyse pour C_{**16**}H_{**16**}F_{**3**}N_{**3**}O_{**2**} = 339,32 | | | | | | | | |
|---|---|---|---|---|---|---|---|---|
| calculés : | C% | 56,64 | H% | 4,75 | F% | 16,80 | N% | 12,38 |
| trouvés : | | 56,5 | | 4,7 | | 17,1 | | 12,3 |

Spectre IR (CHCl_{**3**})
- C≡N: : 2236 cm^{**-1**}
- C=O: : 1778, 1724 cm^{**-1**}
- aromatiques: : 1616, 1575, 1505 cm^{**-1**}
B) En utilisant le 4-(4,4-diméthyl 5-oxo 2-thioxo 1-imidazolidinyl 2-(trifluorométhyl) benzonitrile préparé comme à l'exemple 15 et les réactifs appropriés, on a obtenu les composés suivants :

### Exemple 33 : 4-[4,5-dihydro 4,4-diméthyl 2-(nonylthio) 5-oxo 1H-imidazol-1-yl] 2-(trifluorométhyl) benzonitrile.

rf = 0,35 (éluant : chlorure de méthylène-acétate d'éthyle 97,5-2,5).

### Exemple 34 : 4-[4,5-dihydro 4,4-diméthyl 2-[(3-hydroxypropyl) thio] 5-oxo 1H-imidazol-1-yl] 2-(trifluorométhyl) benzonitrile.

rf = 0,17 (éluant : chlorure de méthylène-acétate d'éthyle 8-2).

### Exemple 35 : [[1-[4-cyano 3-(trifluorométhyl) phényl] 4,5-dihydro 4,4-diméthyl 5-oxo 1H-imidazol-2-yl] thio] acétate d'éthyle.

rf = 0,20 (éluant : cyclohexane-acétate d'éthyle 65-35).
C) En utilisant le thiocyanate préparé à l'exemple 11 et les réactifs appropriés, on a obtenu les composés suivants :

### Exemple 36 : 4-(4,4-diméthyl 3-éthyl 5-imino 2-thioxo 1-imidazolidinyl) 2-(trifluorométhyl) benzonitrile.

rf = 0,16 (éluant : chlorure de méthylène-acétone 95-5).

### Exemple 37 : 4-(4,4-diméthyl 5-imino 3-pentyl 2-thioxo 1-imidazolidinyl) 2-(trifluorométhyl) benzonitrile.

rf = 0,35 (éluant : acétate d'éthyle-cyclohexane 8-2).
D) En utilisant respectivement le 4-(4,4-diméthyl 3-éthyl 5-imino 2-thioxo 1-imidazolidinyl) 2-(trifluorométhyl) benzonitrile préparé comme à l'exemple 36 et le 4-(4,4-diméthyl 5-imino 3-pentyl 2-thioxo 1-imidazolidinyl) 2-(trifluorométhyl) benzonitrile préparé comme à l'exemple 37 et l'acide chlorhydrique au demi, on a obtenu les composés suivants :

### Exemple 38 : 4-(4,4-diméthyl 3-éthyl 5-oxo 2-thioxo 1-imidazolidinyl) 2-(trifluorométhyl) benzonitrile.

rf = 0,38 (éluant : acétate d'éthyle-cyclohexane 1-1).

### Exemple 39 : 4-(4,4-diméthyl 5-oxo 3-pentyl 2-thioxo 1-imidazolidinyl) 2-(trifluorométhyl) benzonitrile.

F = 78°C. rf = 0,66 (éluant : acétate d'éthyle-cyclohexane 8-2).
E) En utilisant le 4-(4,5-dihydro 4,4-diméthyl 2-(méthylthio) 5-oxo 1H-imidazol-1-yl) 2-(trifluorométhyl)-benzonitrile préparé comme à l'exemple 20 et le 4-[4,5-dihydro 4,4-diméthyl 5-oxo 2-[(phénylméthyl) thio] 1H-imidazol-1-yl] 2-(trifluorométhyl) benzonitrile préparé comme à l'exemple 21 et le réactif de Lawesson, on a obtenu les composés suivants :

### Exemple 40 : 4-[4,5-dihydro 4,4-diméthyl 2-(méthylthio) 5-thioxo 1H-imidazol-1-yl] 2-(trifluorométhyl) benzonitrile.

rf = 0,36 (éluant : chlorure de méthylène-acétate d'éthyle 97,5-2,5).

### Exemple 41 : 4-[4,5-dihydro 4,4-diméthyl 2-[(phénylméthyl) thio] 5-thioxo 1H-imidazol-1-yl] 2-(trifluorométhyl) benzonitrile.

rf = 0,62 (éluant : chlorure de méthylène-acétate d'éthyle 98-2).

### Exemple 42 : 3-[4-cyano 3-(trifluorométhyl) phényl] 5,5-diméthyl 2,4-dioxo N-méthyl N-(1-méthyléthyl) 1-imidazolidine acétamide.

On ajoute 0,1 cm^{**3**} de N-méthylmorpholine à 235 mg d'acide 3-[4-cyano 3-(trifluorométhyl) phényl] 5,5-diméthyl 2,4-dioxo 1-imidazolidine acétique préparé comme à l'exemple 9, en suspension dans 4 cm^{**3**} de chlorure de méthylène. On refroidit la solution obtenue à -10°C, ajoute goutte à goutte 0,1 cm^{**3**} de chloroformiate d'isobutyle et agite 25 minutes à -10°C. On ajoute 0,15 cm^{**3**} de N-méthyl N-isopropylamine, laisse revenir à température ambiante en 40 minutes environ, ajoute 5 cm^{**3**} d'une solution aqueuse saturée en bicarbonate de sodium, agite 30 minutes, extrait au chlorure de méthylène, lave la phase organique à l'eau, sèche et évapore le solvant sous pression réduite. Après chromatographie sur silice (éluant : chlorure de méthylène-acétone 96-4), on obtient 147 mg de produit attendu.
Spectre IR (CHCl_{**3**})
- C≡N: : 2236 cm^{**-1**}
- C=O hydantoïne: : 1783, 1728 cm^{**-1**}
- C=O amide: : 1661 cm^{**-1**}
- aromatiques: : 1615, 1575, 1505 cm^{**-1**}

### Exemple 43 : 4,-[4,4-diméthyl 2,5-dioxo 3-(2-hydroxyéthyl) 1-imidazolidinyl] 2-(trifluorométhyl) benzonitrile.

### a) Condensation.

On opère comme à l'exemple 9 à partir de 900 mg de produit obtenu à l'exemple 8 et 1,91 g de 2-bromoéthanolterbutyl-diméthylsilyléther. On obtient 1 g de l'éther silyloxy dérivé. F = 86-87°C après chromatographie sur silice (éluant : cyclohexane-acétate d'éthyle 7-3).

### b) Clivage :

On ajoute 1 cm^{**3**} d'acide chlorhydrique 2N à 380 mg de produit obtenu ci-dessus en solution dans 4 cm^{**3**} de méthanol et 1 cm^{**3**} de chlorure de méthylène. On agite 40 minutes à température ambiante, verse sur 15 cm^{**3**} d'eau, extrait au chlorure de méthylène, lave à l'eau, sèche et évapore le solvant. On purifie le résidu par chromatographie sur silice (éluant : chlorure de méthylène-acétate d'éthyle 7-3), rf = 0,9, cristallise dans l'éther et recueille 270 mg de produit attendu. F = 109-110°C après cristallisation dans l'isopropanol.

| Analyse : | | | | | | | | |
|---|---|---|---|---|---|---|---|---|
| calculés : | C% | 52,79 | H% | 4,23 | F% | 16,70 | N% | 12,31 |
| trouvés : | | 52,5 | | 4,2 | | 16,7 | | 12,1 |

En opérant de manière identique, en utilisant au départ le 2-bromopropanoltertubyldiméthylsilyléther, on a préparé le produit suivant :

### Exemple 44 : 4-[4,4-diméthyl 2,5-dioxo 3-(3-hydroxypropyl) 1-imidazolidinyl] 2-(trifluorométhyl) benzonitrile.

F = 131-132°C. Rf = 0,13 (éluant : CH_{**2**}Cl_{**2**}-AcOEt 75-25).

### Exemple 45 : 4-[3-[2-(acétyloxy) éthyl] 4,4-diméthyl 2,5-dioxo 1-imidazolidinyl] 2-(trifluorométhyl) benzonitrile.

On agite 30 minutes à température ambiante, 215 mg de produit obtenu à l'exemple 43, 15 mg de 4-diméthylaminopyridine, 1 cm^{**3**} de pyridine et 0,5 cm^{**3**} d'anhydride acétique. On verse le milieu réactionnel dans 20 cm^{**3**} d'une solution aqueuse saturée en bicarbonate de sodium, agite 20 minutes, extrait à l'acétate d'éthyle, lave à l'eau, évapore à sec. On élimine la pyridine et l'acide acétique résiduel par distillation, purifie le résidu par chromatographie sur silice (éluant : chlorure de méthylène-acétate d'éthyle 65-35), reprend le résidu (rf = 0,35) à l'isopropanol, concentre partiellement, glace, essore et obtient après séchage, 210 mg de produit attendu. F = 99-100°C.

| Analyse : | | | | | | | | |
|---|---|---|---|---|---|---|---|---|
| calculés : | C% | 53,27 | H% | 4,21 | F% | 14,87 | N% | 10,96 |
| trouvés : | | 53,5 | | 4,3 | | 15,2 | | 10,9 |

En opérant comme dans les exemples précédents, on a préparé les exemples des produits suivants :

### Exemple 46 : 4-[4,4-diméthyl 2,5-dioxo 3-(5-hydroxypentyl) 1-imidazolidinyl] 2-(trifluorométhyl) benzonitrile.

F = 101-102°C.

### Exemple 47 : 4-[4,4-diméthyl 2,5-dioxo 3-(2-méthoxyéthyl) 1-imidazolidinyl] 2-(trifluorométhyl) benzonitrile.

F = 68-69°C.

### Exemple 48 : 4-[4,4-diméthyl 2,5-dioxo 3-(cyanométhyl) 1-imidazolidinyl] 2-(trifluorométhyl) benzonitrile.

F = 186-187°C.

### Exemple 49 : 4-[4,4-diméthyl 2,5-dioxo 3-[(1,3-dioxalan-2-yl) méthyl] 1-imidazolidinyl] 2-(trifluorométhyl) benzonitrile.

F = 135-136°C.

### Exemple 50 : 4-[4,4-diméthyl 2,5-dioxo 3-(2-chloroéthyl) 1-imidazolidinyl] 2-(trifluorométhyl) benzonitrile.

F = 120-121°C.

### Exemple 51 : 1-(3,4-dichlorophényl) 5-imino 3,4,4-triméthyl 2-imidazolidine thione.

On chauffe 16 heures au reflux 2,4 g d'isocyanate de 3,4-dichlorophényle et 1,3 cm^{**3**} de 2-méthylamino 2-cyanopropane dans 23 cm^{**3**} de tétrahydrofuranne en présence de 0,23 cm^{**3**} de triéthylamine. On élimine le solvant sous pression réduite et purifie le résidu par chromatographie sur silice (éluant : chlorure de méthylène-acétone 96-4 puis acétate d'éthylecyclohexane 1-1). Après cristallisation dans l'éther, on obtient 2,54 g de produit attendu. F = 133°C.

### Exemple 52 : 3-(3,4-dichlorophényl) 2-thioxo 1,5,5-triméthyl 4-imidazolidinone.

On chauffe au reflux 45 minutes 1,88 g de produit obtenu à l'exemple 51 en suspension dans 14 cm^{**3**} d'acide chlorhydrique 6N puis ajoute de nouveau 14 cm^{**3**} d'acide chlorhydrique 6N et poursuit le chauffage pendant 2 heures. Après une nouvelle addition de 4 cm^{**3**} d'acide chlorhydrique 6N et chauffage au reflux pendant 1 heure et demie, on laisse revenir à température ambiante, ajoute 100 g de glace et extrait à l'acétate d'éthyle. On lave la phase organique à l'eau, sèche et évapore le solvant. Après chromatographie sur silice (éluant : cyclohexane-acétate d'éthyle 1-1), on obtient 1,84 g de produit attendu. F = 129°C après cristallisation dans l'isopropanol.

| Analyse pour C_{**12**}H_{**12**}Cl_{**2**}N_{**2**}OS = 303,21 | | | | | | | | | | |
|---|---|---|---|---|---|---|---|---|---|---|
| calculés : | C% | 47,54 | H% | 3,99 | Cl% | 23,38 | N% | 9,24 | S% | 10,57 |
| trouvés : | | 47,5 | | 3,8 | | 23,2 | | 9,3 | | 10,5 |

Spectre IR (CHCl_{**3**})
- C=O: : 1753 cm^{**-1**}
- C=S et aromatiques: : 1595, 1570, 1496 cm^{**-1**}

En opérant comme dans les exemples précédents en utilisant les produits et réactifs appropriés, on a préparé les composés suivants :

### Exemple 53 : 3-(3,4-dichlorophényl) 3,5-dihydro 5,5-diméthyl, 2-(méthylthio) 4H-imidazol-4-one.

F = 110°C.

### Exemple 54 : 1-(3,4-dichlorophényl) 3,4,4-triméthyl 2,5-imidazolidine dithione.

F ≈ 146°C.

### Exemple 55 : 1-[4-chloro 3-(trifluorométhyl) phényl] 4,4-diméthyl 2-thioxo 5-imidazolidinone.

F = 176°C.

### Exemple 56 : 1-[4-chloro 3-(trifluorométhyl) phényl] 4,4-diméthyl 5-imino 2-imidazolidine thione.

F = 173-174°C.

### Exemple 57 : 3-(3,4-dichlorophényl) 3,5-dihydro 5,5-diméthyl 2-[(phénylméthyl) thio] 4H-imidazol-4-one.

Spectre IR (CHCl_{**3**})
- C=O: : 1736 cm^{**-1**}
- C=N et aromatiques: : 1578, 1496 cm^{**-1**}

En plus des produits décrits ci-dessus, les produits suivants constituent des produits pouvant être obtenus dans le cadre de la présente invention, à savoir les produits de formule : 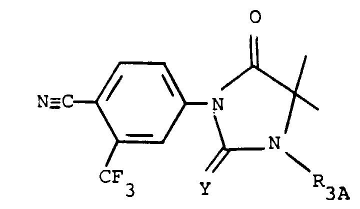 dans laquelle Y_{**A**} représente un atome d'oxygène ou de soufre et R_{**3A**} a les valeurs suivantes :

. -(CH_{**2**})_{**n**}Cl

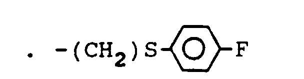

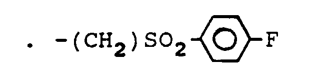

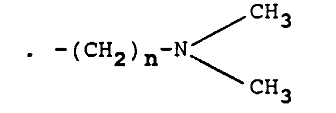

. -(CH_{**2**})_{**n**}-OH

. (CH_{**2**})_{**n**}-COO-alk

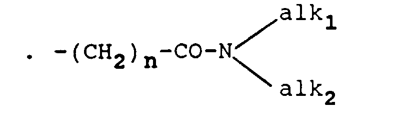

. -(CH_{**2**})_{**n**}-CO-alk

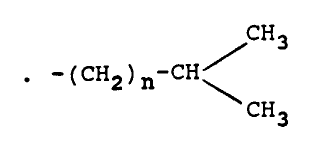

. -(CH_{**2**})_{**n**}-C**≡**N

alk, alk_{**1**} et alk_{**2**} représentant un radical alkyle renfermant jusqu'à 4 atomes de carbone et n représente un nombre entier compris entre 1 et 4.

### Exemple 58 :

On a préparé un comprimé ayant la composition suivante :
- 4-(5-oxo-2-thioxo-3,4,4-triméthyl 1-imidazolinyl)-2-(trifluorométhyl) benzonitrile 100 mg
- Excipient q.s. pour un comprimé terminé à 300 mg
(Détail de l'excipient : lactose, amidon, talc, stéarate de magnésium).

### Etude pharmacologique des produits de l'invention

### 1) Etude de l'affinité des produits de l'invention pour le récepteur androgène

### Récepteur androgène.

Des rats mâles Sprague Dawley EOPS de 180-200 g, castrés de 24 heures, sont sacrifiés, les prostates prélevées, pesées et homogénéisées à 0°C à l'aide d'un potter verre-verre, dans une solution tamponnée (Tris 10mM, saccharose 0,25M, PMSF (phénylméthanesulfonylfluoride) 0,1mM, Molybdate de sodium 20mM, HCl pH 7,4 ; auxquels on ajoute extemporanément 2mM de DTT (DL dithiothreitol), à raison de 1 g de tissu pour 8 ml de tampon.

L'homogénat est ensuite ultracentrifugé à 0°C, 45 minutes à 105 000 g. Des aliquotes du surnageant obtenu (=cytosol), sont incubées 30 minutes et 24 heures à 0°C, avec une concentration constante (T) de Testostérone tritiée et en présence de concentrations croissantes (0 à 2500.10^{**-9**}M), soit de testostérone froide, soit des produits à tester. La concentration de Testostérone tritiée liée (B) est ensuite mesurée dans chaque incubat par la méthode d'adsorption au charbon-dextran.

### Calcul de l'affinité relative de liaison (ARL).

On trace les 2 courbes suivantes : le pourcentage de l'hormone tritiée liée B/T en fonction du logarithme de la concentration de l'hormone de référence froide et B/T en fonction du logarithme de la concentration du produit froid testé. On détermine la droite d'équation I_{**50**}=(B/Tmax + B/Tmin)/2.
B/T max= % de l'hormone tritiée liée pour une incubation de cette hormone tritiée à la concentration (T).
B/T min= % de l'hormone tritiée liée pour une incubation de cette hormone tritiée à la concentration (T) en présence d'un grand excès d'hormone froide (2500.10^{**-9**}M).

Les intersections de la droite I_{**50**} et des courbes, permettent d'évaluer les concentrations de l'hormone de référence froide (CH) et du produit froid testé (CX) qui inhibent de 50 % la liaison de l'hormone tritiée sur le récepteur. L'affinité relative de liaison (ARL) du produit testé est déterminé par l'équation ARL=100 (CH)/(CX).

On obtient les résultats suivants exprimés en ARL.

Produit de référence (Testostérone) : 100

| | Incubation : 30 minutes | Incubation : 24 heures |
|---|---|---|
| Produit de l'exemple 1 | 27,5 | 3 |
| Produit de l'exemple 2 | 22 | 6 |
| Produit de l'exemple 4 | 21 | 5 |
| Produit de l'exemple 11 | 28 | 8 |
| Produit de l'exemple 12 | 128 | 92 |
| Produit de l'exemple 13 | 31 | 39 |
| Produit de l'exemple 14 | 27 | 7 |
| Produit de l'exemple 15 | 69 | 24 |

### 2) Détermination de l'activité androgène ou anti-androgène des produits de l'invention à l'aide du dosage de l'ornithine de carboxylase.

### - Protocole de traitement

Des souris mâles SWISS âgées de 6 semaines, et castrées de 24 heures, reçoivent par voie orale les produits à étudier (suspension en méthyl cellulose à 0,5 %), simultanément avec une injection sous-cutanée de Propionate de testostérone 3 mg/kg (solution en huile de sésame, contenant 5 % d'alcool benzylique) pour déterminer l'activité anti-androgène. L'activité agoniste est déterminèe en l'absence de propionate de testostérone.

Les produits à étudier ainsi que le Propionate de testostérone sont administrés sous un volume de 10 ml/kg.

16 heures après les traitements, les animaux sont sacrifiés, les reins prélevés, puis homogénéisés à 0°C , à l'aide d'un broyeur téflon-verre dans 10 volumes de tampon Tris-HCl 50 mM (pH 7,4) contenant 250 uM de phosphate de pyridoxal, 0,1 mM EDTA, et 5 mM de dithiothreitol. L'homogenat est ensuite centrifugé à 105000 g pendant 45 mn.

### - Principe de dosage

A 37°C, l'ornithine décarboxylase rénale transforme un mélange isotopique d'ornithine froide et d'ornithine tritiée en putrescine froide et putrescine tritiée.

La putrescine est ensuite recueillie sur des papiers sélectifs, échangeurs d'ions. Après séchage, l'excès d'ornithine tritiée et froide non transformée est éliminé, par 3 lavages d'ammoniaque 0,1 M. Les papiers sont séchés, puis la radioactivité est comptée après addition de scintillant Aqualite.

Les résultats sont exprimés en fmoles (10^{**-15**} M) de putrescine tritiée formée/heure/mg de protéines.

On obtient les résultats suivants :

| | | |
|---|---|---|
| Produit de l'exemple 11 | : Antagonisme (PO) | 3 mg/kg : 83 % |
| Produit de l'exemple 12 | : Antagonisme (PO) | 0,1 mg/kg : 12 % |
| | | 0,3 mg/kg : 36 % |
| | | 1 mg/kg : 68 % |
| | | 3 mg/kg : 94% |
| | | 10 mg/kg : 99% |
| | : Angonisme (PO) | 10 mg/kg : 0 % |
| Produit de l'exemple 14 | : Antagonisme (PO) | 3 mg/kg : 87 % |
| Produit de l'exemple 15 | : Antagonisme (PO) | 0,3 mg/kg : 4 % |
| | | 1 mg/kg : 82 % |

### Conclusion :

Les tests indiqués ci-dessus montrent que les produits de l'invention testés possèdent une forte activité anti-androgène et sont dénués d'activité agoniste.

## Revendications (Revendications pour l'(les) Etat(s) contractant(s) suivant(s): AT, BE, CH, DE, DK, FR, GB, IT, LI, LU, NL, PT, SE)

1. Les produits de formule générale (I) : 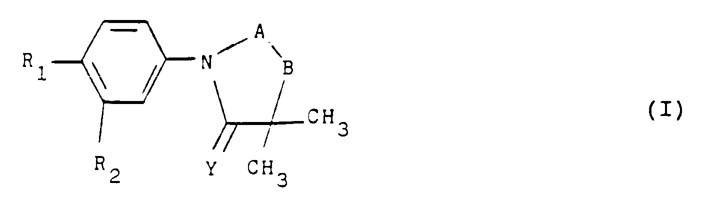 dans laquelle :
R_{**1**} représente un radical cyano ou nitro ou un atome d'halogène,
R_{**2**} représente un radical trifluorométhyle ou un atome d'halogène,
le groupement -A-B- est choisi parmi les radicaux 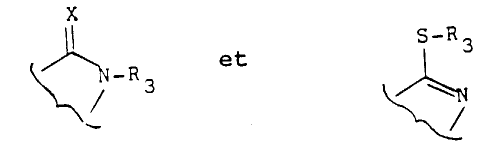 dans lesquels X représente un atome d'oxygène ou de soufre et
R_{**3**} est choisi parmi les radicaux suivants :
- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, S-aryle éventuellement substitué, dans lequel l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,
les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone,
les radicaux aryle et aralkyle étant de plus éventuellement substitués par un radical alkyle, alkényle ou alkynyle, alkoxy, alkényloxy, alkynyloxy ou trifluorométhyle, Y représente un atome d'oxygène ou de soufre ou un radical =NH,
à l'exception des produits dans lesquels le groupement -A-B- représente le radical : 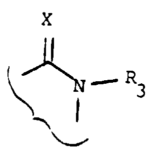 dans lequel X représente un atome d'oxygène et R_{**3**} représente un atome d'hydrogène et Y représente un atome d'oxygène ou un radical NH et R_{**2**} représente un atome d'halogène ou un radical trifluorométhyle et R_{**1**} représente un radical nitro ou un atome d'halogène.

2. Les produits de formule (I) telle que définie à la revendication 1, dans laquelle Y représente un atome d'oxygène, à l'exception des produits dans lesquels le groupement -A-B- représente le radical : 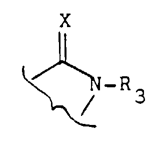 dans lequel X représente un atome d'oxygène et R_{**3**} représente un atome d'hydrogène, R_{**2**} représente un atome d'halogène ou un radical trifluorométhyle et R_{**1**} représente un radical nitro ou un atome d'halogène.

3. Les produits de formule (I) telle que définie à la revendication 1 ou 2 dans laquelle le groupement -A-B- représente le groupement : 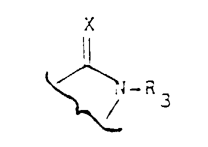 dans lequel X représente un atome de soufre et R_{**3**} a la signification indiquée à la revendication 1.

4. Les produits de formule (I) selon la revendication 3, dans laquelle R_{**3**} représente un atome d'hydrogène ou un radical alkyle ayant au plus 4 atomes de carbone éventuellement substitué par un radical hydroxy.

5. Les produits de formule (I) telle que définie à l'une quelconque des revendications 1 à 4, dans laquelle R_{**1**} représente un radical cyano ou un atome d'halogène.

6. Les produits de formule (I) selon la revendication 5, dans laquelle R_{**1**} représente un atome de chlore.

7. Les produits de formule (I) telle que définie à la revendication 1 ou 2, dans laquelle le groupement -A-B- représente un groupement : 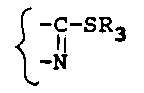 ou un groupement : 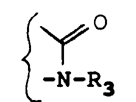 dans lequel R_{**3**} représente un radical alkyle ou alkényle ayant au plus 4 atomes de carbone ou un radical aralkyle éventuellement substitué.

8. Les produits de formule (I) telle que définie à l'une quelconque des revendications 1 à 5 dont les noms suivent :
- le 4-(5-oxo-2-thioxo-3,4,4-triméthyl-1-imidazolidinyl)-2(trifluorométhyl)-benzonitrile,
- le 4-(4,4-diméthyl-5-oxo-2-thioxo 1-imidazolidinyl)-2(trifluorométhyl)-benzonitrile,
- le 4-[4,4-diméthyl 3-(2-hydroxyéthyl) 5-oxo 2-thioxo 1-imidazolidinyl] 2-(trifluorométhyl) benzonitrile,
- le 3-(3,4-dichlorophényl) 2-thioxo 1,5,5-triméthyl 4-imidazolidinone.

9. Les produits de formule (I) telle que définie à l'une quelconque des revendications 1, 2 et 7, dont les noms suivent :
- le 1-(4-nitro-3-(trifluorométhyl) phényl)-3,4,4-triméthyl-2,5-imidazolidinedione,
- le 4-[[4,5-dihydro 4,4-diméthyl 5-oxo 2-(phénylméthyl) thio] 1H-imidazol-1-yl] 2-(trifluorométhyl) benzonitrile.

10. Procédé de préparation des produits de formule générale (I) telle que définie à la revendication 1, caractérisé en ce que :
soit l'on fait agir en présence d'une base tertiaire un produit de formule (II) : 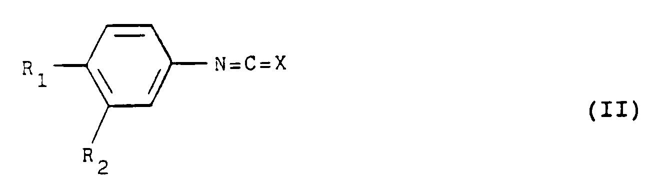 dans laquelle R_{**1**}, R_{**2**} et X ont la signification indiquée ci-dessus, avec un produit de formule (III) : 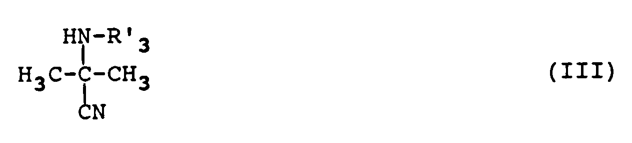 dans laquelle R′_{**3**} a les valeurs indiquées ci-dessus pour R_{**3**} dans lequel les éventuelles fonctions réactives sont éventuellement protégées et étant entendu que si R_{**1**} représente un radical nitro ou un atome d'halogène, si R_{**2**} représente un atome d'halogène ou un radical CF_{**3**} et X représente un atome d'oxygène, R′_{**3**} ne peut pas représenter un atome d'hydrogène, pour obtenir un produit de formule (IV) : 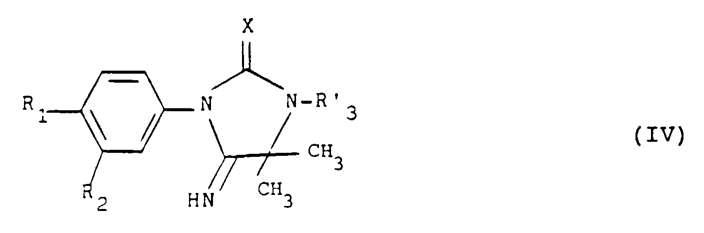 dans laquelle R_{**1**}, R_{**2**}, X et R′_{**3**} ont la signification précédente, produits de formule (IV) que, si nécessaire ou si désiré l'on soumet à l'une quelconque ou plusieurs des réactions suivantes, dans un ordre quelconque :
a) réaction d'élimination des éventuels groupements protecteurs que peut porter R′_{**3**} ;
b) réaction d'hydrolyse du groupement >C=NH en fonction cétone et le cas échéant transformation du groupement >C=S en groupement >C=O ;
c) réaction de transformation du ou des groupements >C=O en groupement >C=S ;
d) action sur les produits de formule (IV) dans laquelle R′_{**3**} représente un atome d'hydrogène, et après hydrolyse au groupement >C=NH en fonction cétone d'un réactif de formule Hal-R˝_{**3**} dans laquelle R˝_{**3**} a les valeurs de R′_{**3**} à l'exception de la valeur hydrogène et Hal représente un atome d'halogène pour obtenir des produits de formule (I) dans laquelle le groupement -A-B- représente le groupement 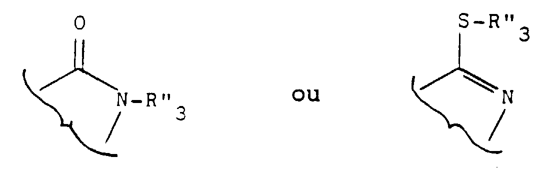 dans lesquels R˝_{**3**} a la signification indiquée précédemment puis, si désiré, action sur ces produits, d'un agent d'élimination des éventuels groupements protecteurs que peut porter R˝_{**3**} ou le cas échéant, action d'un agent d'estérification, d'amidification ou de salification,
soit l'on fait agir un réactif de formule Hal-R˝_{**3**} dans laquelle Hal et R˝_{**3**} ont les valeurs indiquées précédemment sur un produit de formule (IV′) : 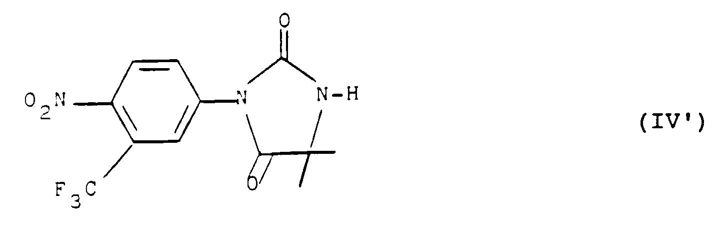 pour obtenir un produit de formule (IV˝) : 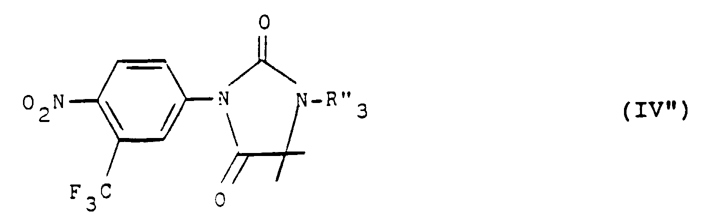 produit de formule (IV") que, si nécessaire ou si désiré l'on soumet à l'une quelconque ou plusieurs des réactions suivantes dans un ordre quelconque :
a) réaction d'élimination des éventuels groupements protecteurs que peut porter R"_{**3**} puis le cas échéant action d'un agent d'estérification, d'amidification ou de salification ;
b) réaction de transformation du ou des groupements >C=O en groupements >C=S.

11. A titre de médicaments, les produits de formule (I) tels que définis aux revendications 1 à 7, pharmaceutiquement acceptables.

12. A titre de médicaments, les produits de formule (I) tels que définis à la revendication 8 ou 9.

13. Les compositions pharmaceutiques contenant, à titre de principe actif, l'un au moins des médicaments tels que définis à l'une quelconque des revendications 11 et 12.

14. Les produits de formule (IVi) : 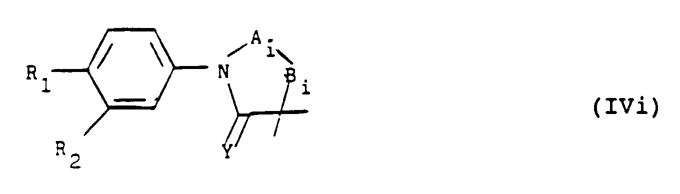 dans laquelle R_{**1**}, R_{**2**} et Y ont les significations indiquées à la revendication 1 et le groupement : 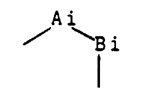 est choisi parmi les radicaux : 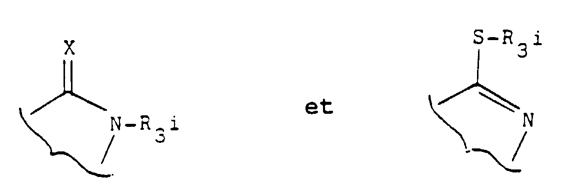 dans lesquels X représente un atome d'oxygène ou de soufre et R_{**3i**} est choisi parmi les valeurs de R_{**3**} comportant une fonction réactive protegée,
à l'exception des produits dans lesquels le groupement -A_{**i**}B_{**i**}- représente le radical : 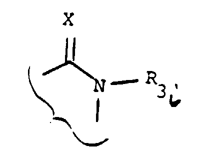 dans lequel X représente un atome d'oxygène et R_{**3i**} représente un atome d'hydrogène et Y représente un atome d'oxygène ou un radical NH et R_{**2**} représente un atome d'halogène ou un radical trifluorométhyle et R_{**1**} représente un radical nitro ou un atome d'halogène.

## Revendications (Revendications pour l'(les) Etat(s) contractant(s) suivant(s): ES)

1. Procédé pour préparer les produits de formule générale (I) : 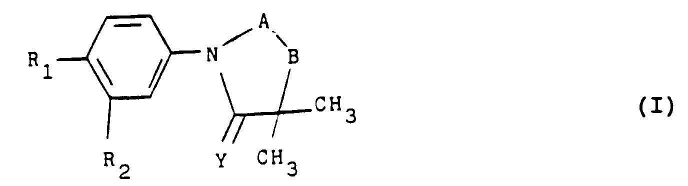 dans laquelle :
R_{**1**} représente un radical cyano ou nitro ou un atome d'halogène,
R_{**2**} représente un radical trifluorométhyle ou un atome d'halogène,
le groupement -A-B- est choisi parmi les radicaux 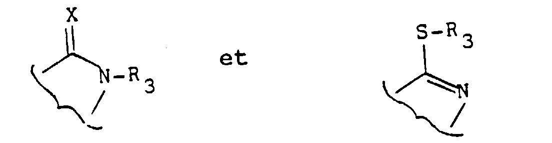 dans lesquels X représente un atome d'oxygène ou de soufre et
R_{**3**} est choisi parmi les radicaux suivants :
- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, S-aryle éventuellement substitué, dans lequel l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,
les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone,
les radicaux aryle et aralkyle étant de plus éventuellement substitués par un radical alkyle, alkényle ou alkynyle, alkoxy, alkényloxy, alkynyloxy ou trifluorométhyle, Y représente un atome d'oxygène ou de soufre ou un radical =NH,
à l'exception des produits dans lesquels le groupement -A-B-représente le radical : 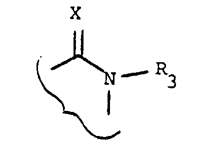 dans lequel X représente un atome d'oxygène et R_{**3**} représente un atome d'hydrogène et Y représente un atome d'oxygène ou un radical NH et R_{**2**} représente un atome d'halogène ou un radical trifluorométhyle et R_{**1**} représente un radical nitro ou un atome d'halogène, caractérisé en ce que :
soit l'on fait agir en présence d'une base tertiaire un produit de formule (II) : 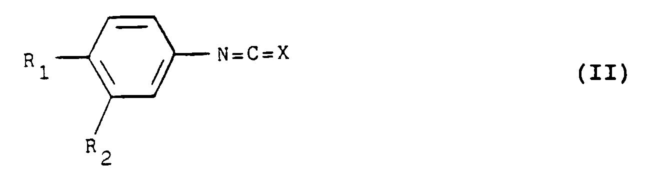 dans laquelle R_{**1**}, R_{**2**} et X ont la signification indiquée ci-dessus, avec un produit de formule (III) : 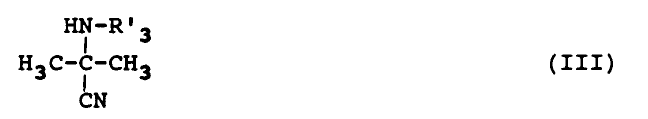 dans laquelle R′_{**3**} a les valeurs indiquées ci-dessus pour R_{**3**} dans lequel les éventuelles fonctions réactives sont éventuellement protégées et étant entendu que si R_{**1**} représente un radical nitro ou un atome d'halogène, si R_{**2**} représente un atome d'halogène ou un radical CF_{**3**} et X représente un atome d'oxygène, R′_{**3**} ne peut pas représenter un atome d'hydrogène, pour obtenir un produit de formule (IV) : 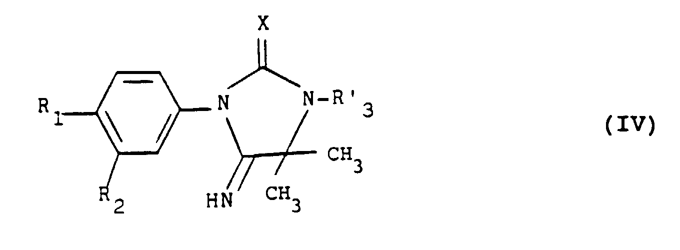 dans laquelle R_{**1**}, R_{**2**}, X et R′_{**3**} ont la signification précédente, produits de formule (IV) que, si nécessaire ou si désiré l'on soumet à l'une quelconque ou plusieurs des réactions suivantes, dans un ordre quelconque :
a) réaction d'élimination des éventuels groupements protecteurs que peut porter R′_{**3**} ;
b) réaction d'hydrolyse du groupement >C=NH en fonction cétone et le cas échéant transformation du groupement >C=S en groupement >C=O ;
c) réaction de transformation du ou des groupements >C=O en groupement >C=S ;
d) action sur les produits de formule (IV) dans laquelle R′_{**3**} représente un atome d'hydrogène, et après hydrolyse au groupement >C=NH en fonction cétone d'un réactif de formule Hal-R˝_{**3**} dans laquelle R˝_{**3**} a les valeurs de R′_{**3**} à l'exception de la valeur hydrogène et Hal représente un atome d'halogène pour obtenir des produits de formule (I) dans laquelle le groupement -A-B- représente le groupement 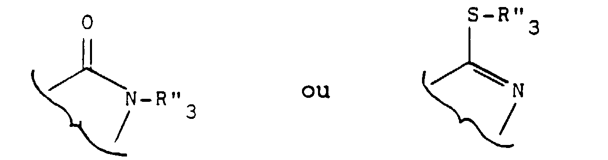 dans lesquels R˝_{**3**} a la signification indiquée précédemment puis, si désiré, action sur ces produits, d'un agent d'élimination des éventuels groupements protecteurs que peut porter R˝_{**3**} ou le cas échéant, action d'un agent d'estérification, d'amidification ou de salification,
soit l'on fait agir un réactif de formule Hal-R˝_{**3**} dans laquelle Hal et R˝_{**3**} ont les valeurs indiquées précédemment sur un produit de formule (IV′) : 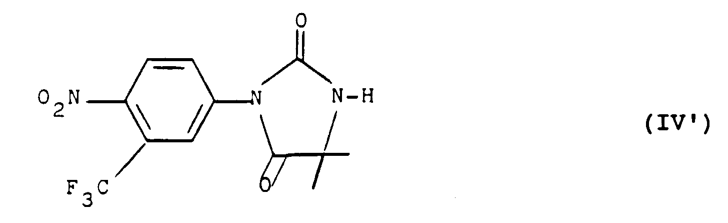 pour obtenir un produit de formule (IV˝) : 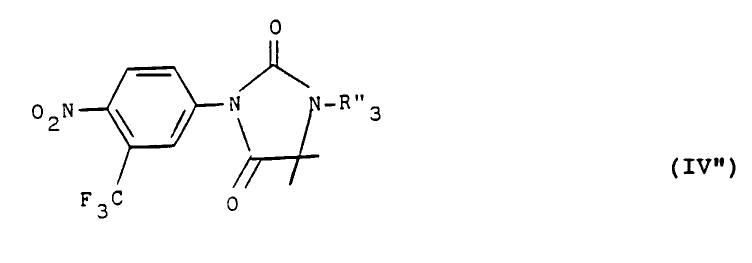 produit de formule (IV˝) que, si nécessaire ou si désiré l'on soumet à l'une quelconque ou plusieurs des réactions suivantes dans un ordre quelconque :
a) réaction d'élimination des éventuels groupements protecteurs que peut porter R˝_{**3**} puis le cas échéant action d'un agent d'estérification, d'amidification ou de salification ;
b) réaction de transformation du ou des groupements >C=O en groupements >C=S.

2. Procédé selon la revendication 1 pour préparer un produit de formule (I) telle que définie à la revendication 1, dans laquelle Y représente un atome d'oxygène, à l'exception des produits dans lesquels le groupement -A-B- représente le radical : 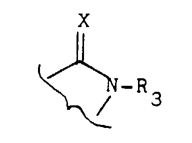 dans lequel X représente un atome d'oxygène et R_{**3**} représente un atome d'hydrogène, R_{**2**} représente un atome d halogène ou un radical trifluorométhyle et R_{**1**} représente un radical nitro ou un atome d'halogène, caractérisé en ce que
soit l'on soumet un composé de formule (IV) telle que définie à la revendication 1 à l'exception des produits dans lesquels X représente un atome d'oxygène, R′_{**3**} représente un atome d'hydrogène, R_{**2**} représente un atome d'halogène ou un radical trifluorométhyle et R_{**1**} représente un radical nitro ou un atome d'halogène, à l'une quelconque ou plusieurs des réactions suivantes, dans un ordre quelconque :
a) réaction d'élimination des éventuels groupements protecteurs que peut porter R′_{**3**} ;
b) réaction d'hydrolyse du groupement >C=NH en fonction cétone puis si désiré action d'un réactif de formule Hal-R˝_{**3**} dans laquelle R˝_{**3**} a les valeurs de R′_{**3**} à l'exception de la valeur hydrogène et Hal représente un atome d'halogène pour obtenir des produits de formule (I) dans laquelle le groupement -A-B-représente le groupement 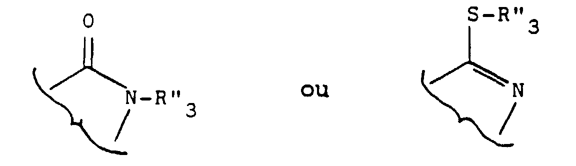 dans lesquels R˝_{**3**} a la signification indiquée précédemment puis, si désiré, action sur ces produits, d'un agent d'élimination des éventuels groupements protecteurs que peut porter R˝_{**3**} ou le cas échéant, action d'un agent d'estérification, d'amidification ou de salification,
soit l'on fait agir un réactif de formule Hal-R˝_{**3**} dans laquelle Hal et R˝_{**3**} ont les valeurs indiquées précédemment sur un produit de formule (IV′) telle que définie à la revendication 1 pour obtenir un un produit de formule (IV˝) que, si nécessaire ou si désiré l'on soumet à une réaction d'élimination des éventuels groupements protecteurs que peut porter R˝_{**3**} et le cas échéant action d'un agent d'estérification, d'amidification ou de salification.

3. Procédé selon la revendication 1 ou 2 pour préparer un produit de formule (I) telle que définie à la revendication 1 dans laquelle le groupement -A-B- représente le groupement 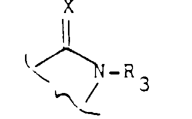 dans lequel X représente un atome de soufre et R_{**3**} a la signification indiquée précédemment caractérisé en ce que soit l'on utilise au départ un produit de formule (II) dans laquelle X représente un atome de soufre, spot l'on soumet un produit de formule (IV) ou (IV") à l'une quelconque ou plusieurs des réactions suivantes, dans un ordre quelconque :
- réaction d'élimination des éventuels groupements protecteurs que peut porter R'_{**3**} u R"_{**3**} ;
- réaction de transformation du ou des groupements >C=O en groupement >C=S.

4. Procédé selon la revendication 3 pour préparer un produit de formule (I) telle que définie à la revendication 3 dans laquelle R_{**3**} représente un atome d'hydrogène ou un radical alkyle ayant au plus 4 atomes de carbone éventuellement substitué par un radical hydroxy, caractérisé en ce que soit l'on utilise au départ un produit de formule (III) dans laquelle R'_{**3**} a les valeurs de R_{**3**} indiquées ci-dessus éventuellement protégées, soit l'on traite le produit de formule (IV') par un réactif de formule Hal-R"_{**3**} dans lequel R"_{**3**} a les valeurs de R_{**3**} indiquées ci-dessus éventuellement protégées à l'exception de la valeur hydrogène puis le cas échéant soumet le produit obtenu à l'action d'un agent d'élimination des groupements protecteurs.

5. Procédé selon l'une quelconque des revendications 1 à 4, caractérisé en ce que l'on utilise au départ un produit de formule (II) dans laquelle R_{**1**} représente un radical cyano ou un atome d'halogène.

6. Procédé selon la revendication 5, caractérisé en ce que l'on utilise au départ un produit de formule (II) dans laquelle R_{**1**} représente un atome de chlore.

7. Procédé selon la revendication 1 ou 2 pour préparer un produit de formule (I) telle que définie à la revendication 1 dans laquelle le groupement -A-B- représente un groupement 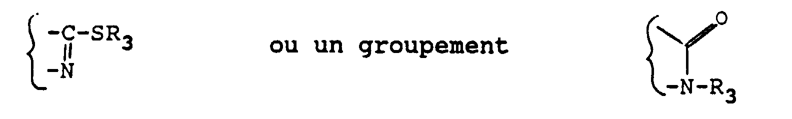 dans lequel R_{**3**} représente un radical alkyle ou alkényle ayant au plus 4 atomes de carbone ou un radical aralkyle éventuellement substitué, caractérisé en ce que l'on utilise au départ soit un produit de formule (III) dans laquelle R'_{**3**} a les valeurs de R_{**3**} indiquées ci-dessus, soit un réactif de formule Hal-R"_{**3**} dans lequel R"_{**3**} a les valeurs de R_{**3**} indiquées ci-dessus.

## Revendications (Revendications pour l'(les) Etat(s) contractant(s) suivant(s): GR)

1. Procédé pour préparer les produits de formule générale (I) : 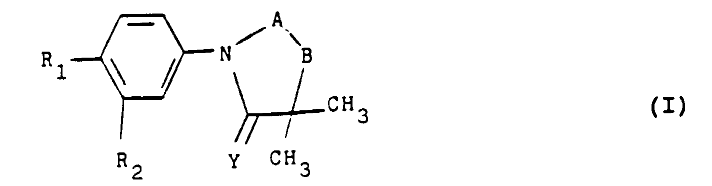 dans laquelle :
R_{**1**} représente un radical cyano ou nitro ou un atome d'halogène,
R_{**2**} représente un radical trifluorométhyle ou un atome d'halogène,
le groupement -A-B- est choisi parmi les radicaux 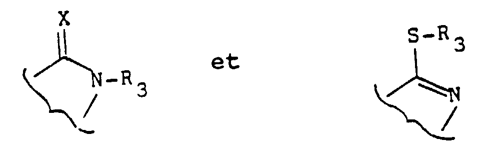 dans lesquels X représente un atome d'oxygène ou de soufre et
R_{**3**} est choisi parmi les radicaux suivants :
- un atome d'hydrogène,
- les radicaux alkyle, alkényle, alkynyle, aryle ou arylalkyle ayant au plus 12 atomes de carbone, ces radicaux étant éventuellement substitués par un ou plusieurs substituants choisis parmi les radicaux hydroxy, halogène, mercapto, cyano, acyle ou acyloxy ayant au plus 7 atomes de carbone, S-aryle éventuellement substitué, dans lequel l'atome de soufre est éventuellement oxydé sous forme de sulfoxyde ou de sulfone, carboxy libre, estérifié, amidifié ou salifié, amino, mono ou dialkylamino ou un radical hétérocyclique comprenant 3 à 6 chaînons et renfermant un ou plusieurs hétéroatomes choisis parmi les atomes de soufre, d'oxygène ou d'azote,
les radicaux alkyle, alkényle ou alkynyle étant de plus éventuellement interrompus par un ou plusieurs atomes d'oxygène, d'azote ou de soufre éventuellement oxydé sous forme de sulfoxyde ou de sulfone,
les radicaux aryle et aralkyle étant de plus éventuellement substitués par un radical alkyle, alkényle ou alkynyle, alkoxy, alkényloxy, alkynyloxy ou trifluorométhyle, Y représente un atome d'oxygène ou de soufre ou un radical =NH,
à l'exception des produits dans lesquels le groupement -A-B-représente le radical : 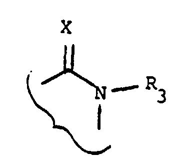 dans lequel X représente un atome d'oxygène et R_{**3**} représente un atome d'hydrogène et Y représente un atome d'oxygène ou un radical NH et R_{**2**} représente un atome d'halogène ou un radical trifluorométhyle et R_{**1**} représente un radical nitro ou un atome d'halogène, caractérisé en ce que :
soit l'on fait agir en présence d'une base tertiaire un produit de formule (II) : 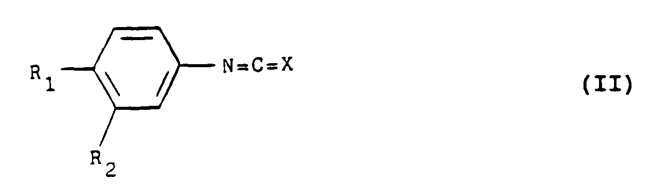 dans laquelle R_{**1**}, R_{**2**} et X ont la signification indiquée ci-dessus, avec un produit de formule (III) : 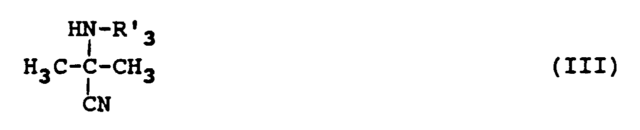 dans laquelle R′_{**3**} a les valeurs indiquées ci-dessus pour R_{**3**} dans lequel les éventuelles fonctions réactives sont éventuellement protégées et étant entendu que si R_{**1**} représente un radical nitro ou un atome d'halogène, si R_{**2**} représente un atome d'halogène ou un radical CF_{**3**} et X représente un atome d'oxygène, R′_{**3**} ne peut pas représenter un atome d'hydrogène, pour obtenir un produit de formule (IV) : 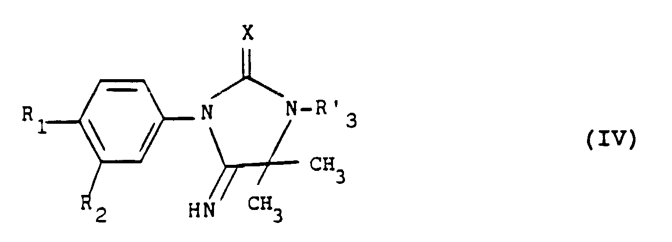 dans laquelle R_{**1**}, R_{**2**}, X et R′_{**3**} ont la signification précédente, produits de formule (IV) que, si nécessaire ou si désiré l'on soumet à l'une quelconque ou plusieurs des réactions suivantes, dans un ordre quelconque :
a) réaction d'élimination des éventuels groupements protecteurs que peut porter R′_{**3**} ;
b) réaction d'hydrolyse du groupement >C=NH en fonction cétone et le cas échéant transformation du groupement >C=S en groupement >C=O ;
c) réaction de transformation du ou des groupements >C=O en groupement >C=S ;
d) action sur les produits de formule (IV) dans laquelle R′_{**3**} représente un atome d'hydrogène, et après hydrolyse au groupement >C=NH en fonction cétone d'un réactif de formule Hal-R˝_{**3**} dans laquelle R˝_{**3**} a les valeurs de R′_{**3**} à l'exception de la valeur hydrogène et Hal représente un atome d'halogène pour obtenir des produits de formule (I) dans laquelle le groupement -A-B- représente le groupement 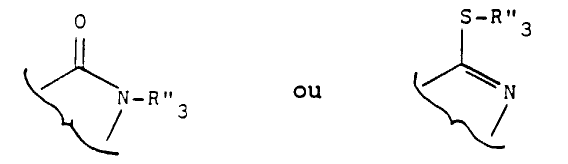 dans lesquels R˝_{**3**} a la signification indiquée précédemment puis, si désiré, action sur ces produits, d'un agent d'élimination des éventuels groupements protecteurs que peut porter R˝_{**3**} ou le cas échéant, action d'un agent d'estérification, d'amidification ou de salification,
soit l'on fait agir un réactif de formule Hal-R˝_{**3**} dans l'aquelle Hal et R˝_{**3**} ont les valeurs indiquées précédemment sur un produit de formule (IV′) : 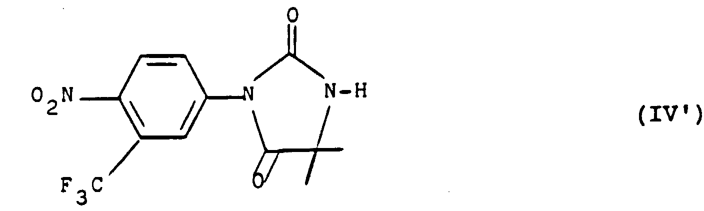 pour obtenir un produit de formule (IV˝) : 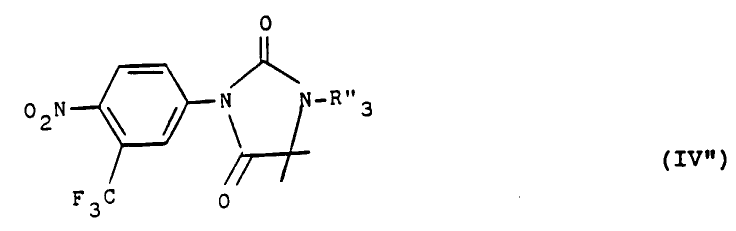 produit de formule (IV˝) que, si nécessaire ou si désiré l'on soumet à l'une quelconque ou plusieurs des réactions suivantes dans un ordre quelconque :
a) réaction d'élimination des éventuels groupements protecteurs que peut porter R˝_{**3**} puis le cas échéant action d'un agent d'estérification, d'amidification ou de salification ;
b) réaction de transformation du ou des groupements >C=O en groupements >C=S.

2. Procédé selon la revendication 1 pour préparer un produit de formule (I) telle que définie à la revendication 1, dans laquelle Y représente un atome d'oxygène, à l'exception des produits dans lesquels le groupement -A-B- représente le radical : 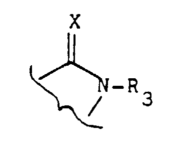 dans lequel X représente un atome d'oxygène et R_{**3**} représente un atome d'hydrogène, R_{**2**} représente un atome d'halogène ou un radical trifluorométhyle et R_{**1**} représente un radical nitro ou un atome d'halogène, caractérisé en ce que
soit l'on soumet un composé de formule (IV) telle que définie à la revendication 1 à l'exception des produits dans lesquels X représente un atome d'oxygène, R′_{**3**} représente un atome d'hydrogène, R_{**2**} représente un atome d'halogène ou un radical trifluorométhyle et R_{**1**} représente un radical nitro ou un atome d'halogène, à l'une quelconque ou plusieurs des réactions suivantes, dans un ordre quelconque :
a) réaction d'élimination des éventuels groupements protecteurs que peut porter R′_{**3**} ;
b) réaction d'hydrolyse du groupement >C=NH en fonction cétone puis si désiré action d'un réactif de formule Hal-R˝_{**3**} dans laquelle R˝_{**3**} a les valeurs de R′_{**3**} à l'exception de la valeur hydrogène et Hal représente un atome d'halogène pour obtenir des produits de formule (I) dans laquelle le groupement -A-B-représente le groupement 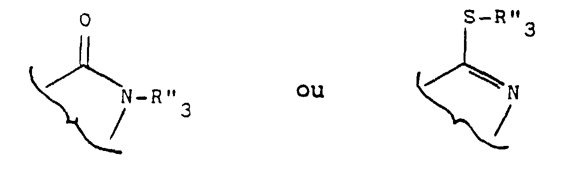 dans lesquels R˝_{**3**} a la signification indiquée précédemment puis, si désiré, action sur ces produits, d'un agent d'élimination des éventuels groupements protecteurs que peut porter R˝3 ou le cas échéant, action d'un agent d'estérification, d'amidification ou de salification,
soit l'on fait agir un réactif de formule Hal-R˝_{**3**} dans laquelle Hal et R˝_{**3**} ont les valeurs indiquées précédemment sur un produit de formule (IV′) telle que définie à la revendication 1 pour obtenir un un produit de formule (IV˝) que, si nécessaire ou si désiré l'on soumet à une réaction d'élimination des éventuels groupements protecteurs que peut porter R˝_{**3**} et le cas échéant action d'un agent d'estérification, d'amidification ou de salification.

3. Procédé selon la revendication 1 ou 2 pour préparer un produit de formule (I) telle que définie à la revendication 1 dans laquelle le groupement -A-B- represente le groupement 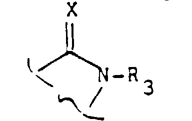 dans lequel X représente un atome de soufre et R_{**3**} a la signification indiquée précédemment caractérisé en ce que soit l'on utilise au départ un produit de formule (II) dans laquelle X représente un atome de soufre, spot l'on soumet un produit de formule (IV) ou (IV˝) à l'une quelconque ou plusieurs des réactions suivantes, dans un ordre quelconque :
- réaction d'élimination des éventuels groupements protecteurs que peut porter R′_{**3**} u R˝_{**3**} ;
- réaction de transformation du ou des groupements >C=O en groupement >C=S.

4. Procédé selon la revendication 3 pour préparer un produit de formule (I) telle que définie à la revendication 3 dans laquelle R_{**3**} représente un atome d'hydrogène ou un radical alkyle ayant au plus 4 atomes de carbone éventuellement substitué par un radical hydroxy, caractérisé en ce que soit l'on utilise au départ un produit de formule (III) dans laquelle R′_{**3**} a les valeurs de R_{**3**} indiquées ci-dessus éventuellement protégées, soit l'on traite le produit de formule (IV′) par un réactif de formule Hal-R˝_{**3**} dans lequel R˝_{**3**} a les valeurs de R_{**3**} indiquées ci-dessus éventuellement protégées à l'exception de la valeur hydrogène puis le cas échéant soumet le produit obtenu à l'action d'un agent d'élimination des groupements protecteurs.

5. Procédé selon l'une quelconque des revendications 1 à 4, caractérisé en ce que l'on utilise au départ un produit de formule (II) dans laquelle R_{**1**} représente un radical cyano ou un atome d'halogène.

6. Procédé selon la revendication 5, caractérisé en ce que l'on utilise au départ un produit de formule (II) dans laquelle R_{**1**} représente un atome de chlore.

7. Procédé selon la revendication 1 ou 2 pour préparer un produit de formule (I) telle que définie à la revendication 1 dans laquelle le groupement -A-B- représente un groupement 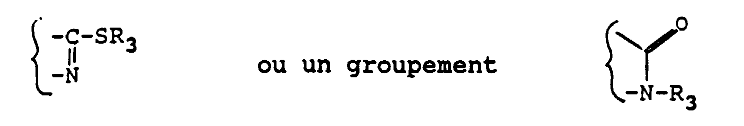 dans lequel R_{**3**} représente un radical alkyle ou alkényle ayant au plus 4 atomes de carbone ou un radical aralkyle éventuellement substitué, caractérisé en ce que l'on utilise au départ soit un produit de formule (III) dans laquelle R'_{**3**} a les valeurs de R_{**3**} indiquées ci-dessus, soit un réactif de formule Hal-R"_{**3**} dans lequel R"_{**3**} a les valeurs de R_{**3**} indiquées ci-dessus.

8. Procédé selon l'une quelconque des revendications 1 à 5, caractérisé en ce que l'on choisit les produits et réactifs de départ de manière telle que l'on prépare l'un quelconque des produits dont les noms suivent :
- le 4-(5-oxo-2-thioxo-3,4,4-triméthyl-1-imidazolidinyl)-2(trifluorométhyl)-benzonitrile,
- le 4-(4,4-diméthyl-5-oxo-2-thioxo 1-imidazolidinyl)-2(trifluorométhyl)-benzonitrile,
- le 4-[4,4-diméthyl 3-(2-hydroxyéthyl) 5-oxo 2-thioxo 1-imidazolidinyl] 2-(trifluorométhyl) benzonitrile,
- le 3-(3,4-dichlorophényl) 2-thioxo 1,5,5-triméthyl 4-imidazolidinone.

9. Procédé selon la revendication 1, 2 ou 7, caractérisé en ce que l'on choisit les produits et réactifs de départ de manière telle que l'on prépare l'un quelconque des produits dont les noms suivent :
- le 1-(4-nitro-3-(trifluorométhyl) phényl)-3,4,4-triméthyl-2,5-imidazolidinedione,
- le 4-[[4,5-dihydro 4,4-diméthyl 5-oxo 2-(phénylméthyl) thio] 1H-imidazol-1-yl] 2-(trifluorométhyl) benzonitrile.

10. Procédé pour préparer des compositions pharmaceutiques, caractérisé en ce que l'on met à titre de principe actif l'un au moins des composés de formule (I) telle que définie à la revendication 1 sous une forme destinée à cet usage.

11. Procédé pour préparer des compositions pharmaceutiques, caractérisé en ce que l'on met à titre de principe actif l'un au moins des composés de formule (I) telle que définie à l'une quelconque des revendications 2 à 7 sous une forme destinée à cet usage.

12. Procédé pour préparer des compositions pharmaceutiques, caractérisé en ce que l'on met à titre de principe actif l'un au moins des composés de formule (I) telle que définie à la revendication 8 ou 9 sous une forme destinée à cet usage.

## Patentansprüche (Patentansprüche für folgende(n) Vertragsstaat(en): AT, BE, CH, DE, DK, FR, GB, IT, LI, LU, NL, PT, SE)

1. Verbindungen der allgemeinen Formel (I) 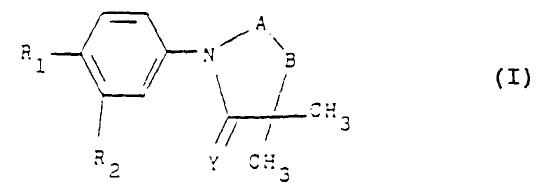 worin:
R₁ eine Cyano- oder Nitrogruppe oder ein Halogenatom bedeutet,
R₂ einen Trifluormethylrest oder ein Halogenatom bedeutet, die Gruppierung -A-B- aus den Resten 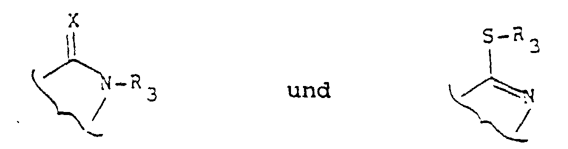 ausgewählt ist, worin X ein Sauerstoffatom oder ein Schwefelatom bedeutet, und R₃ aus den nachstehenden Resten ausgewählt ist:
- ein Wasserstoffatom,
- Alkyl-, Alkenyl-, Alkinyl-, Aryl- oder Arylalkylreste mit höchstens 12 Kohlenstoffatomen, wobei diese Reste gegebenenfalls durch einen oder mehrere Substituenten, ausgewählt aus Hydroxyl-, Halogen-, Mercapto-, Cyano-, Acyl- oder Acyloxyresten mit höchstens 7 Kohlenstoffatomen, gegebenenfalls substituiertem S-Aryl, worin das Schwefelatom gegebenenfalls in Form eines Sulfoxids oder Sulfons oxidiert ist, freier, veresterter, in ein Amid oder Salz überführter Carboxylgruppe, Amino-, Mono- oder Dialkylaminogruppe oder einem heterocyclischen Rest, umfassend 3 bis 6 Kettenglieder und umfassend ein oder mehrere Heteroatome, ausgewählt aus Schwefel-, Sauerstoff- oder Stickstoffatomen, substituiert sind, wobei die Alkyl-, Alkenyl- oder Alkinylreste außerdem gegebenenfalls durch ein oder mehrere Sauerstoff-, Stickstoff- oder Schwefelatom(e), das/die gegebenenfalls in Form des Sulfoxids oder Sulfons oxidiert ist/sind, unterbrochen sind, wobei die Aryl- und Aralkylreste außerdem gegebenenfalls durch einen Alkyl-, Alkenyl- oder Alkinylrest, Alkoxy-, Alkenyloxy-, Alkinyloxy- oder Trifluormethylrest substituiert sind,
Y ein Sauerstoff- oder Schwefelatom oder die Gruppe =NH bedeutet, ausgenommen die Verbindungen, in denen die Gruppierung -A-B- den Rest 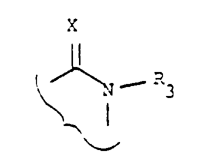 bedeutet, worin X ein Sauerstoffatom bedeutet und R₃ ein Wasserstoffatom bedeutet, und Y ein Sauerstoffatom oder die Gruppe NH bedeutet, und R₂ ein Halogenatom oder einen Trifluormethylrest bedeutet, und R₁ eine Nitrogruppe oder ein Halogenatom bedeutet.

2. Verbindungen der Formel (I) nach Anspruch 1, worin Y ein Sauerstoffatom bedeutet, ausgenommen die Verbindungen, in denen die Gruppierung -A-B- den Rest 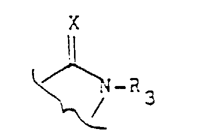 bedeutet, worin X ein Sauerstoffatom bedeutet und R₃ ein Wasserstoffatom bedeutet, R₂ ein Halogenatom oder einen Trifluormethylrest bedeutet, und R₁ eine Nitrogruppe oder ein Halogenatom bedeutet.

3. Verbindungen der Formel (I) nach Anspruch 1 oder 2, worin die Gruppierung -A-B- die Gruppierung 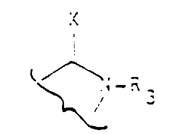 bedeutet, worin X ein Schwefelatom bedeutet und R₃ die in Anspruch 1 angegebene Bedeutung besitzt.

4. Verbindungen der Formel (I) nach Anspruch 3, worin R₃ ein Wasserstoffatom oder einen Alkylrest mit höchstens 4 Kohlenstoffatomen, der gegebenenfalls durch eine Hydroxylgruppe substituiert ist, bedeutet.

5. Verbindungen der Formel (I) nach einem der Ansprüche 1 bis 4, worin R₁ eine Cyanogruppe oder ein Halogenatom bedeutet.

6. Verbindungen der Formel (I) nach Anspruch 5, worin R₁ ein Chloratom bedeutet.

7. Verbindungen der Formel (I) nach Anspruch 1 oder 2, worin die Gruppierung -A-B- eine Gruppierung 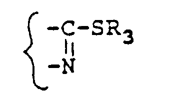 oder eine Gruppierung 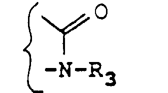 bedeutet, worin R₃ einen Alkyl- oder Alkinylrest mit höchstens 4 Kohlenstoffatomen oder einen gegebenenfalls substituierten Aralkylrest bedeutet.

8. Verbindungen der Formel (I) nach einem der Ansprüche 1 bis 5, nämlich:
- 4-(5-oxo-2-thioxo-3,4,4-trimethyl-1-imidazolidinyl)-2(trifluormethyl)benzonitril,
- 4-(4,4-Dimethyl-5-oxo-2-thioxo-1-imidazolidinyl)-2(trifluormethyl)benzonitril,
- 4-[4,4-Dimethyl-3-(2-hydroxyethyl)-5-oxo-2-thioxo-1-imidazolidinyl]-2-(trifluormethyl)benzonitril,
- 3-(3,4-Dichlorphenyl)-2-thioxo-1,5,5-trimethyl-4-imidazolidinon.

9. Verbindungen der Formel (I) nach einem der Ansprüche 1, 2 und 7, nämlich:
- 1-(4-Nitro-3-(trifluormethyl)phenyl)-3,4,4-trimethyl-2,5-imidazolidindion,
- 4-[[4,5-Dihydro-4,4-dimethyl-5-oxo-2-(phenylmethyl)thio]-1H-imidazol-1-yl]-2-(trifluormethyl)benzonitril.

10. Verfahren zur Herstellung von Verbindungen der allgemeinen Formel (I) nach Anspruch 1, dadurch **gekennzeichnet,** daß man
entweder in Gegenwart einer tertiären Base eine Verbindung der Formel (II) 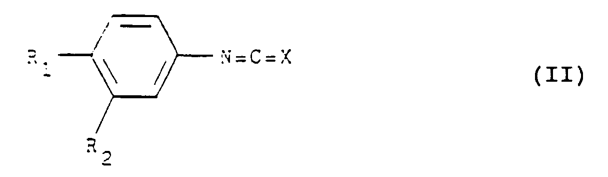 worin R₁, R₂ und X wie vorstehend definiert sind, mit einer Verbindung der Formel (III) 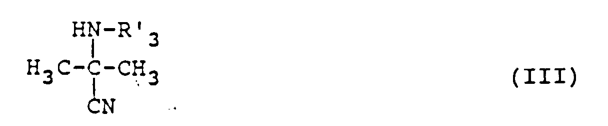 worin R'₃ wie vorstehend für R₃ definiert ist, worin die gegebenenfalls vorhandenen reaktiven Funktionen gegebenenfalls geschützt sind und mit der Maßgabe, daß, wenn R₁ eine Nitrogruppe oder ein Halogenatom bedeutet, wenn R₂ ein Halogenatom oder den Rest CF₃ bedeutet und X ein Sauerstoffatom bedeutet, R'₃ kein Wasserstoffatom bedeuten kann, umsetzt, um eine Verbindung der Formel (IV) 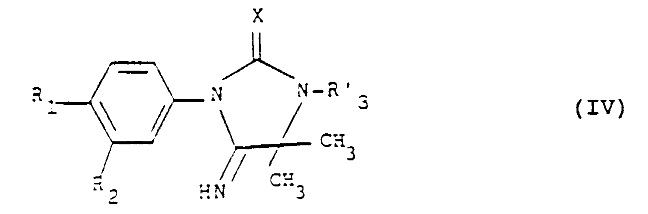 zu erhalten, worin R₁, R₂, X und R'₃ wie vorstehend definiert sind, wobei man die Verbindungen der Formel (IV), sofern notwendig oder erforderlich, einer oder mehreren der nachstehenden Reaktionen in beliebiger Reihenfolge unterwirft:
a) Eliminationsreaktion der gegebenenfalls vorhandenen Schutzgruppen, die R'₃ tragen kann;
b) Hydrolysereaktion der Gruppierung >C=NH zu der Ketonfunktion und gegebenenfalls Umwandlung der Gruppierung >C=S in die Gruppierung >C=O;
c) Transformationsreaktion der Gruppierung(en) >C=O in die Gruppierung >C=S;
d) Einwirkung eines Reagenses der Formel Hal-R"₃, worin R"₃ die Definitionen für R'₃, ausgenommen die Definition Wasserstoff, besitzt und Hal ein Halogenatom bedeutet, auf die Verbindungen der Formel (IV), worin R'₃ ein Wasserstoffatom bedeutet und nach Hydrolyse der Gruppierung >C=NH in die Ketonfunktion, um Verbindungen der Formel (I) zu erhalten, worin die Gruppierung -A-B- die Gruppierung 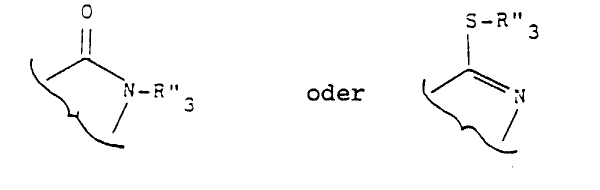 bedeutet, worin R"₃ wie vorstehend definiert ist, anschließend gegebenenfalls Einwirkung eines Mittels zur Abspaltung gegebenenfalls vorhandener Schutzgruppen, die R"₃ tragen kann, oder gegebenenfalls Einwirkung eines Mittels zur Veresterung, zur Überführung in ein Amid oder zur Salzbildung, auf diese Verbindungen,
oder ein Reagens der Formel Hal-R"₃, worin Hal und R"₃ wie vorstehend definiert sind, auf eine Verbindung der Formel (IV') 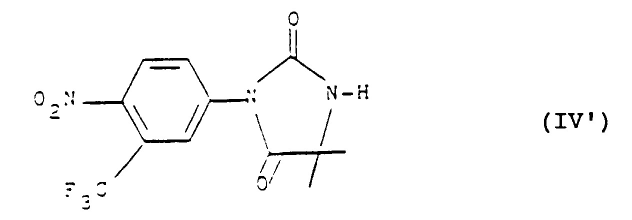 einwirken läßt, um eine Verbindung der Formel (IV") 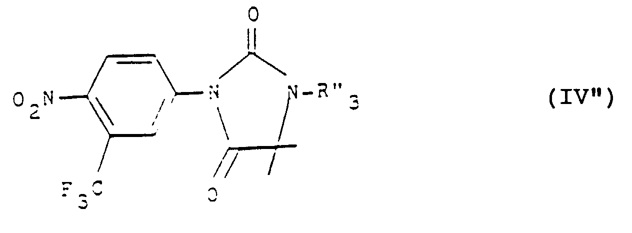 zu erhalten, wobei man die Verbindung der Formel (IV"), sofern notwendig oder erwünscht, einer oder mehreren der nachstehenden Reaktionen in beliebiger Reihenfolge unterwirft:
a) Eliminierungsreaktion gegebenenfalls vorhandener Schutzgruppen, die R"₃ tragen kann, dann gegebenenfalls Einwirkung eines Mittels zur Veresterung, zur Amidbildung oder zur Salzbildung;
b) Transformationsreaktion der Gruppierung(en) >C=O in die Gruppierungen >C=S.

11. Pharmazeutisch verträgliche Verbindungen der Formel (I) nach den Ansprüchen 1 bis 7 als Arzneimittel.

12. Verbindungen der Formel (I) nach Anspruch 8 oder 9 als Medikamente.

13. Pharmazeutische Präparate, umfassend als Wirkstoff mindestens eines der Arzneimittel nach einem der Ansprüche 11 und 12.

14. Verbindungen der Formel (IVi) 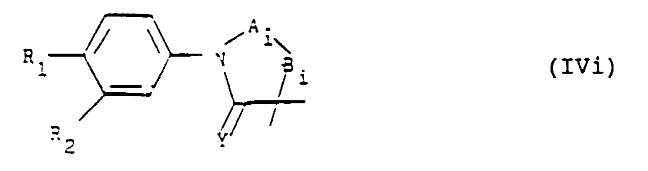 worin R₁, R₂ und Y wie in Anspruch 1 definiert sind und die Gruppierung 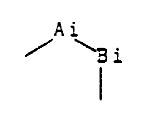 aus den Resten 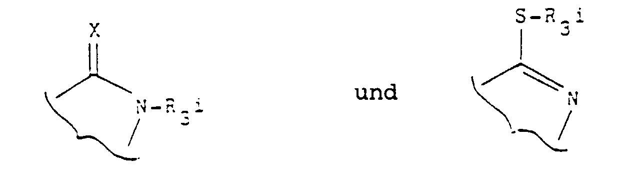 worin X ein Sauerstoff- oder Schwefelatom bedeutet, und R₃ᵢ aus den Bedeutungen von R₃, umfassend eine geschützte reaktive Funktion, ausgewählt ist, ausgenommen die Verbindungen, in denen die Gruppierung -Aᵢ-Bᵢ- den Rest 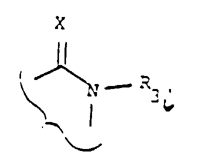 bedeutet, worin X ein Sauerstoffatom bedeutet, und R₃ᵢ ein Wasserstoffatom bedeutet, und Y ein Sauerstoffatom oder die Gruppe NH bedeutet, und R₂ ein Halogenatom oder eine Trifluormethylgruppe bedeutet, und R₁ eine Nitrogruppe oder ein Halogenatom bedeutet.

## Patentansprüche (Patentansprüche für folgende(n) Vertragsstaat(en): ES)

1. Verfahren zur Herstellung der Verbindungen der allgemeinen Formel (I) 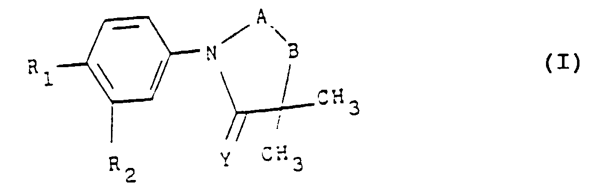 worin
R₁ eine Cyano- oder Nitrogruppe oder ein Halogenatom bedeutet,
R₂ einen Trifluormethylrest oder ein Halogenatom bedeutet, die Gruppierung -A-B- aus den Resten 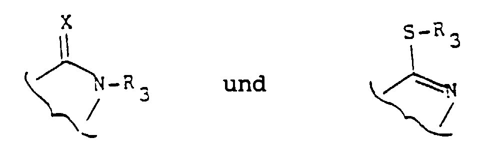 ausgewählt ist, worin X ein Sauerstoffatom oder ein Schwefelatom bedeutet, und R₃ aus den nachstehenden Resten ausgewählt ist:
- ein Wasserstoffatom,
- Alkyl-, Alkenyl-, Alkinyl-, Aryl- oder Arylalkylreste mit höchstens -12 Kohlenstoffatomen, wobei diese Reste gegebenenfalls durch einen oder mehrere Substituenten, ausgewählt aus Hydroxyl-, Halogen-, Mercapto-, Cyano-, Acyl- oder Acyloxyresten mit höchstens 7 Kohlenstoffatomen, gegebenenfalls substituiertem S-Aryl, worin das Schwefelatom gegebenenfalls in Form eines Sulfoxids oder Sulfons oxidiert ist, freier, veresterter, in ein Amid oder Salz überführter Carboxylgruppe, Amino-, Mono- oder Dialkylaminogruppe oder einem heterocyclischen Rest, umfassend 3 bis 6 Kettenglieder und umfassend ein oder mehrere Heteroatome, ausgewählt aus Schwefel-, Sauerstoff- oder Stickstoffatomen, substituiert sind, wobei die Alkyl-, Alkenyl- oder Alkinylreste außerdem gegebenenfalls durch ein oder mehrere Sauerstoff-, Stickstoff- oder Schwefelatom(e), das/die gegebenenfalls in Form des Sulfoxids oder Sulfons oxidiert ist/sind, unterbrochen sind, wobei die Aryl- und Aralkylreste außerdem gegebenenfalls durch einen Alkyl-, Alkenyl- oder Alkinylrest, Alkoxy-, Alkenyloxy-, Alkinyloxy- oder Trifluormethylrest substituiert sind,
Y ein Sauerstoff- oder Schwefelatom oder die Gruppe =NH bedeutet, ausgenommen die Verbindungen, in denen die Gruppierung -A-B- den Rest 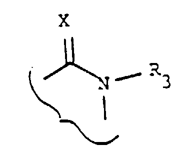 bedeutet, worin X ein Sauerstoffatom bedeutet, und R₃ ein Wasserstoffatom bedeutet, und Y ein Sauerstoffatom oder die Gruppe NH bedeutet, und R₂ ein Halogenatom oder einen Trifluormethylrest bedeutet, und R₁ eine Nitrogruppe oder ein Halogenatom bedeutet, dadurch gekennzeichnet, daß man entweder in Gegenwart einer tertiären Base eine Verbindung der Formel (II) 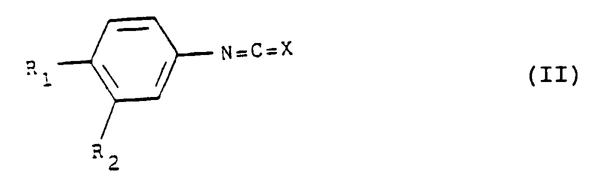 worin R₁, R₂ und X wie vorstehend definiert sind, mit einer Verbindung der Formel (III) 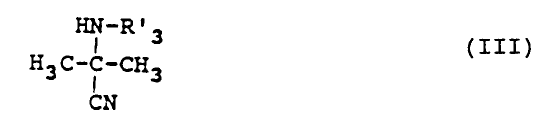 worin R'₃ wie vorstehend für R₃ definiert ist, worin die gegebenenfalls vorhandenen reaktiven Funktionen gegebenenfalls geschützt sind und mit der Maßgabe, daß, wenn R₁ eine Nitrogruppe oder ein Halogenatom bedeutet, wenn R₂ ein Halogenatom oder den Rest CF₃ bedeutet und X ein Sauerstoffatom bedeutet, R'₃ kein Wasserstoffatom bedeuten kann, umsetzt, um eine Verbindung der Formel (IV) 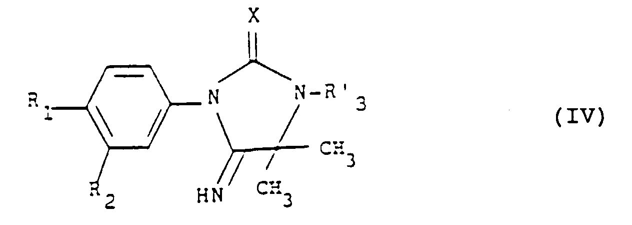 zu erhalten, worin R₁, R₂, X und R'₃ wie vorstehend definiert sind, wobei man die Verbindungen der Formel (IV), sofern notwendig oder erforderlich, einer oder mehreren der nachstehenden Reaktionen in beliebiger Reihenfolge unterwirft:
a) Eliminationsreaktion der gegebenenfalls vorhandenen Schutzgruppen, die R'₃ tragen kann;
b) Hydrolysereaktion der Gruppierung >C=NH zu der Ketonfunktion und gegebenenfalls Umwandlung der Gruppierung >C=S in die Gruppierung >C=O;
c) Transformationsreaktion der Gruppierung(en) >C=O in die Gruppierung >C=S;
d) Einwirkung eines Reagenses der Formel Hal-R"₃, worin R"₃ die Definitionen für R'₃, ausgenommen die Definition Wasserstoff, besitzt und Hal ein Halogenatom bedeutet, auf die Verbindungen der Formel (IV), worin R'₃ ein Wasserstoffatom bedeutet und nach Hydrolyse der Gruppierung >C=NH in die Ketonfunktion, um Verbindungen der Formel (I) zu erhalten, worin die Gruppierung -A-B- die Gruppierung 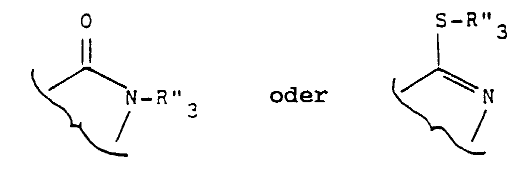 bedeutet, worin R"₃ wie vorstehend definiert ist, anschließend gegebenenfalls Einwirkung eines Mittels zur Abspaltung gegebenenfalls vorhandener Schutzgruppen, die R"₃ tragen kann, oder gegebenenfalls Einwirkung eines Mittels zur Veresterung, zur Überführung in ein Amid oder zur Salzbildung, auf diese Verbindungen,
oder ein Reagens der Formel Hal-R"₃, worin Hal und R"₃ wie vorstehend definiert sind, auf eine Verbindung der Formel (IV') 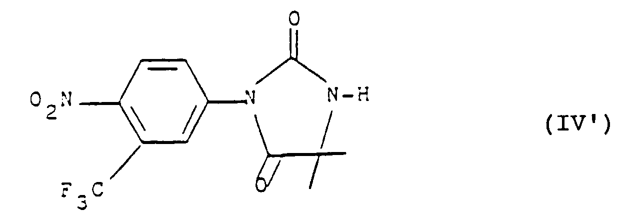 einwirken läßt, um eine Verbindung der Formel (IV") 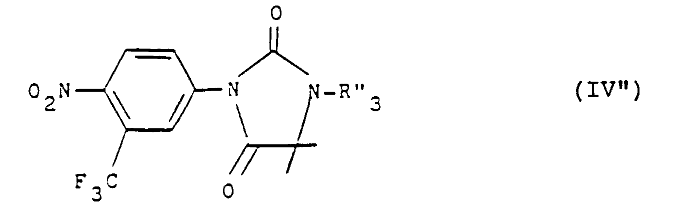 zu erhalten, wobei man die Verbindung der Formel (IV"), sofern notwendig oder erwünscht, einer oder mehreren der nachstehenden Reaktionen in beliebiger Reihenfolge unterwirft:
a) Eliminierungsreaktion gegebenenfalls vorhandener Schutzgruppen, die R"₃ tragen kann, dann gegebenenfalls Einwirkung eines Mittels zur Veresterung, zur Amidbildung oder zur Salzbildung;
b) Transformationsreaktion der Gruppierung(en) >C=O in die Gruppierungen >C=S.

2. Verfahren nach Anspruch 1 zur Herstellung einer Verbindung der Formel (I) nach Anspruch 1, worin Y ein Sauerstoffatom bedeutet, ausgenommen die Verbindungen, in denen die Gruppierungen -A-B- den Rest 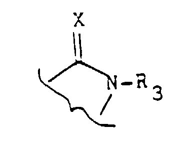 bedeutet, worin X ein Sauerstoffatom bedeutet, und R₃ ein Wasserstoffatom bedeutet, R₂ ein Halogenatom oder einen Trifluormethylrest bedeutet, und R₁ eine Nitrogruppe oder ein Halogenatom bedeutet, dadurch **gekennzeichnet,** daß man
entweder eine Verbindung der Formel (IV), wie in Anspruch 1 definiert, ausgenommen die Verbindungen, in denen X ein Sauerstoffatom bedeutet, R'₃ ein Wasserstoffatom bedeutet, R₂ ein Halogenatom oder einen Trifluormethylrest bedeutet, und R₁ eine Nitrogruppe oder ein Halogenatom bedeutet, einer oder mehreren der nachstehenden Reaktionen in beliebiger Reihenfolge unterwirft:
a) Eliminierungsreaktion gegebenenfalls vorhandener Schutzgruppierungen, die R'₃ tragen kann;
b) Hydrolysereaktion der Gruppierungen >C=NH in eine Ketonfunktion, anschließend gegebenenfalls Einwirkung eines Reagenses der Formel Hal-R"₃, worin R"₃ wie R'₃ definiert ist, ausgenommen die Definition Wasserstoff, und Hal ein Halogenatom bedeutet, um die Verbindungen der Formel (I) zu erhalten, worin die Gruppierung -A-B- die Gruppierung 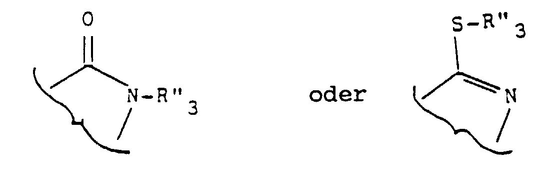 bedeutet, worin R"₃ wie vorstehend angegeben definiert ist, anschließend gegebenenfalls Einwirkung eines Mittels zur Abspaltung gegebenenfalls vorhandener Schutzgruppen, die R"₃ tragen kann, auf diese Verbindungen oder gegebenenfalls Einwirkung eines Mittels zur Veresterung, zur Amidbildung oder zur Salzbildung,
oder ein Reagens der Formel Hal-R"₃, worin Hal und R"₃ wie vorstehend definiert sind, mit einer Verbindung der Formel (IV'), wie in Anspruch 1 definiert, umsetzt, um eine Verbindung der Formel (IV") zu erhalten, die man, sofern notwendig oder erwünscht, einer Reaktion zur Abspaltung gegebenenfalls vorhandener Schutzgruppen, die R"₃ tragen kann, unterwirft und gegebenenfalls Einwirkung eines Mittels zur Veresterung, Amidbildung oder Salzbildung.

3. Verfahren nach Anspruch 1 oder 2 zur Herstellung einer Verbindung der Formel (I), wie in Anspruch 1 definiert, worin die Gruppierung -A-B- die Gruppierunq 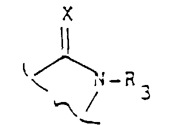 bedeutet, worin X ein Schwefelatom bedeutet, und R₃ wie vorstehend definiert ist, dadurch **gekennzeichnet,** daß man entweder von einer Verbindung der Formel (II) ausgeht, worin X ein Schwefelatom bedeutet, oder eine Verbindung der Formel (IV) oder (IV") einer oder mehreren der nachstehenden Reaktionen in beliebiger Reihenfolge unterwirft:
- Reaktion zur Abspaltung gegebenenfalls vorhandener Schutzgruppen, die R'₃ oder R"₃ tragen kann;
- Transformationsreaktion der Gruppierung(en) >C=O in die Gruppierung >C=S.

4. Verfahren nach Anspruch 3 zur Herstellung einer Verbindung der Formel (I) nach Anspruch 3, worin R₃ ein Wasserstoffatom oder einen Alkylrest mit höchstens 4 Kohlenstoffatomen, der gegebenenfalls durch eine Hydroxygruppe substituiert ist, bedeutet, dadurch **gekennzeichnet,** daß man entweder von einer Verbindung der Formel (III), worin R'₃, die vorstehend für R₃ angegebenen Bedeutung besitzt, und gegebenenfalls geschützt ist, ausgeht oder die Verbindung der Formel (IV') mit einem Reagens der Formel Hal-R"₃ behandelt, worin R"₃ die vorstehend für R₃ angegebenen Bedeutungen besitzt und gegebenenfalls geschützt ist, ausgenommen die Bedeutung Wasserstoff, anschließend gegebenenfalls die so erhaltene Verbindung der Wirkung eines Mittels zur Abspaltung der Schutzgruppen unterwirft.

5. Verfahren nach einem der Ansprüche 1 bis 4, dadurch **gekennzeichnet,** daß man von einer Verbindung der Formel (II), worin R₁ eine Cyanogruppe oder ein Halogenatom bedeutet, ausgeht.

6. Verfahren nach Anspruch 5, dadurch **gekennzeichnet,** daß man von einer Verbindung der Formel (II), worin R₁ ein Chloratom bedeutet, ausgeht.

7. Verfahren nach Anspruch 1 oder 2 zur Herstellung einer Verbindung der Formel (I), wie in Anspruch 1 definiert, worin die Gruppierung -A-B- eine Gruppierung 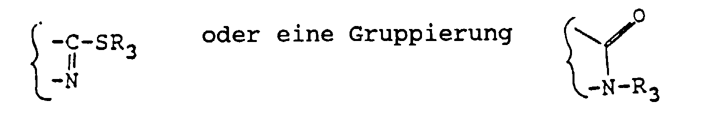 bedeutet, worin R₃ einen Alkyl- oder Alkenylrest mit höchstens -4 Kohlenstoffatomen oder einen gegebenenfalls substituierten Aralkylrest bedeutet, dadurch **gekennzeichnet,** daß man entweder von einer Verbindung der Formel (III), worin R'₃ die für R₃ vorstehend angegebenen Bedeutungen besitzt, oder einem Reagens der Formel HalR"₃, worin R"₃ die vorstehend gegebenen Bedeutungen für R₃ besitzt, ausgeht.

## Patentansprüche (Patentansprüche für folgende(n) Vertragsstaat(en): GR)

1. Verfahren zur Herstellung der Verbindungen der allgemeinen Formel (I) 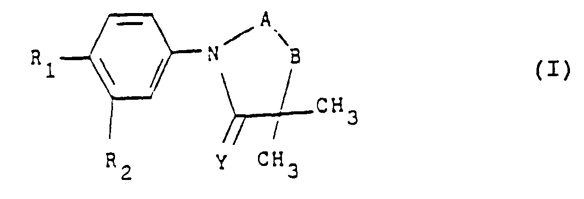 worin
R₁ eine Cyano- oder Nitrogruppe oder ein Halogenatom bedeutet,
R₂ einen Trifluormethylrest oder ein Halogenatom bedeutet, die Gruppierung -A-B- aus den Resten 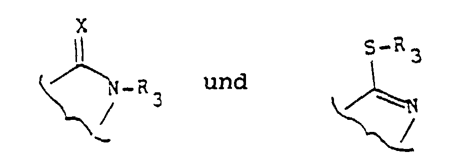 ausgewählt ist, worin X ein Sauerstoffatom oder ein Schwefelatom bedeutet, und R₃ aus den nachstehenden Resten ausgewählt ist:
- ein Wasserstoffatom,
- Alkyl-, Alkenyl-, Alkinyl-, Aryl- oder Arylalkylreste mit höchstens 12 Kohlenstoffatomen, wobei diese Reste gegebenenfalls durch einen oder mehrere Substituenten, ausgewählt aus Hydroxyl-, Halogen-, Mercapto-, Cyano-, Acyl- oder Acyloxyresten mit höchstens 7 Kohlenstoffatomen, gegebenenfalls substituiertem S-Aryl, worin das Schwefelatom gegebenenfalls in Form eines Sulfoxids oder Sulfons oxidiert ist, freier, veresterter, in ein Amid oder Salz überführter Carboxylgruppe, Amino-, Mono- oder Dialkylaminogruppe oder einem heterocyclischen Rest, umfassend 3 bis 6 Kettenglieder und umfassend ein oder mehrere Heteroatome, ausgewählt aus Schwefel-, Sauerstoff- oder Stickstoffatomen, substituiert sind, wobei die Alkyl-, Alkenyl- oder Alkinylreste außerdem gegebenenfalls durch ein oder mehrere Sauerstoff-, Stickstoff- oder Schwefelatom(e), das/die gegebenenfalls in Form des Sulfoxids oder Sulfons oxidiert ist/sind, unterbrochen sind, wobei die Aryl- und Aralkylreste außerdem gegebenenfalls durch einen Alkyl-, Alkenyl- oder Alkinylrest, Alkoxy-, Alkenyloxy-, Alkinyloxy- oder Trifluormethylrest substituiert sind,
Y ein Sauerstoff- oder Schwefelatom oder die Gruppe =NH bedeutet, ausgenommen die Verbindungen, in denen die Gruppierung -A-B- den Rest 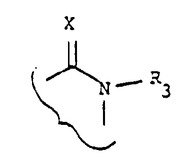 bedeutet, worin X ein Sauerstoffatom bedeutet, und R₃ ein Wasserstoffatom bedeutet, und Y ein Sauerstoffatom oder die Gruppe NH bedeutet, und R₂ ein Halogenatom oder einen Trifluormethylrest bedeutet, und R₁ eine Nitrogruppe oder ein Halogenatom bedeutet, dadurch gekennzeichnet, daß man entweder in Gegenwart einer tertiären Base eine Verbindung der Formel (II) 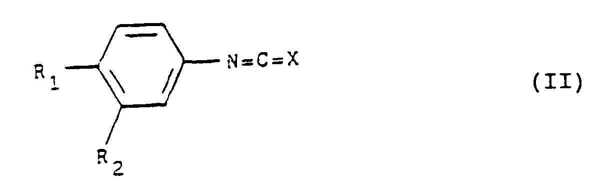 worin R₁, R₂ und X wie vorstehend definiert sind, mit einer Verbindung der Formel (III) 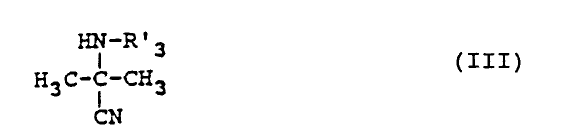 worin R'₃ wie vorstehend für R₃ definiert ist, worin die gegebenenfalls vorhandenen reaktiven Funktionen gegebenenfalls geschützt sind und mit der Maßgabe, daß, wenn R₁ eine Nitrogruppe oder ein Halogenatom bedeutet, wenn R₂ ein Halogenatom oder den Rest CF₃ bedeutet und X ein Sauerstoffatom bedeutet, R'₃ kein Wasserstoffatom bedeuten kann, umsetzt, um eine Verbindung der Formel (IV) 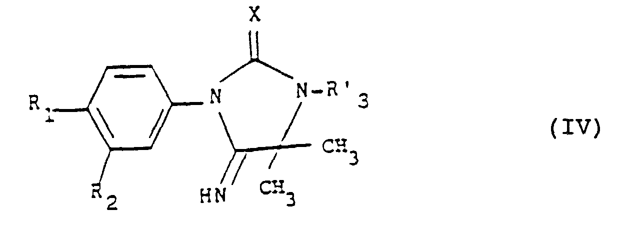 zu erhalten, worin R₁, R₂, X und R'₃ wie vorstehend definiert sind, wobei man die Verbindungen der Formel (IV), sofern notwendig oder erforderlich, einer oder mehreren der nachstehenden Reaktionen in beliebiger Reihenfolge unterwirft:
a) Eliminationsreaktion der gegebenenfalls vorhandenen Schutzgruppen, die R'₃ tragen kann;
b) Hydrolysereaktion der Gruppierung >C=NH zu der Ketonfunktion und gegebenenfalls Umwandlung der Gruppierung >C=S in die Gruppierung >C=O;
c) Transformationsreaktion der Gruppierung(en) >C=O in die Gruppierung >C=S;
d) Einwirkung eines Reagenses der Formel Hal-R"₃, worin R"₃ die Definitionen für R'₃, ausgenommen die Definition Wasserstoff, besitzt und Hal ein Halogenatom bedeutet, auf die Verbindungen der Formel (IV), worin R'₃ ein Wasserstoffatom bedeutet und nach Hydrolyse der Gruppierung >C=NH in die Ketonfunktion, um Verbindungen der Formel (I) zu erhalten, worin die Gruppierung -A-B- die Gruppierung 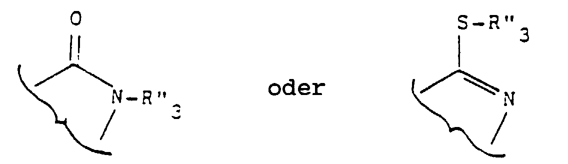 bedeutet, worin R"₃ wie vorstehend definiert ist, anschließend gegebenenfalls Einwirkung eines Mittels zur Abspaltung gegebenenfalls vorhandener Schutzgruppen, die R"₃ tragen kann, oder gegebenenfalls Einwirkung eines Mittels zur Veresterung, zur Überführung in ein Amid oder zur Salzbildung, auf diese Verbindungen,
oder ein Reagens der Formel Hal-R"₃, worin Hal und R"₃ wie vorstehend definiert sind, auf eine Verbindung der Formel (IV') 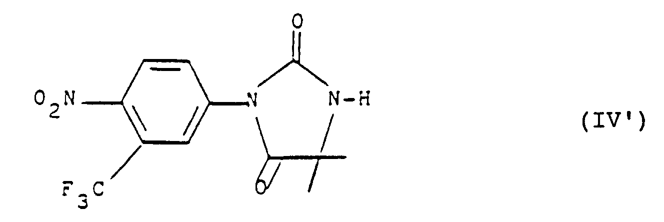 einwirken läßt, um eine Verbindung der Formel (IV") 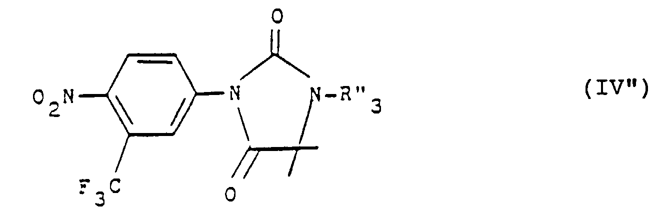 zu erhalten, wobei man die Verbindung der Formel (IV"), sofern notwendig oder erwünscht, einer oder mehreren der nachstehenden Reaktionen in beliebiger Reihenfolge unterwirft:
a) Eliminierungsreaktion gegebenenfalls vorhandener Schutzgruppen, die R"₃ tragen kann, dann gegebenenfalls Einwirkung eines Mittels zur Veresterung, zur Amidbildung oder zur Salzbildung;
b) Transformationsreaktion der Gruppierung(en) >C=O in die Gruppierungen >C=S.

2. Verfahren nach Anspruch 1 zur Herstellung einer Verbindung der Formel (I) nach Anspruch 1, worin Y ein Sauerstoffatom bedeutet, ausgenommen die Verbindungen, in denen die Gruppierungen -A-B- den Rest 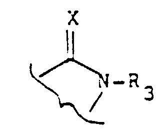 bedeutet, worin X ein Sauerstoffatom bedeutet, und R₃ ein Wasserstoffatom bedeutet, R₂ ein Halogenatom oder einen Trifluormethylrest bedeutet, und R₁ eine Nitrogruppe oder ein Halogenatom bedeutet, dadurch **gekennzeichnet,** daß man
entweder eine Verbindung der Formel (IV), wie in Anspruch 1 definiert, ausgenommen die Verbindungen, in denen X ein Sauerstoffatom bedeutet, R'₃ ein Wasserstoffatom bedeutet, R₂ ein Halogenatom oder einen Trifluormethylrest bedeutet, und R₁ eine Nitrogruppe oder ein Halogenatom bedeutet, einer oder mehreren der nachstehenden Reaktionen in beliebiger Reihenfolge unterwirft:
a) Eliminierungsreaktion gegebenenfalls vorhandener Schutzgruppierungen, die R'₃ tragen kann;
b) Hydrolysereaktion der Gruppierungen >C=NH in eine Ketonfunktion, anschließend gegebenenfalls Einwirkung eines Reagenses der Formel Hal-R"₃, worin R"₃ wie R'₃ definiert ist, ausgenommen die Definition Wasserstoff, und Hal ein Halogenatom bedeutet, um die Verbindungen der Formel (I) zu erhalten, worin die Gruppierung -A-B- die Gruppierung 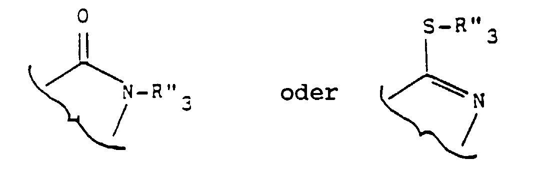 bedeutet, worin R"₃ wie vorstehend angegeben definiert ist, anschließend gegebenenfalls Einwirkung eines Mittels zur Abspaltung gegebenenfalls vorhandener Schutzgruppen, die R"₃ tragen kann, auf diese Verbindungen oder gegebenenfalls Einwirkung eines Mittels zur Veresterung, zur Amidbildung oder zur Salzbildung,
oder ein Reagens der Formel Hal-R"₃, worin Hal und R"₃ wie vorstehend definiert sind, mit einer Verbindung der Formel (IV'), wie in Anspruch 1 definiert, umsetzt, um eine Verbindung der Formel (IV") zu erhalten, die man, sofern notwendig oder erwünscht, einer Reaktion zur Abspaltung gegebenenfalls vorhandener Schutzgruppen, die R"₃ tragen kann, unterwirft und gegebenenfalls Einwirkung eines Mittels zur Veresterung, Amidbildung oder Salzbildung.

3. Verfahren nach Anspruch 1 oder 2 zur Herstellung einer Verbindung der Formel (I), wie in Anspruch 1 definiert. worin die Gruppierung -A-B- die Gruppierung 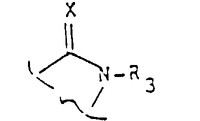 bedeutet, worin X ein Schwefelatom bedeutet und R₃ wie vorstehend definiert ist, dadurch **gekennzeichnet,** daß man entweder von einer Verbindung der Formel (II) ausgeht, worin X ein Schwefelatom bedeutet, oder eine Verbindung der Formel (IV) oder (IV") einer oder mehreren der nachstehenden Reaktionen in beliebiger Reihenfolge unterwirft:
- Reaktion zur Abspaltung gegebenenfalls vorhandener Schutzgruppen, die R'₃ oder R"₃ tragen kann;
- Transfonmationsreaktion der Gruppierung(en) >C=O in die Gruppierung >C=S.

4. Verfahren nach Anspruch 3 zur Herstellung einer Verbindung der Formel (I) nach Anspruch 3, worin R₃ ein Wasserstoffatom oder einen Alkylrest mit höchstens 4 Kohlenstoffatomen, der gegebenenfalls durch eine Hydroxygruppe substituiert ist, bedeutet, dadurch **gekennzeichnet,** daß man entweder von einer Verbindung der Formel (III), worin R'₃ die vorstehend für R₃ angegebene Bedeutung besitzt und gegebenenfalls geschützt ist, ausgeht oder die Verbindung der Formel (IV') mit einem Reagens der Formel Hal-R"₃ behandelt, worin R"₃ die vorstehend für R₃ angegebenen Bedeutungen besitzt und gegebenenfalls geschützt ist, ausgenommen die Bedeutung Wasserstoff, anschließend gegebenenfalls die so erhaltene Verbindung der Wirkung eines Mittels zur Abspaltung der Schutzgruppen unterwirft.

5. Verfahren nach einem der Ansprüche 1 bis 4, dadurch **gekennzeichnet,** daß man von einer Verbindung der Formel (II), worin R₁ eine Cyanogruppe oder ein Halogenatom bedeutet, ausgeht.

6. Verfahren nach Anspruch 5, dadurch **gekennzeichnet,** daß man von einer Verbindung der Formel (II), worin R₁ ein Chloratom bedeutet, ausgeht.

7. Verfahren nach Anspruch 1 oder 2 zur Herstellung einer Verbindung der Formel (I), wie in Anspruch 1 definiert, worin die Gruppierung -A-B- eine Gruppierung 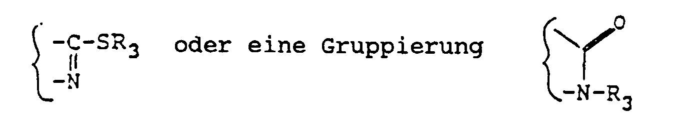 bedeutet, worin R₃ einen Alkyl- oder Alkenylrest mit höchstens 4 Kohlenstoffatomen oder einen gegebenenfalls substituierten Aralkylrest bedeutet, dadurch **gekennzeichnet,** daß man entweder von einer Verbindung der Formel (III), worin R'₃ die für R₃ vorstehend angegebenen Bedeutungen besitzt, oder einem Reagens der Formel HalR"₃, worin R"₃ die vorstehend gegebenen Bedeutungen für R₃ besitzt, ausgeht.

8. Verfahren nach einem der Ansprüche 1 bis 5, dadurch **gekennzeichnet,** daß man die Verbindungen und Ausgangsreagentien so herstellt, daß man eine der nachstehenden Verbindungen herstellt, nämlich:
- 4-(5-Oxo-2-thioxo-3,4,4-trimethyl-1-imidazolidinyl)-2(trifluormethyl)benzonitril,
- 4-(4,4-Dimethyl-5-oxo-2-thioxo-1-imidazolidinyl)-2(trifluormethyl)benzonitril,
- 4-[4,4-Dimethyl-3-(2-hydroxyethyl)-5-oxo-2-thioxo-1-imidazolidinyl]-2-(trifluormethyl)benzonitril,
- 3-(3,4-Dichlorphenyl)-2-thioxo-1,5,5-trimethyl-4-imidazolidinon.

9. Verfahren nach Anspruch 1, 2 oder 7, dadurch **gekennzeichnet,** daß man die Verbindungen und Ausgangsreagentien so herstellt, daß man eine der nachstehenden Verbindungen herstellt, nämlich:
- 1-(4-Nitro-3-(trifluormethyl)phenyl)-3,4,4-trimethyl-2,5-imidazolidindion,
- 4-[[4,5-Dihydro-4,4-dimethyl-5-oxo-2-(phenylmethyl)thio]-1H-imidazol-1-yl]-2-(trifluormethyl)benzonitril.

10. Verfahren zur Herstellung von pharmazeutischen Präparaten, dadurch **gekennzeichnet,** daß man als Wirkstoff mindestens eine der Verbindungen der Formel (I) nach Anspruch 1 in einer für diese Verwendung bestimmten Form einsetzt.

11. Verfahren zur Herstellung von pharmazeutischen Präparaten, dadurch **gekennzeichnet,** daß man als Wirkstoff mindestens eine der Verbindungen der Formel (I) nach einem der Ansprüche 2 bis 7 in einer für diese Verwendung bestimmten Form einsetzt.

12. Verfahren zur Herstellung von pharmazeutischen Präparaten, dadurch **gekennzeichnet,** daß man als Wirkstoff mindestens eine der Verbindungen der Formel (I) nach Anspruch 8 oder 9 in einer für diese Verwendung bestimmten Form einsetzt.

## Claims (Claims for the following Contracting State(s): AT, BE, CH, DE, DK, FR, GB, IT, LI, LU, NL, PT, SE)

1. The products of general formula (I): 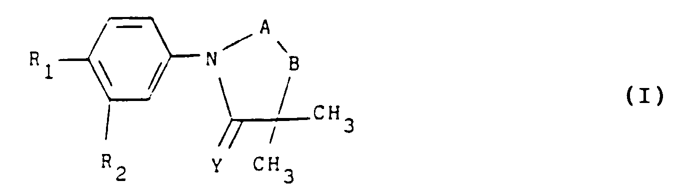 in which:
R₁ represents a cyano or nitro radical or a halogen atom,
R₂ represents a trifluoromethyl radical or a halogen atom, the group -A-B- is chosen from the radicals: 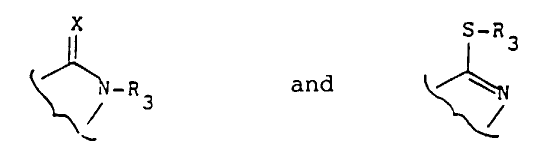 in which X represents an oxygen or sulphur atom and R₃ is chosen from the following radicals:
- a hydrogen atom,
- alkyl, alkenyl, alkynyl, aryl or arylalkyl radicals having at most 12 carbon atoms, these radicals being optionally substituted by one or more substituents chosen from the following radicals: hydroxy, halogen, mercapto, cyano, acyl or acyloxy having at most 7 carbon atoms, optionally substituted S-aryl, in which the sulphur atom is optionally oxidized in the form of the sulphoxide or sulphone, free, esterified, amidified or salified carboxy, amino, mono- or dialkylamino or a heterocyclic radical having 3 to 6 links and containing one or more heteroatoms chosen from sulphur, oxygen or nitrogen atoms,
the alkyl, alkenyl or alkynyl radicals being moreover optionally interrupted by one or more oxygen, nitrogen or sulphur atoms optionally oxidized in the form of the sulphoxide or sulphone,
the aryl and aralkyl radicals moreover being optionally substituted by one of the following radicals: alkyl, alkenyl or alkynyl, alkoxy, alkenyloxy, alkynyloxy or trifluoromethyl,
Y represents an oxygen or sulphur atom or an =NH radical, with the exception of the products in which the group -A-B- represents the radical: 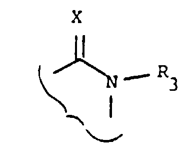 in which X represents an oxygen atom and R₃ represents a hydrogen atom and Y represents an oxygen atom or an NH radical and R₂ represents a halogen atom or a trifluoromethyl radical and R₁ represents a nitro radical or a halogen atom.

2. The products of formula (I) as defined in claim 1, in which Y represents an oxygen atom, with the exception of the products in which the group -A-B- represents the radical: 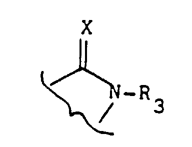 in which X represents an oxygen atom and R₃ represents a hydrogen atom, R₂ represents a halogen atom or a trifluoromethyl radical and R₁ represents a nitro radical or a halogen atom.

3. The products of formula (I) as defined in claim 1 or 2 in which the group -A-B- represents the group: 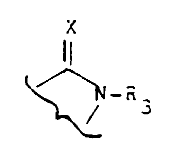 in which X represents a sulphur atom and R₃ has the meaning indicated in claim 1.

4. The products of formula (I) according to claim 3, in which R₃ represents a hydrogen atom or an alkyl radical having at most 4 carbon atoms optionally substituted by a hydroxy radical.

5. The products of formula (I) as defined in any one of claims 1 to 4, in which R₁ represents a cyano radical or a halogen atom.

6. The products of formula (I) according to claim 5, in which R₁ represents a chlorine atom.

7. The products of formula (I) as defined in claim 1 or 2, in which the qroup -A-B- represents a group: 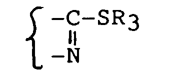 or a group: 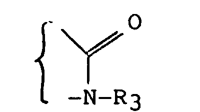 in which R₃ represents an alkyl or alkenyl radical having at most 4 carbon atoms or an optionally substituted aralkyl radical.

8. The products of formula (I) as defined in any one of claims 1 to 5 of which the names follow:
- 4-(5-oxo-2-thioxo-3,4,4-trimethyl-1-imidazolidinyl)-2(trifluoromethyl)-benzonitrile,
- 4-(4,4-dimethyl-5-oxo-2-thioxo-1-imidazolidinyl)-2(trifluoromethyl)-benzonitrile,
- 4-[4,4-dimethyl 3-(2-hydroxyethyl) 5-oxo 2-thioxo 1-imidazolidinyl] 2-(trifluoromethyl) benzonitrile,
- 3-(3,4-dichlorophenyl) 2-thioxo 1,5,5-trimethyl 4-imidazolidinone.

9. The products of formula (I) as defined in any one of claims 1, 2 and 7, of which the names follow:
- 1-(4-nitro-3-(trifluoromethyl) phenyl)-3,4,4-trimethyl-2,5-imidazolidinedione,
- 4-[[4,5-dihydro 4,4-dimethyl 5-oxo 2-(phenylmethyl) thio] 1H-imidazol-1-yl] 2-(trifluoromethyl) benzonitrile.

10. Preparation process for the products of general formula (I) as defined in claim 1, characterized in that:
either a product of formula (II): 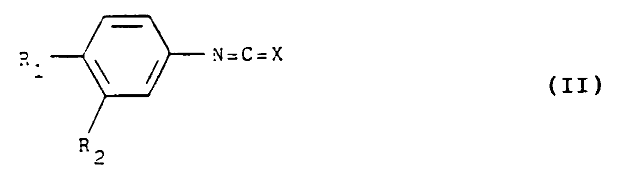 in which R₁, R₂ and X have the meaning indicated above, is reacted in the presence of a tertiary base with a product of formula (III): 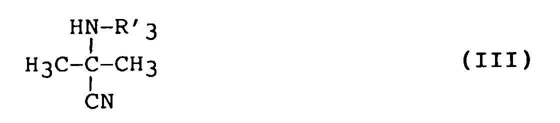 in which R'₃ has the values indicated above for R₃ in which the optional reactive functions are optionally protected and it being understood that if R₁ represents a nitro radical or a halogen atom, if R₂ represents a halogen atom or a CF₃ radical and X represents an oxygen atom, R'₃ cannot represent a hydrogen atom, in order to obtain a product of formula (IV): 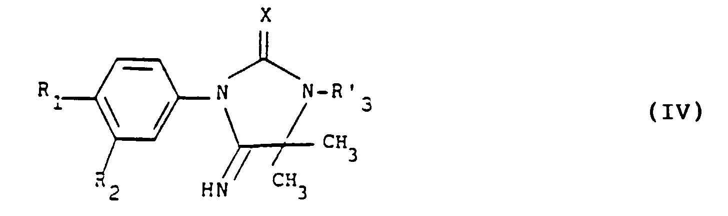 in which R₁, R₂, X and R'₃ have the previous meaning, which products of formula (IV) are, if necessary or if desired, subjected to any one or more of the following reactions, in any order:
a) elimination reaction of the optional protective groups which can be carried by R'₃;
b) hydrolysis reaction of the >C=NH group into a ketone function and if appropriate conversion of the >C=S group into the >C=O group;
c) conversion reaction of the >C=O group or groups into the >C=S group;
d) the action on the products of formula (IV) in which R'₃ represents a hydrogen atom, and after hydrolysis of the >C=NH group into a ketone function, of a reagent of formula Hal-R"₃ in which R"₃ has the values of R'₃ with the exception of the hydrogen value and Hal represents a halogen atom in order to obtain products of formula (I) in which the group -A-B- represents the group 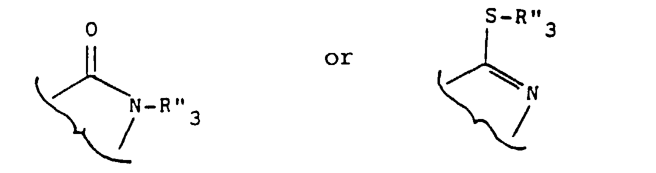 in which R"₃ has the meaning indicated previously then, if desired, the action on these products of an elimination agent of the optional protective groups which can be carried by R"₃ or if appropriate, the action of an esterification,
amidification or salification agent,
or a reagent of formula Hal-R"₃ in which Hal and R"₃ have the values indicated previously is reacted on a product of formula (IV'): 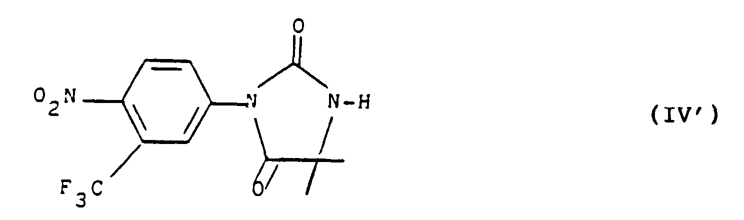 in order to obtain a product of formula (IV"): 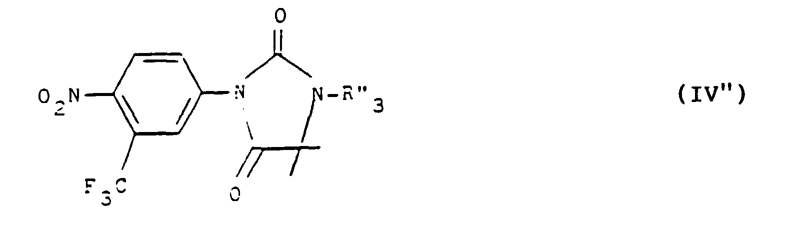 which product of formula (IV") is, if necessary or if desired, subjected to any one or more of the following reactions in any order:
a) elimination reaction of the optional protective groups which can be carried by R"₃ then if appropriate the action of an esterification, amidification or salification agent;
b) conversion reaction of the >C=O group or groups into >C=S groups.

11. As medicaments, the pharmaceutically acceptable products of formula (I) as defined in claims 1 to 7.

12. As medicaments, the products of formula (I) as defined in claim 8 or 9.

13. The pharmaceutical compositions containing, as active ingredient, at least one of the medicaments as defined in any one of claims 11 and 12.

14. The products of formula (IVi): 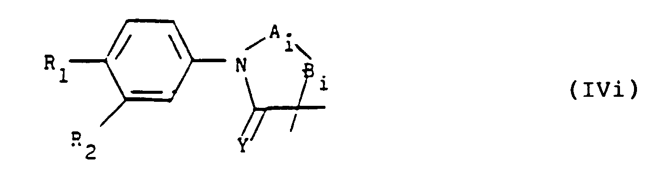 in which R₁, R₂ and Y have the meanings indicated in claim 1 and the group: 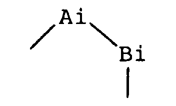 is chosen from the radicals: 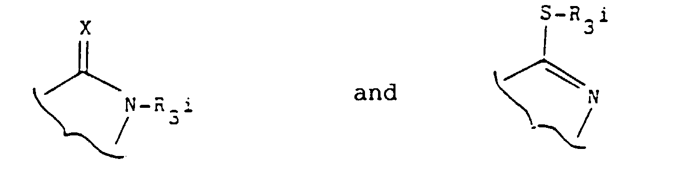 in which X represents an oxygen or sulphur atom and R₃ᵢ is chosen from the values of R₃ containing a protected reactive function,
with the exception of the products in which the group -Aᵢ-Bᵢ-represents the radical: 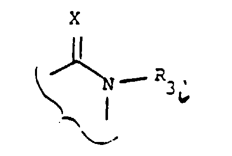 in which X represents an oxygen atom and R₃ᵢ represents a hydrogen atom and Y represents an oxygen atom or an NH radical and R₂ represents a halogen atom or a trifluoromethyl radical and R₁ represents a nitro radical or a halogen atom.

## Claims (Claims for the following Contracting State(s): ES)

1. Process for preparing the products of general formula (I): 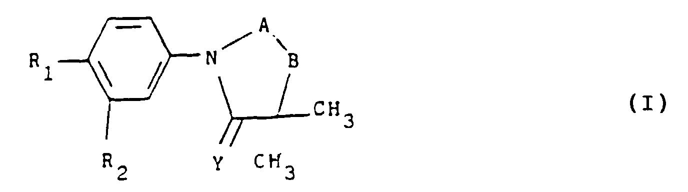 in which:
R₁ represents a cyano or nitro radical or a halogen atom,
R₂ represents a trifluoromethyl radical or a halogen atom, the group -A-B- is chosen from the radicals: 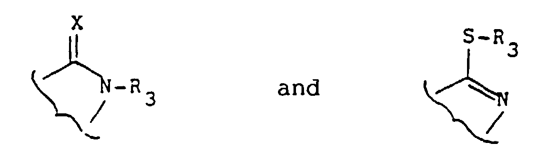 in which X represents an oxygen or sulphur atom and R₃ is chosen from the following radicals:
- a hydrogen atom,
- alkyl, alkenyl, alkynyl, aryl or arylalkyl radicals having at most 12 carbon atoms, these radicals being optionally substituted by one or more substituents chosen from the following radicals: hydroxy, halogen, mercapto, cyano, acyl or acyloxy having at most 7 carbon atoms, optionally substituted S-aryl, in which the sulphur atom is optionally oxidized in the form of the sulphoxide or sulphone, free, esterified, amidified or salified carboxy, amino, mono- or dialkylamino or a heterocyclic radical having 3 to 6 links and containing one or more heteroatoms chosen from sulphur, oxygen or nitrogen atoms,
the alkyl, alkenyl or alkynyl radicals being moreover optionally interrupted by one or more oxygen, nitrogen or sulphur atoms optionally oxidized in the form of the sulphoxide or sulphone,
the aryl and aralkyl radicals moreover being optionally substituted by one of the following radicals: alkyl, alkenyl or alkynyl, alkoxy, alkenyloxy, alkynyloxy or trifluoromethyl,
Y represents an oxygen or sulphur atom or an =NH radical, with the exception of the products in which the group -A-B- represents the radical: 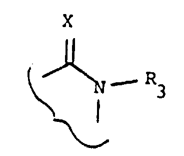 in which X represents an oxygen atom and R₃ represents a hydrogen atom and Y represents an oxygen atom or an NH radical and R₂ represents a halogen atom or a trifluoromethyl radical and R₁ represents a nitro radical or a halogen atom, characterized in that:
either a product of formula (II): 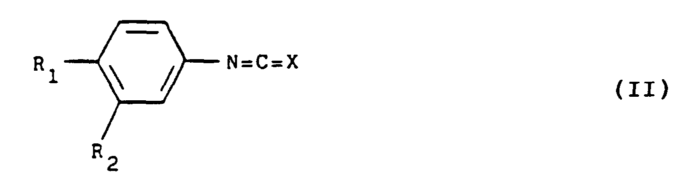 in which R₁, R₂ and X have the meaning indicated above, is reacted in the presence of a tertiary base with a product of formula (III): 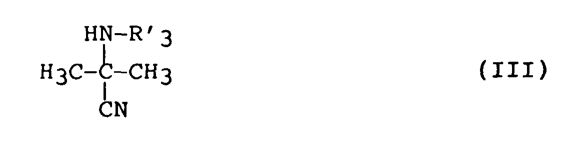 in which R'₃ has the values indicated above for R₃ in which the optional reactive functions are optionally protected and it being understood that if R₁ represents a nitro radical or a halogen atom, if R₂ represents a halogen atom or a CF₃ radical and X represents an oxygen atom, R'₃ cannot represent a hydrogen atom, in order to obtain a product of formula (IV): 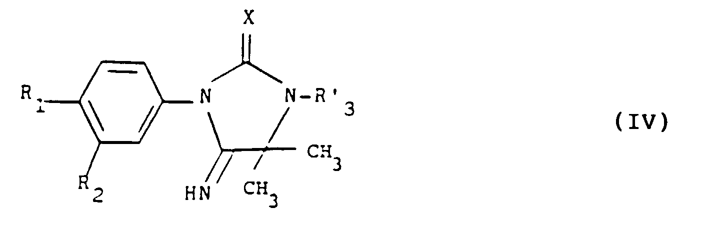 in which R₁, R₂, X and R'₃ have the previous meaning, which products of formula (IV) are, if necessary or if desired, subjected to any one or more of the following reactions, in any order:
a) elimination reaction of the optional protective groups which can be carried by R'₃;
b) hydrolysis reaction of the >C=NH group into a ketone function and if appropriate conversion of the >C=S group into the >C=O group;
c) conversion reaction of the >C=O group or groups into the >C=S group;
d) the action on the products of formula (IV) in which R'₃ represents a hydrogen atom, and after hydrolysis of the >C=NH group into a ketone function, of a reagent of formula Hal-R"₃ in which R"₃ has the values of R'₃ with the exception of the hydrogen value and Hal represents a halogen atom in order to obtain products of formula (I) in which the group -A-B- represents the group 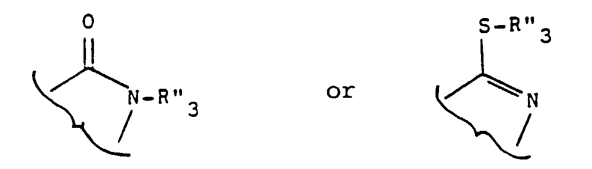 in which R"₃ has the meaning indicated previously then, if desired, the action on these products of an elimination agent of the optional protective groups which can be carried by R"₃ or if appropriate, the action of an esterification,
amidification or salification agent,
or a reagent of formula Hal-R"₃ in which Hal and R"₃ have the values indicated previously is reacted on a product of formula (IV'): 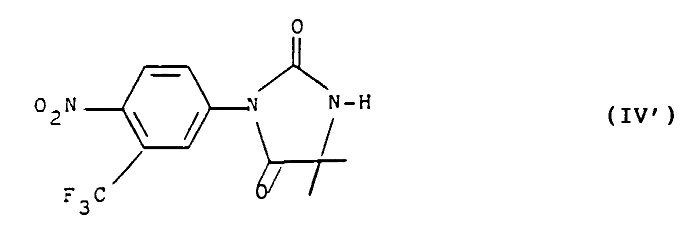 in order to obtain a product of formula (IV"): 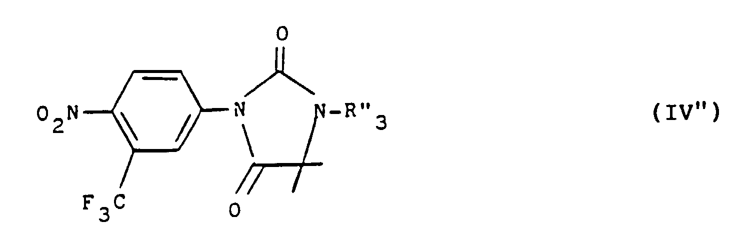 which product of formula (IV") is, if necessary or if desired, subjected to any one or more of the following reactions in any order:
a) elimination reaction of the optional protective groups which can be carried by R"₃ then if appropriate the action of an esterification, amidification or salification agent;
b) conversion reaction of the >C=O group or groups into >C=S groups.

2. Process according to claim 1 for preparing a product of formula (I) as defined in claim 1, in which Y represents an oxygen atom, with the exception of the products in which the group -A-B- represents the radical: 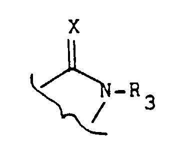 in which X represents an oxygen atom and R₃ represents a hydrogen atom, R₂ represents a halogen atom or a trifluoromethyl radical and R₁ represents a nitro radical or a halogen atom, characterized in that
either a compound of formula (IV) as defined in claim 1 with the exception of the products in which X represents an oxygen atom, R'₃ represents a hydrogen atom, R₂ represents a halogen atom or a trifluoromethyl radical and R₁ represents a nitro radical or a halogen atom, is subjected to any one or more of the following reactions, in any order:
a) elimination reaction of the optional protective groups which can be carried by R'₃;
b) hydrolysis reaction of the >C=NH group into the ketone function then if desired the action of a reagent of formula Hal-R"₃ in which R"₃ has the values of R'₃ with the exception of the hydrogen value and Hal represents a halogen atom, in order to obtain products of formula (I) in which the group -A-B- represents the group 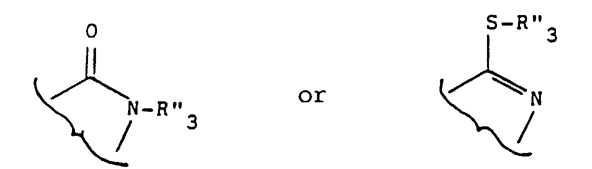 in which R"₃ has the meaning indicated previously then, if desired, the action on these products of an elimination agent of the optional protective groups which can be carried by R"₃ or if appropriate, the action of an esterification,
amidification or salification agent,
or a reagent of formula Hal-R"₃ in which Hal and R"₃ have the values indicated previously is reacted on a product of formula (IV') as defined in claim 1 in order to obtain a product of formula (IV") which, if necessary or if desired, is subjected to an elimination reaction of the optional protective groups which can be carried by R"₃ and if appropriate to the action of an esterification, amidification or salification agent.

3. Process according to claim 1 or 2 for preparing a product of formula (I) as defined in claim 1 in which the group -A-B- represents the group 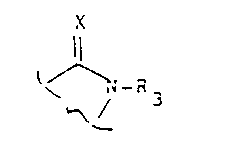 in which X represents a sulphur atom and R₃ has the meaning indicated previously, characterized in that either a product of formula (II) is used at the start in which X represents a sulphur atom, or a product of formula (IV) or (IV") is subjected to any one or more of the following reactions, in any order:
- elimination reaction of the optional protective groups which can be carried by R'₃ or R"₃;
- conversion reaction of the >C=O group or groups into the >C=S group.

4. Process according to claim 3 for preparing a product of formula (I) as defined in claim 3 in which R₃ represents a hydrogen atom or an alkyl radical having at most 4 carbon atoms optionally substituted by a hydroxy radical,
characterized in that either a product of formula (III) is used at the start in which R'₃ has the optionally protected values of R₃ indicated above, or the product of formula (IV') is treated with a reagent of formula Hal-R"₃ in which R"₃ has the optionally protected values of R₃ indicated above with the exception of the hydrogen value then if appropriate the product obtained is subjected to the action of an elimination agent of the protective groups.

5. Process according to any one of claims 1 to 4, characterized in that a product of formula (II) is used at the start in which R₁ represents a cyano radical or a halogen atom.

6. Process according to claim 5, characterized in that a product of formula (II) is used at the start in which R₁ represents a chlorine atom.

7. Process according to claim 1 or 2 for preparing a product of formula (I) as defined in claim 1 in which the group -A-B- represents a group 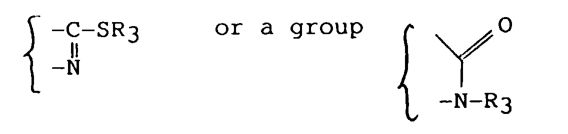 in which R₃ represents an alkyl or alkenyl radical having at most 4 carbon atoms or an optionally substituted aralkyl radical, characterized in that there is used at the start either a product of formula (III) in which R'₃ has the values of R₃ indicated above, or a reagent of formula Hal-R"₃ in which R"₃ has the values of R₃ indicated above.

## Claims (Claims for the following Contracting State(s): GR)

1. Process for preparing the products of general formula (I): 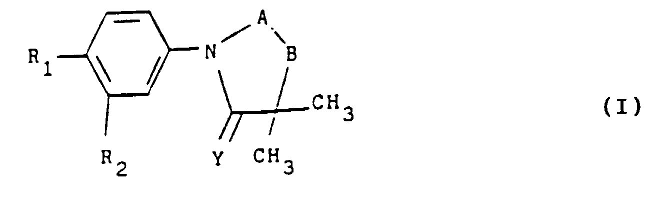 in which:
R₁ represents a cyano or nitro radical or a halogen atom,
R₂ represents a trifluoromethyl radical or a halogen atom, the group -A-B- is chosen from the radicals: 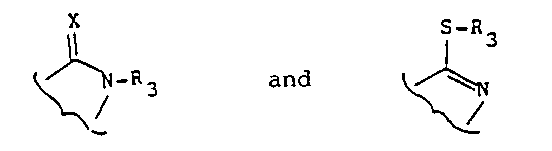 in which X represents an oxygen or sulphur atom and R₃ is chosen from the following radicals:
- a hydrogen atom,
- alkyl, alkenyl, alkynyl, aryl or arylalkyl radicals having at most 12 carbon atoms, these radicals being optionally substituted by one or more substituents chosen from the following radicals: hydroxy, halogen, mercapto, cyano, acyl or acyloxy having at most 7 carbon atoms, optionally substituted S-aryl, in which the sulphur atom is optionally oxidized in the form of the sulphoxide or sulphone, free, esterified, amidified or salified carboxy, amino, mono- or dialkylamino or a heterocyclic radical having 3 to 6 links and containing one or more heteroatoms chosen from sulphur, oxygen or nitrogen atoms,
the alkyl, alkenyl or alkynyl radicals being moreover optionally interrupted by one or more oxygen, nitrogen or sulphur atoms optionally oxidized in the form of the sulphoxide or sulphone,
the aryl and aralkyl radicals moreover being optionally substituted by one of the following radicals: alkyl, alkenyl or alkynyl, alkoxy, alkenyloxy, alkynyloxy or trifluoromethyl,
Y represents an oxygen or sulphur atom or an =NH radical, with the exception of the products in which the group -A-B- represents the radical: 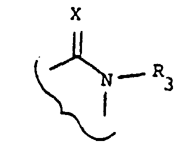 in which X represents an oxygen atom and R₃ represents a hydrogen atom and Y represents an oxygen atom or an NH radical and R₂ represents a halogen atom or a trifluoromethyl radical and R₁ represents a nitro radical or a halogen atom, characterized in that:
either a product of formula (II): 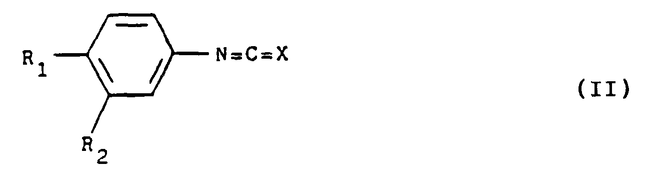 in which R₁, R₂ and X have the meaning indicated above, is reacted in the presence of a tertiary base with a product of formula (III): 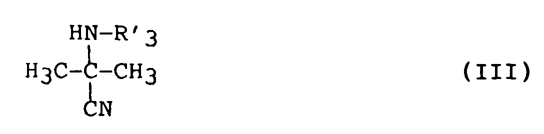 in which R'₃ has the values indicated above for R₃ in which the optional reactive functions are optionally protected and it being understood that if R₁ represents a nitro radical or a halogen atom, if R₂ represents a halogen atom or a CF₃ radical and X represents an oxygen atom, R'₃ cannot represent a hydrogen atom, in order to obtain a product of formula (IV): 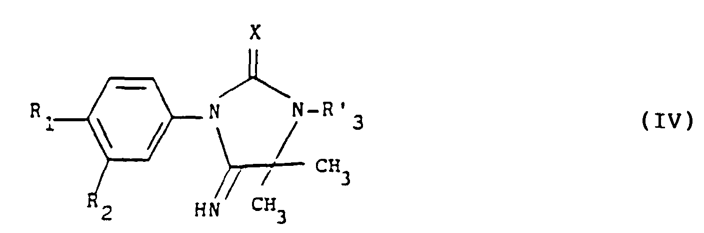 in which R₁, R₂, X and R'₃ have the previous meaning, which products of formula (IV) are, if necessary or if desired, subjected to any one or more of the following reactions, in any order:
a) elimination reaction of the optional protective groups which can be carried by R'₃;
b) hydrolysis reaction of the >C=NH group into a ketone function and if appropriate conversion of the >C=S group into the >C=O group;
c) conversion reaction of the >C=O group or groups into the >C=S group;
d) the action on the products of formula (IV) in which R'₃ represents a hydrogen atom, and after hydrolysis of the >C=NH group into a ketone function, of a reagent of formula Hal-R"₃ in which R"₃ has the values of R'₃ with the exception of the hydrogen value and Hal represents a halogen atom in order to obtain products of formula (I) in which the group -A-B- represents the group 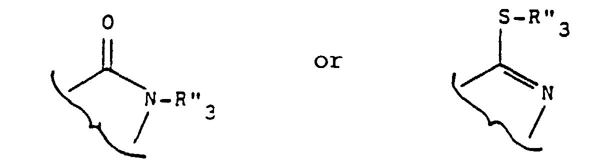 in which R"₃ has the meaning indicated previously then, if desired, the action on these products of an elimination agent of the optional protective groups which can be carried by R"₃ or if appropriate, the action of an esterification,
amidification or salification agent,
or a reagent of formula Hal-R"₃ in which Hal and R"₃ have the values indicated previously is reacted on a product of formula (IV'): 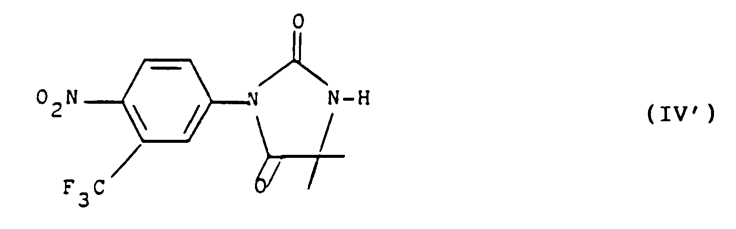 in order to obtain a product of formula (IV"): 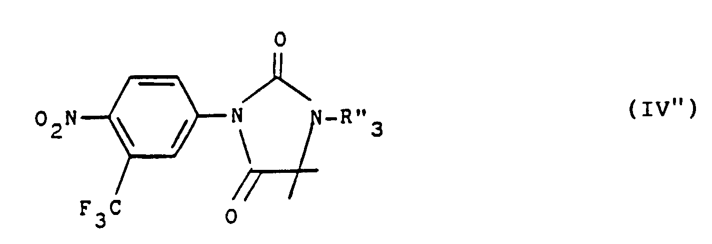 which product of formula (IV") is, if necessary or if desired, subjected to any one or more of the following reactions in any order:
a) elimination reaction of the optional protective groups which can be carried by R"₃ then if appropriate the action of an esterification, amidification or salification agent;
b) conversion reaction of the >C=O group or groups into >C=S groups.

2. Process according to claim 1 for preparing a product of formula (I) as defined in claim 1, in which Y represents an oxygen atom, with the exception of the products in which the group -A-B- represents the radical: 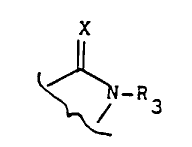 in which X represents an oxygen atom and R₃ represents a hydrogen atom, R₂ represents a halogen atom or a trifluoromethyl radical and R₁ represents a nitro radical or a halogen atom, characterized in that
either a compound of formula (IV) as defined in claim 1 with the exception of the products in which X represents an oxygen atom, R'₃ represents a hydrogen atom, R₂ represents a halogen atom or a trifluoromethyl radical and R₁ represents a nitro radical or a halogen atom, is subjected to any one or more of the following reactions, in any order:
a) elimination reaction of the optional protective groups which can be carried by R'₃;
b) hydrolysis reaction of the >C=NH group into the ketone function then if desired the action of a reagent of formula Hal-R"₃ in which R"₃ has the values of R'₃ with the exception of the hydrogen value and Hal represents a halogen atom, in order to obtain products of formula (I) in which the group -A-B- represents the group 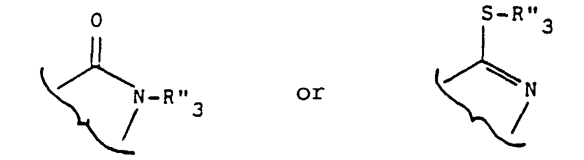 in which R"₃ has the meaning indicated previously then, if desired, the action on these products of an elimination agent of the optional protective groups which can be carried by R"₃ or if appropriate, the action of an esterification,
amidification or salification agent,
or a reagent of formula Hal-R"₃ in which Hal and R"₃ have the values indicated previously is reacted on a product of formula (IV') as defined in claim 1 in order to obtain a product of formula (IV") which, if necessary or if desired, is subjected to an elimination reaction of the optional protective groups which can be carried by R"₃ and if appropriate to the action of an esterification, amidification or salification agent.

3. Process according to claim 1 or 2 for preparing a product of formula (I) as defined in claim 1 in which the group -A-B- represents the group 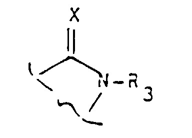 in which X represents a sulphur atom and R₃ has the meaning indicated previously, characterized in that either a product of formula (II) is used at the start in which X represents a sulphur atom, or a product of formula (IV) or (IV") is subjected to any one or more of the following reactions, in any order:
- elimination reaction of the optional protective groups which can be carried by R'₃ or R"₃;
- conversion reaction of the >C=O group or groups into the >C=S group.

4. Process according to claim 3 for preparing a product of formula (I) as defined in claim 3 in which R₃ represents a hydrogen atom or an alkyl radical having at most 4 carbon atoms optionally substituted by a hydroxy radical,
characterized in that either a product of formula (III) is used at the start in which R'₃ has the optionally protected values of R₃ indicated above, or the product of formula (IV') is treated with a reagent of formula Hal-R"₃ in which R"₃ has the optionally protected values of R₃ indicated above with the exception of the hydrogen value then if appropriate the product obtained is subjected to the action of an elimination agent of the protective groups.

5. Process according to any one of claims 1 to 4, characterized in that a product of formula (IT) is used at the start in which R₁ represents a cyano radical or a halogen atom.

6. Process according to claim 5, characterized in that a product of formula (II) is used at the start in which R₁ represents a chlorine atom.

7. Process according to claim 1 or 2 for preparing a product of formula (I) as defined in claim 1 in which the group -A-B- represents a group 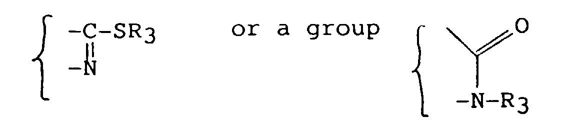 in which R₃ represents an alkyl or alkenyl radical having at most 4 carbon atoms or an optionally substituted aralkyl radical, characterized in that there is used at the start either a product of formula (III) in which R'₃ has the values of R₃ indicated above, or a reagent of formula Hal-R"₃ in which R"₃ has the values of R₃ indicated above.

8. Process according to any one of claims 1 to 5, characterized in that the starting products and reagents are chosen in such a way that any one of the products of which the names follow is prepared:
- 4-(5-oxo-2-thioxo-3,4,4-trimethyl-1-imidazolidinyl)-2(trifluoromethyl)-benzonitrile,
- 4-(4,4-dimethyl-5-oxo-2-thioxo 1-imidazolidinyl)-2(trifluoromethyl)-benzonitrile,
- 4-[4,4-dimethyl 3-(2-hydroxyethyl) 5-oxo 2-thioxo 1imidazolidinyl] 2-(trifluoromethyl) benzonitrile,
- 3-(3,4-dichlorophenyl) 2-thioxo 1,5,5-trimethyl 4-imidazolidinone.

9. Process according to claim 1, 2 or 7, characterized in that the starting products and reagents are chosen in such a way that any one of the products of which the names follow is prepared:
- 1-(4-nitro-3-(trifluoromethyl) phenyl)-3,4,4-trimethyl-2,5-imidazolidinedione,
- 4-[[4,5-dihydro 4,4-dimethyl 5-oxo 2-(phenylmethyl) thio] 1H-imidazol-1-yl] 2-(trifluoromethyl) benzonitrile.

10. Process for preparing pharmaceutical compositions, characterized in that at least one of the compounds of formula (I) as defined in claim 1 is put as active ingredient in a form intended for this use.

11. Process for preparing pharmaceutical compositions, characterized in that at least one of the compounds of formula (I) as defined in any one of claims 2 to 7 is put as active ingredient in a form intended for this use.

12. Process for preparing pharmaceutical compositions, characterized in that at least one of the compounds of formula (I) as defined in claim 8 or 9 is put as active ingredient in a form intended for this use.
